(19) Europäisches Patentamt
European Patent Office
Office européen des brevets

(11) Veröffentlichungsnummer: **0 279 350 B1**

# (12) EUROPÄISCHE PATENTSCHRIFT

(45) Veröffentlichungstag der Patentschrift: **01.12.93**

(51) Int. Cl.[5]: **C07K 5/06**, A61K 37/02, C07D 223/32, C07D 225/04

(21) Anmeldenummer: **88101975.6**

(22) Anmeldetag: **11.02.88**

Die Akte enthält technische Angaben, die nach dem Eingang der Anmeldung eingereicht wurden und die nicht in dieser Patentschrift enthalten sind.

## (54) Annelierte Azepinon- und Azocinon-Derivate, Verfahren zu ihrer Herstellung, diese enthaltende Mittel und ihre Verwendung, sowie Zwischenprodukte bei ihrer Herstellung.

(30) Priorität: **14.02.87 DE 3704661**

(43) Veröffentlichungstag der Anmeldung:
**24.08.88 Patentblatt 88/34**

(45) Bekanntmachung des Hinweises auf die Patenterteilung:
**01.12.93 Patentblatt 93/48**

(84) Benannte Vertragsstaaten:
**AT BE CH DE ES FR GB GR IT LI LU NL SE**

(56) Entgegenhaltungen:
**EP-A- 0 107 095**
**FR-A- 2 369 269**

(73) Patentinhaber: **HOECHST AKTIENGESELL-SCHAFT**

**D-65926 Frankturt(DE)**

(72) Erfinder: **Urbach, Hansjörg, Dr.**
**Le Lavandoustrasse 41**
**D-6242 Kronberg/Taunus(DE)**
Erfinder: **Henning, Rainer, Dr.**
**Rotenhofstrasse 31**
**D-6234 Hattersheim am Main(DE)**
Erfinder: **Schölkens, Bernward, Dr.**
**Hölderlinstrasse 62**
**D-6233 Kelkheim (Taunus)(DE)**
Erfinder: **Becker, Reinhard, Dr.**
**Adelheidstrasse 101**
**D-6200 Wiesbaden(DE)**

EP 0 279 350 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Benzazepin-2-one sind aus EP-A-72 352, Benzthiazepinon- und Benzthiazocinon-Derivate aus der DE-A1 34 26 720 bekannt. Es werden Verfahren zu ihrer Herstellung sowie ihre Verwendung als Inhibitoren des Angiostensin-Converting-Enzyme (ACE) beschrieben.

Die vorliegende Erfindung beruht auf der Tatasache, daß bestimmte neuartige Derivate des Azepinons und des Azocinons stark inhibierend auf das Angiotensin-Converting Enzyme wirken. Diese Eigenschaft macht diese Verbindungen besonders wertvoll zur Anwendung an Säugern - vorzugsweise am Menschen - bei der Behandlung oder Prophylaxe von Erkrankungen, die auf eine ACE-Hemmung reagieren, wie kardiovaskulare Störungen (z.B. Bluthochdruck), kardiale Zustände (z.B. Herzinsuffizienz) und Glaukome.

Die Erfindung betrifft Verbindungen der Formel I,

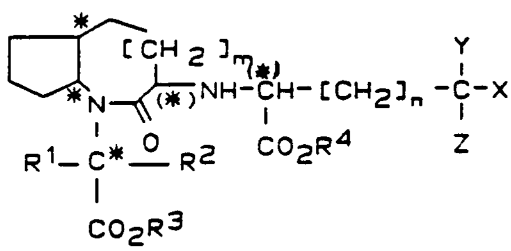

(I)

in welcher

m = 1 oder 2 und

| | |
|---|---|
| $R^1$ und $R^2$ | gleich oder verschieden sind und Wasserstoff, $(C_1\text{-}C_6)$-Alkyl, das gegebenenfalls monosubstituiert sein kann durch Hydroxy, Mercapto, $(C_1\text{-}C_2)$-Alkoxy, $(C_1\text{-}C_2)$-Alkylmercapto, Carboxy, $(C_1\text{-}C_2)$-Alkxoycarbonyl, 3-Indolyl, Imidazolyl, Carbamoyl, Amino oder Guanidino, $(C_2\text{-}C_6)$-Alkenyl, $(C_3\text{-}C_9)$-Cycloalkyl, $(C_3\text{-}C_9)$-Cycloalkenyl, $(C_3\text{-}C_7)$-Cycloalkyl-$(C_1\text{-}C_4)$-alkyl, $(C_6\text{-}C_{12})$-Aryl, teilhydriertes $(C_6\text{-}C_{12})$-Aryl oder $(C_6\text{-}C_{12})$-Aryl-$(C_1\text{-}C_4)$-alkyl, das im Arylteil eine Hydroxygruppe tragen kann, bedeuten, |
| $R^3$ und $R^4$ | gleich oder verschieden sind und Wasserstoff, $(C_1\text{-}C_6)$-Alkyl, $(C_2\text{-}C_6)$-Alkenyl oder $(C_6\text{-}C_{12})$-Aryl-$(C_1\text{-}C_4)$-Alkyl, das im Arylteil durch Methoxy oder Nitro monosubstituiert sein kann, bedeuten, |
| Y | Wasserstoff oder Hydroxy, |
| Z | Wasserstoff oder |
| Y | und Z zusammen Sauerstoff bedeuten und |
| X | für $(C_1\text{-}C_6)$-Alkyl, das durch Amino, Acylamino, $(C_1\text{-}C_4)$-Alkylamino und/oder Di-$(C_1\text{-}C_4)$-Alkylamino substituiert sein kann, $(C_2\text{-}C_6)$-Alkenyl, $(C_5\text{-}C_9)$-Cycloalkyl, |

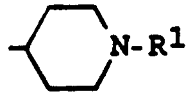

$(C_6\text{-}C_{12})$-Aryl, das durch $(C_1\text{-}C_4)$-Alkyl, $(C_1\text{-}C_4)$-Alkoxy, Hydroxy, Halogen, Nitro, Amino, $(C_1\text{-}C_4)$-Alkylamino, Di-$(C_1\text{-}C_4)$-Alkylamino und/oder Methylendioxy mono-, di-oder trisubstituiert sein kann, oder Indol-3-yl steht, wobei in den obengenannten Resten $R^1$, $R^2$ und X freies Hydroxy, Mercapto, Carboxy, Amino oder Guanidino gegebenenfalls durch in der Peptidchemie übliche Schutzgruppen geschützt sind,

sowie deren physiologisch unbedenklichen Salze.

Bevorzugt sind Verbindungen der Formel I, in welcher

m = 1 und

n = 1 ist,

| | |
|---|---|
| $R^1$ | Wasserstoff, $(C_1\text{-}C_3)$-Alkyl, $(C_3\text{-}C_6)$-Cycloalkyl, $(C_3\text{-}C_6)$-Cycloalkyl-$(C_1\text{-}C_2)$-alkyl oder Allyl, |
| $R^2$ | Wasserstoff, $(C_1\text{-}C_3)$-Alkyl, $(C_3\text{-}C_6)$-Cycloalkyl, $(C_3\text{-}C_6)$-Cycloalkyl-$(C_1\text{-}C_2)$-alkyl oder Allyl, |
| $R^3$ | Wasserstoff, $(C_1\text{-}C_4)$-Alkyl, Benzyl oder 4-Methoxybenzyl, |
| $R^4$ | Wasserstoff, $(C_1\text{-}C_4)$-Alkyl, Benzyl oder 4-Methoxybenzyl, |
| X | Phenyl oder mit Fluor und/oder Chlor mono- oder disubstituiertes Phenyl, Methyl, Cyclohex- |

yl oder Aminoethyl,

Y Wasserstoff oder Hydroxy und

Z Wasserstoff bedeuten, oder

Y und Z zusammen für Sauerstoff steht,

insbesondere aber Verbindungen der Formel I, in welcher

m = 1,

n = 1,

$R^1$, $R^2$, und $R^3$ jeweils Wasserstoff, $R^4$ = Wasserstoff oder Ethyl, Y und Z jeweils Wasserstoff und X Phenyl bedeuten.

Als besonders bevorzugte Verbindungen seien erwähnt:

(5a-S,8a-S)-1-Carboxymethyl-3-(S)-[1-(S)-ethoxycarbonyl-3-phenyl-propylamino]-2-oxo-perhydrocyclopent-[b]azepin
(5a-S,8a-R)-1-Carboxymethyl-3-(S)-[1-(S)-ethoxycarbonyl-3-phenyl-propylamino]-2-oxo-perhydrocyclopent-[b]azepin
(5a-R,8a-S)-1-Carboxymethyl-3-(S)-[1-(S)-ethoxycarbonyl-3-phenyl-propylamino]-2-oxo-perhydrocyclopent-[b]azepin
(5a-R,8a-R)-1-Carboxymethyl-3-(S)-[1-(S)-ethoxycarbonyl-3-phenyl-propylamino]-2-oxo-perhydrocyclopent-[b]azepin
(5a-S,8a-S)-1-Carboxymethyl-3-(S)-[1-(S)-carboxy-3-phenyl-propylamino]-2-oxo-perhydrocyclopent[b]azepin
(5a-S,8a-R)-1-Carboxymethyl-3-(S)-[1-(S)-carboxy-3-phenyl-propylamino]-2-oxo-perhydrocyclopent[b]azepin
(5a-R,8a-S)-1-Carboxymethyl-3-(S)-[1-(S)-carboxy-3-phenyl-propylamino]-2-oxo-perhydrocyclopent[b]azepin
(5a-R,8a-R)-1-Carboxymethyl-3-(S)-[1-(S)-carboxy-3-phenyl-propylamino]-2-oxo-perhydrocyclopent[b]azepin
(5a-S,8a-S)-1-Carboxymethyl-3-(S)-[1-(S)-ethoxycarbonyl-butylamino]-2-oxo-perhydrocyclopent[b]azepin
(5a-S,8a-R)-1-Carboxymethyl-3-(S)-[1-(S)-ethoxycarbonyl-butylamino]-2-oxo-perhydrocyclopent[b]azepin
(5a-R,8a-S)-1-Carboxymethyl-3-(S)-[1-(S)-ethoxycarbonyl-butylamino]-2-oxo-perhydrocyclopent[b]azepin
(5a-R,8a-R)-1-Carboxymethyl-3-(S)-[1-(S)-ethoxycarbonyl-butylamino]-2-oxo-perhydrocyclopent[b]azepin
(5a-S,8a-S)-1-Carboxymethyl-3-(S)-[1-(S)-Carboxy-butylamino]-2-oxo-perhydrocyclopent[b]azepin
(5a-S,8a-R)-1-Carboxymethyl-3-(S)-[1-(S)-carboxy-butylamino]-2-oxo-perhydrocyclopent[b]azepin
(5a-R,8a-S)-1-Carboxymethyl-3-(S)-[1-(S)-carboxy-butylamino]-2-oxo-perhydrocyclopent[b]azepin
(5a-R,8a-R)-1-Carboxymethyl-3-(S)-[1(S)-carboxy-butylamino]-2-oxo-perhydrocyclopent[b]azepin
(5a-S,8a-S)-1-Carboxymethyl-3-(S)-[1-(S)-ethoxycarbonyl-5-amino-pentylamino]-2-oxo-perhydrocyclopent[b]-azepin
(5a-S,8a-R)-1-Carboxymethyl-3-(S)-[1-(S)-ethoxycarbonyl-5-amino-pentylamino]-2-oxo-perhydrocyclopent[b]-azepin
(5a-R,8a-S)-1-Carboxymethyl-3-(S)-[1-(S)-ethoxycarbonyl-5-amino-pentylamino]-2-oxo-perhydrocyclopent[b]-azepin
(5a-R,8a-R)-1-Carboxymethyl-3-(S)-[1-(S)-ethoxycarbonyl-5-amino-pentylamino]-2-oxo-perhydrocyclopent[b]-azepin
(5a-S,8a-S)-1-Carboxymethyl-3-(S)-[1-(S)-carboxy-5-amino-pentylamino]-2-oxo-perhydrocyclopent[b]azepin
(5a-S,8a-R)-1-Carboxymethyl-3-(S)-[1-(S)-carboxy-5-amino-pentylamino]-2-oxo-perhydrocyclopent[b]azepin
(5a-R,8a-S)-1-Carboxymethyl-3-(S)-[1-(S)-carboxy-5-amino-pentylamino]-2-oxo-perhydrocyclopent[b]azepin
(5a-R,8a-R)-1-Carboxymethyl-3-(S)-[1-(S)-carboxy-5-amino-pentylamino]-2-oxo-perhydrocyclopent[b]azepin
(6a-S,9a-S)-1-Carboxymethyl-3-(S)-[1-(S)-ethoxycarbonyl-3-phenyl-propylamino]-2-oxo-perhydro-2H-cyclopent[b]azocin
(6a-S,9a-R)-1-Carboxymethyl-3-(S)-[1-(S)-ethoxycarbonyl-3-phenyl-propylamino]-2-oxo-perhydro-2H-cyclopent[b]azocin
(6a-R,9a-S)-1-Carboxymethyl-3-(S)-[1-(S)-ethoxycarbonyl-3-phenyl-propylamino]-2-oxo-perhydro-2H-cyclopent[b]azocin
(6a-R,9a-R)-1-Carboxymethyl-3-(S)-[1-(S)-ethoxycarbonyl-3-phenyl-propylamino]-2-oxo-perhydro-2H-cyclopent[b]azocin
(6a-S,9a-S)-1-Carboxymethyl-3-(S)-[1-(S)-carboxy-3-phenyl-propylamino]-2-oxo-perhydro-2H-cyclopent[b]-azocin
(6a-S,9a-R)-1-Carboxymethyl-3-(S)-[1-(S)-carboxy-3-phenyl-propylamino]-2-oxo-perhydro-2H-cyclopent[b]-azocin
(6a-R,9a-S)-1-Carboxymethyl-3-(S)-[1-(S)-carboxy-3-phenyl-propylamino]-2-oxo-perhydro-2H-cyclopent[b]-azocin
(6a-R,9a-R)-1-Carboxymethyl-3-(S)-[1-(S)-carboxy-3-phenyl-propylamino]-2-oxo-perhydro-2H-cyclopent[b]-azocin

Als Salze kommen insbesondere Alkali- und Erdalkalisalze, Salze mit physiologisch verträglichen Aminen und Salze mit anorganischen oder organischen Säuren, wie z.B. HCl, HBr, $H_2SO_4$, Maleinsäure, Fumarsäure oder Weinsäure in Frage.

Unter Aryl ist hier wie im folgenden gegebenenfalls substituiertes Phenyl, Naphthyl oder Biphenylyl, insbesondere aber Phenyl zu verstehen. Entsprechendes gilt für Aralkyl. Unter Acyl wird insbesondere ($C_1$-$C_6$)-Alkanoyl, Benzoyl, t-Butoxycarbonyl (Boc) und Benzyloxycarbonyl (Z) verstanden. Alkyl kann geradkettig oder verzweigt sein.

Verbindungen der Formel I besitzen chirale C-Atome, die mit einem Stern gekennzeichnet sind. Sowohl die R- als auch die S-Konfiguration an allen Asymmetriezentren sind Gegenstand der Erfindung. Die Verbindungen der Formel I können daher als optische Isomere, als Diastereomere, als Racemate oder als Gemische derselben vorliegen. Bevorzugt sind jedoch Verbindungen der Formel I in denen die mit einem Stern [(*)] markierten C-Atome die S-Konfiguration aufweisen.

Die Erfindung betrifft ferner ein Verfahren zur Herstellung der Verbindungen der Formel I, das dadurch gekennzeichnet ist, daß man

a) eine Verbindung der Formel II,

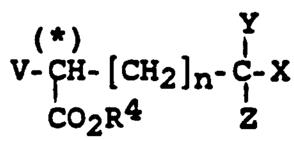

(II)

in der n, $R^4$, X, Y und Z wie oben definiert sind,
$R^4$ jedoch nicht Wasserstoff bedeutet und V für eine nucleophil substituierbare Abgangsgruppe steht, mit einer Verbindung der Formel III,

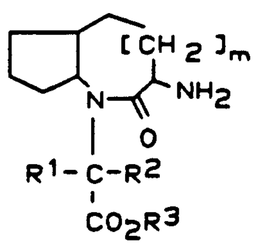

(III)

in der m, $R^1$, $R^2$ und $R^3$ und wie oben definiert sind,
$R^3$ jedoch nicht Wasserstoff bedeutet, umsetzt,

b) eine Verbindung der vorstehend definierten Formel III mit einer Verbindung der Formel IV,

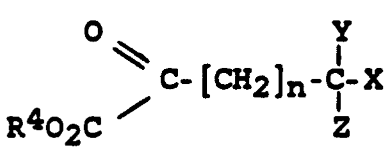

(IV)

in der n, $R^4$ und X vorstehende Bedeutungen haben und Y und Z jeweils Wasserstoff bedeuten, in Gegenwart eines Reduktionsmittels umsetzt,

c) eine Verbindung der Formel V,

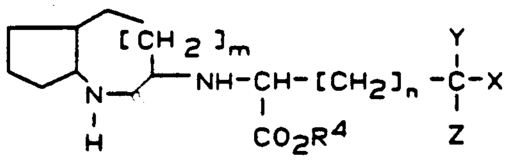

(V)

in der m, n, $R^4$, X, Y und Z wie oben definiert sind,
mit einer Verbindung der Formel VI,

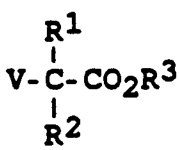

(VI)

in der $R^1$, $R^2$, $R^3$ und V wie oben unter a) definiert sind, alkyliert,
d) eine Verbindung der Formel VII,

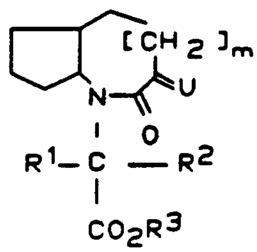

(VII)

in der m, $R^1$, $R^2$ und $R^3$ die oben unter a) definierten Bedeutungen haben und U eine Oxo-Gruppe oder zusammen Wasserstoff und eine unter a) definierte Gruppe V bedeutet, mit einem Amin der Formel VIII,

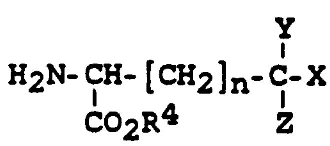

(VIII)

in der n, $R^4$ und X wie oben unter a) definiert
sind und Y und Z Wasserstoff bedeuten, umgesetzt, wobei im Falle U = Oxo die Umsetzung in Gegenwart eines Reduktionsmittels erfolgt,

e) eine Verbindung der Formel IX,

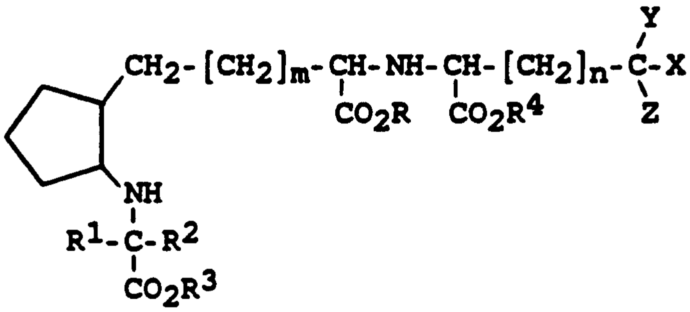

(IX)

in der m, n, $R^1$, $R^2$, $R^3$, $R^4$, X, Y und Z die oben unter a) angegebenen Bedeutungen besitzen und R Wasserstoff oder eine Estergruppe bedeutet, cyclisiert oder

f) zur Herstellung von Verbindungen der Formel I, in der Y und Z zusammen Sauerstoff bedeutet, eine Verbindung der unter a) definierten Formel III mit einer Verbindung der Formel X

$R^4O_2C\text{-}CH = CH\text{-}CO\text{-}X$ (X)

in der $R^4$ und X wie oben in Formel I definiert sind,in an sich bekannter Weise in einer Michael-Reaktion (Organikum, 6. Auflage, (1967) 492) umsetzt oder die oben genannte Verbindung der Formel III in an sich bekannter Weise in einer Mannich-Reaktion (Bull. Soc. Chim. France 1973, 620) umsetzt mit einer Verbindung der Formel XI,

$OHC\text{-}CO_2R^4$ (XI)

und einer Verbindung der Formel XII,

$X\text{-}CO\text{-}CH_3$ (XII)

in welchen $R^4$ und X die oben in Formel I definierten Bedeutungen haben. Falls Y and Z zusammen Sauerstoff (d.h. Oxo) bedeuten, diese Gruppe zu gegebenenfalls Y = Hydroxy oder Wasserstoff und Z = Wasserstoff reduziert,

i) gewünschtenfalls temporär zum Schutz funktioneller Gruppen eventuell eingeführte Schutzgruppen in an sich bekannter Weise abspaltet,

ii) gegebenenfalls Carboxygruppen $CO_2R^3$ und/oder $CO_2R^4$ ($R^3$,$R^4$ = H) in an sich bekannter Weise unter Bildung von Verbindungen der Formel I ($R^3$ und/oder $R^4 \neq$ H) verestert,

iii) gegebenenfalls die Reste $R^3$ und/oder $R^4$ ($R^3$, $R^4 \neq$ H) hydrolytisch oder hydrogenolytisch unter Bildung der freien Carboxygruppe(n) abspaltet,

oder die Reihenfolge dieser Schritte i - iii umkehrt,
und die auf diese Weise erhaltenen Verbindungen der Formel I gegebenenfalls in ihre physiologisch annehmbaren Salze überführt.

Bei der Verfahrensvariante a) bedeutet V eine nucleophil substituierbare Abgangsgruppe ("nucelofuge" Gruppe) wie z.B. Chlor, Brom, Jod, Mesyloxy, Tosyloxy oder Trifluormethylsulfonyloxy.

Die Umsetzung einer Verbindung der Formel II mit einer Verbindung der Formel III zur Herstellung einer Verbindung der Formel I erfolgt in einer an sich bekannten nucleophilen Substitutionsreaktion. Besonders geeignet erwiesen sich Verbindungen der Formel II, in der Y und Z gleich Wasserstoff und V gleich Trifluormethylsulfonyloxy bedeuten. Läßt man Verbindungen dieser Art mit Verbindungen der Formel III, in der m, $R^1$, $R^2$ und $R^3$ die oben angegebenen Bedeutungen haben, reagieren und setzt eine optisch aktive Verbindung der Formel II ein, z.B. eine Verbindung der Formel II mit der R-Konfiguration an dem mit einem Stern [(*)] bezeichneten Kohlenstoffatom, so werden unter Walden-Umkehr die entsprechenden optisch reinen S-konfigurierten Derivate der Formel I erhalten. Die Reaktion läßt sich vorzugsweise in einem aprotischen polaren oder unpolaren Lösungsmittel wie z.B. Methylenchlorid, Chloroform, Toluol, Dimethylformamid, Dimethoxyethan, Dimethylsulfoxid, Tetrachlorkohlenstoff, Essigsäureethylester, Hexan, Ether,

Tetrahydrofuran oder Hexamethylphophorsäuretriamid im Temperaturbereich zwischen -80°C und +150°C, vorzugsweise zwischen -20°C und dem Siedepunkt des Reaktionsgemisches durchführen. Um die entstehende Verbindung HV, wie z.B. Trifluormethansulfonsäure abzufangen, wird die Reaktion zweckmäßig in Gegenwart, vorzugsweise mindestens einem Äquivalent einer Base durchgeführt, die nicht mit den Verbindungen der Formel I oder II oder III abreagieren kann. Als vorteilhaft haben sich tert. Amine wie Triethylamin oder auch Pyridin erwiesen. Auch die entstanden Aminosäurederivate können als Säurefänger dienen. Geeignet sind auch anorganische Basen wie z.B. $Na_2CO_3$, $K_2CO_3$, $NaHCO_3$ oder im Falle der Triflate auch $Na_2SO_4$.

Das Verfahren gemäß Variante b) entspricht der in J. Amer. Chem. Soc. 93 (1971) 2897 beschriebenen Verfahrensweise. Die Verbindungen der Formeln III und IV werden miteinander in Gegenwart eines Reduktionsmittels wie komplexen Metallhydriden (z.B. Natriumcyanborhydrid oder Natriumborhydrid) oder Wasserstoff und Metallkatalysatoren (z.B. Raney-Nickel, Pd, Pt) in einem inerten Lösungsmittel bei einer Temperatur zwischen -20 und 150°C umgesetzt.

Die Umsetzung von Verbindungen der Formel V mit Verbindungen der Formel VI gemäß Verfahrensvariante c) erfolgt in der Weise, daß mit einer starken Base wie z.B. Natriumhydrid oder Natriumamid das Lactam V deprotoniert wird und dann in an sich bekannter Weise in einem aprotischen polaren oder unpolaren Lösungsmittel wie Dimethylformamid, Dimethoxyethan oder Methylenchlorid zwischen -20°C und 150°C weiter umsetzt.

Brauchbare Reduktionsmittel für Verfahrensvariante d) sind z.B. unter b) beschrieben. Falls U für H + V steht, erfolgt die Umsetzung gegebenfalls in Gegenwart einer organischen oder anorganischen Base, wie Triethylamin oder KOH.

Bei der Verfahrensvariante e) steht der Rest R für Wasserstoff oder eine Estergruppe, wobei ($C_1$-$C_6$)-Alkylester oder ($C_7$ bis $C_9$)-Aralkylester (wie Benzylester) bevorzugt sind. Die Cyclisierung erfolgt in an sich bekannter Weise, z.B. nach den Methoden, wie sie in der Peptidchemie üblich sind (wie Carbodiimid-Methode, Peptides, Vol. I, Academic Press, London, New York 1965, S. 108), oder unter saurer oder basischer Katalyse in einem polaren Lösungsmittel, vorzugsweise einem Alkohol, bei einer Temperatur zwischen etwa 10°C und dem Siedepunkt des Reaktionsgemisches.

Werden nach den Verfahren a) bis f) Verbindungen der Formel I erhalten, in denen $R^3$ und/oder $R^4$ ungleich Wasserstoff sind, so können diese gegebenenfalls einer Hydrogenolyse oder einer sauren oder basischen Hydrolyse unterworfen werden, um Verbindungen der Formel I zu erhalten, in denen $R^3$ und/oder $R^4$ gleich Wasserstoff sind.

Verbindungen der Formel I mit Y = Hydroxy und Z = Wasserstoff können beispielsweise auch durch Reduktion einer gemäß obigen Verfahrensweisen erhaltenen Verbindung I mit Y und Z zusammen Sauerstoff erhalten werden. Diese Reduktion kann mit einem Reduktionsmittel wie Natriumborhydrid und anderen komplexen Boranten oder beispielsweise Boran-Amin-Komplexen erfolgen.

Verbindungen der Formel I, in welcher $R^3$ und/oder $R^4$ für Wasserstoff stehen, können gegebenenfalls nach an sich bekannten Methoden in ihre Ester überführt werden. Eine wichtige Methode ist die direkte Veresterung der freien Säuren (Alkoholyse von Carbonsäure).

Infolge der geringen Carbonylaktivität reagieren Carbonsäuren im allgemeinen nur langsam mit Alkoholen.

Durch Zusatz starker Säuren (Schwefelsäure, wasserfreier Chlorwasserstoff, Sulfonsäuren, saure Ionenaustauscher) kann die Veresterung erheblich beschleunigt werden.

Das Veresterungsgleichgewicht kann nach rechts verschoben werden, indem man den Alkohol in 50 bis 10fachem Überschuß einsetzt oder indem man dem Reaktionsgemisch ständig Wasser entzieht.

Im einfachsten Fall wird das gebildete Wasser von der zugesetzten Katalysatorsäure (Schwefelsäure, Chlorwasserstoffsäure) gebunden. Auch die Entfernung des Wassers durch azeotrope Destillation ist vorteilhaft. Die Wahl des Schleppers richtet sich nach dem Siedepunkt der am tiefsten siedenden organischen Komponente. Für die Darstellung von Ethylestern und Propylestern ist Chloroform oder Tetrachlorkohlenstoff brauchbar. Höhere Alkohole ab Butanol bilden selbst Azeotrope mit Wasser, so daß kein weiterer Schlepper zugesetzt werden muß.

Bei der sogenannten extraktiven Veresterung wird der gebildete Ester von einem Lösungsmittel, das nur sehr wenig Wasser löst, selektiv aus dem Reaktionsgemisch herausgelöst. Die Methode ist vor allem für die Darstellung von Methylestern geeignet, wo die azeotrope Veresterung mit einfachen Mitteln nicht gelingt.

Im Gegensatz zur direkten Veresterung der Carbonsäuren lassen sich aus den entsprechenden Säurehalogeniden auch die Ester der tertiären Alkohole und der Phenole darstellen.

Zum Schutz freier funktioneller Gruppen in den Resten $R^1$, $R^2$, $R^3$, $R^4$ und X eignen sich die folgenden in der Peptidchemie üblichen Schutzgruppen.

Phenolische OH-Gruppen schützt man beispielsweise mit Acylgruppen vom Benzyloxycarbonyltyp (wie Z, BrZ) oder mit O-Alkylgruppen (wie Bzl, Mob, Pic, $Bu^t$).

Zum Schutz alkoholischer OH-Gruppen dienen vor allem o-Alkylgruppen wie $Bu^t$ oder Bzl.

Häufig verwendete Carboxylschutzgruppen sind die Alkylestergruppen wie OMe, OEt, OBzl, ONbzl, OMbzl, OPic, $OBu^t$ oder OPac.

Die Aminogruppe wird vorteilhaft mit einer Schutzgruppe vom Urethantyp geschützt, wie insbesondere Boc oder Z, aber auch Pyoc, Fmoc, Tcboc, Ddz, Bpoc, $Z(NO_2)$, Moc, Mboc, Iboc, Adoc oder Adpoc.

Der Schutz der Guanidinogruppe ist beispielsweise mittels Nitrierung oder einem Teil der vorstehenden Urethanschutzgruppen möglich.

Thiol schützt man z.B. als Thioether, S-Acetal oder umsymmetriches Disulfid.

Schuztgruppe in der Peptidsynthese (Abkürzungen, Einführung und Abspaltung dieser Gruppen) werden zusammenfassend in Kontakte Merck 7/79, Seiten 14-22 und 1/80 Seiten 23-35 beschrieben (siehe hierzu auch Schröder, Lübke "The Peptides" Volume 1, Academic Press New York, London 1965).

Verbindungen der Formel II bis IX können als Racemate, Diastereomere oder optische reine Verbindungen eingesetzt werden. Diastereomere der Formel I können beispielsweise durch Kristallisation oder Chromatographie voneinander getrennt werden. Werden überhaupt durch die beschriebenen Reaktionen Diastereomere erzeugt, so können diese durch Kristallisation oder Chromatographie getrennt werden.

Die Erfindung betrifft auch Verbindungen der Formel XIII,

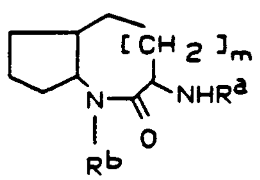

(XIII)

in der m wie oben definiert ist,

a) $R^a$ und $R^b$ jeweils Wasserstoff bedeuten,

b) $R^a$ Wasserstoff bedeutet und $R^b$ für einen Rest

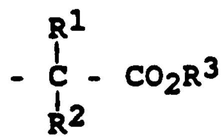

in dem $R^1$, $R^2$ und $R^3$ wie oben definiert sind, steht,

c) $R^a$ für einen Rest

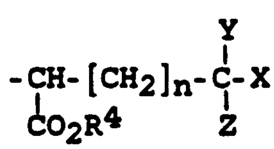

in dem n, $R^4$, X, Y und Z wie oben definiert sind, steht und $R^b$ Wasserstoff bedeutet, oder

d) $R^a$ für einen Rest

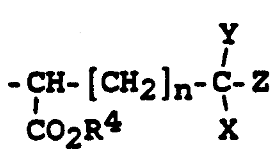

,

in dem n, $R^4$, X, Y und Z wie oben definiert sind, und $R^b$ für einen Rest

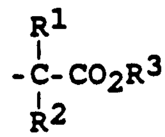

in dem $R^1$, $R^2$ und $R^3$ wie oben definiert sind, stehen
sowie ein
Verfahren zur Herstellung dieser Verbindungen, das dadurch gekennzeichnet ist, daß man
a) Verbindungen der Formel XIV,

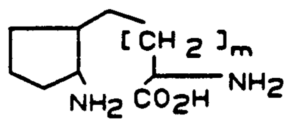

(XIV)

in welcher m wie oben definiert ist, cyclisiert zu Verbindungen der Formel XV (≅ Formel XIII mit $R^a$, $R^b$ jeweils = Wasserstoff),

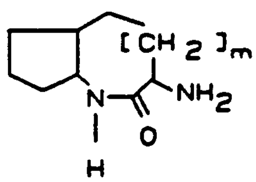

(XV)

in welcher m wie oben definiert ist,
b) Verbindungen der Formel XVI,

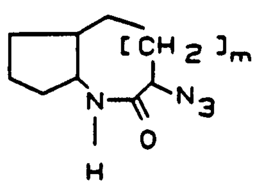

(XVI)

in welcher m wie oben definiert ist, zu Verbindungen der oben definierten Formel XV, reduziert,
c) Verbindungen der obigen Formel XV gegebenenfalls mit Verbindungen der Formel VI,

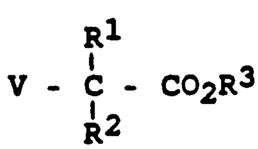

(VI)

in welcher V, $R^1$, $R^2$ und $R^3$ wie beim Verfahren zur Herstellung von Verbindungen der Formel I unter c) definiert sind, zu Verbindungen der Formel III (≙ Formel XIII mit $R^a$ = H, $R^b \neq$ H) alkyliert,
d) Verbindungen der obigen Formel XV gegebenenfalls mit Verbindungen der beim Herstellungsverfahren für Verbindungen der Formel I unter a) definierten Formel II zu Verbindungen der Formel V (≅ Formel XIII mit $R^a \neq$ H, $R^b$ = H) umsetzt,
e) Verbindungen der obigen Formel XV mit einer Verbindung der Formel X

$R^4O_2C\text{-}CH=CH\text{-}CO\text{-}X$ (X)

in der $R^4$ und X wie oben in Formel I definiert sind, in an sich bekannter Weise in einer Michael-Reaktion

umsetzt oder die obengenannte Verbindung der Formel III
in an sich bekannter Weise in einer Mannich-Reaktion umsetzt mit einer Verbindung der Formel XI,

$OCH\text{-}CO_2R^4$ (XI)

und einer Verbindung der Formel XII,

$X\text{-}CO\text{-}CH_3$ (XII)

in welchen $R^4$ und X die im Anspruch 1 in Formel I definierten Bedeutungen haben, falls Y und Z zusammen Sauerstoff bedeutet, diese Gruppe gegebenenfals zu Y = Hydroxy oder Wasserstoff und Z = Wasserstoffe reduziert,
f) oder eine Verbindung der Formel XVI mit Verbindungen der Formel VI zu Verbindungen der Formel XVII,

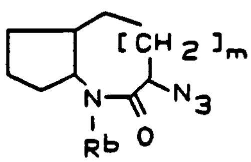

(XVII)

in der m und $R^b$ ($R^b \neq H$) wie oben definiert unter b) (S. 16) sind, alkyliert und diese Verbindungen durch katalytische Hydrierung mit Metallkatalysatoren oder durch Reaktion mit komplexen Hydriden in Verbindungen der Formel XIII ($R^a$ = H, $R^b \neq H$) überführt.

Verbindungen der Formel XIV als Ausgangsverbindungen für die Verfahrensvariante a) werden hergestellt, indem man Verbindungen der Formel XVIII

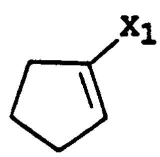

(XVIII)

in welcher $X^1$ für Dialkylamino mit 2 bis 10 C-Atomen oder für einen Rest der Formel

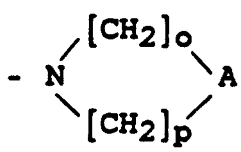

worin o und p eine ganze Zahl von 1 bis 3, (o + p) ≧ 3 und A $CH_2$, NH, N-($C_1$-$C_6$)-Alkyl, O oder S bedeuten, steht
mit Verbindungen der Formel XIX

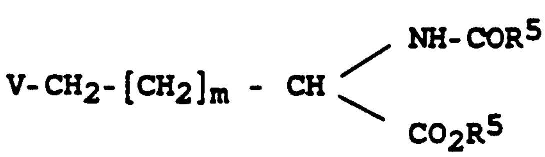

(XIX)

in welcher V und m die oben angegebene Bedeutung besizten, wobei V vorzugsweise für Chlor, Brom, Jod oder Tosyloxy steht, und $R^5$ Wasserstoff oder ($C_1$-$C_4$)-Alkyl bedeutet, in Gegenwart einer Base, wie z.B. einem tertiären Amin, in einem oganischen aprotischen polaren oder unpolaren Lösungsmittel bei Temperaturen von -20°C bis 150°C, vorzugsweise 20°C bis 150°C und anschließender milder saurer Hydrolyse (z.B. mit wäßrigen Mineralsäuren) zu Verbindungen der Formel XX umsetzt,

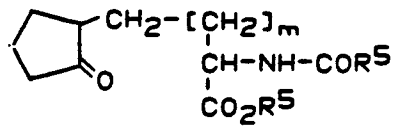

(XX)

in welcher m und $R^5$ wie oben definiert sind.

Verbindungen der Formel XIX lassen sich nach Schutz der Carboxyl- und Aminogruppe unter Bedingungen wie sie in der Aminosäure- bzw. Peptidchemie üblich sind, aus Homoserin (m = 1) herstellen. Entsprechend verfährt man zur Herstellung der Verbindungen der Formel XIX mit m = 2.

Die Verbindungen der Formel XX werden in an sich bekannter Weise mit Hydroxylamin bzw. den Salzen in die Oxime XXa, in der m und $R^5$ die obige Bedeutung besitzen, überführt, die unter reduktiven Bedingungen wiederum in die Amine der Formel XX b, in der m und $R^5$ die obige Bedeutung besitzen, überführt werden. Die Reduktion kann mit Wasserstoff und einem Metallkatalysator wie z.B. Pt, Pt auf Kohle, $PtO_2$, Pd, Pd auf Kohle oder Raney-Nickel erfolgen oder mit komplexen Hydriden wie z.B. Natriumcyanborhydrid.

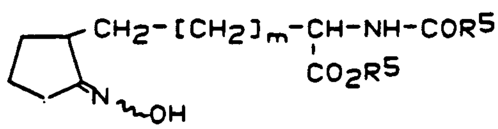

(XXa)

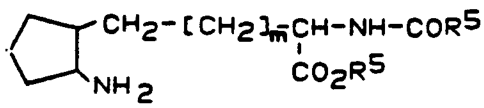

(XX b)

Die Verbindungen der Formel XX b liegen als Diastereomerengemische vor, die gegebenenfalls durch Kristallisation oder Chromatographie getrennt werden können.

Die so erhaltenen Verbindungen der Formel XX b werden sauer oder alkalisch in an sich bekannter Weise zu Verbindungen der Formel XIV verseift, die je nach Hydrolysebedingungen als entsprechende Salze anfallen können. Die Cyclisierung zu den Verbindungen der Formel XV kann unter sauren oder alkalischen Bedingungen in einem polaren oder apolaren Lösungsmittel zwischen Raumtemperatur und dem Siedepunkt des Reaktionsgemisches, vorzugsweise bei erhöhter Temperatur oder unter den Bedingungen, die in der Peptidchemie zur Bildung von Amidbindungen üblich sind, erfolgen. Besonders vorteilhaft erweist sich zur Cyclisierung der Verbindungen der Formel XIV die Reaktion mit einem Diimid, wie z.B. Dicyclohexylcarbodiimid, wobei die Gegenwart von Hydroxybenztriazol die Reaktion beschleunigen kann. Als Lösungsmittel kann ein aprotisches polares oder unpolares Lösungsmittel genommen werden. Als besonders vorteilhaft erweist sich z.B. Dimethylformamid. Die Reaktionstemperatur kann im Bereich von -20° bis 80°C liegen. Besonders zweckmäßig kann die Cyclisierung mit Alkylphosphonsäure- oder Dialkylphosphinsäureanhydriden in einem aprotischen polaren oder unpolaren Lösungsmittel erreicht werden. Besonders bewährt haben sich n-Propanphosphonsäureanhydrid oder Methyl-ethyl-phosphinsäureanhydrid unter Reaktionsbedingungen, die in den US-Patenten 4 331 592 und 4 426 325 beschrieben sind.

Die Umsetzung wird z.B. im neutralen oder schwach alkalischen Medium durchgeführt. Am einfachsten ist es, den pH-Wert des Mediums durch Zugabe von aliphatischen oder cycloaliphatischen tertiären Basen wie z.B. N-Methylmorpholin, N-Ethylmorpholin oder Trialkylaminen mit bis zu 6 C-Atomen pro Alkylrest einzustellen. Beim Arbeiten in gemischtwäßrigen Systemen können anstelle der organischen Base auch als Puffersysteme wirkende alkalische Salze wie z.B. solche der Kohlensäure oder der Phosphorsäure verwendet werden.

Azide der Formel XVI erhält man, indem man Verbindungen der Formel XXI,

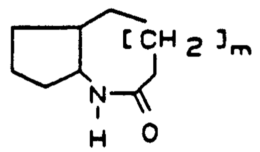

(XXI)

in der m die oben angegebene Bedeutung besitzt, zu Verbindungen der Formel XXII

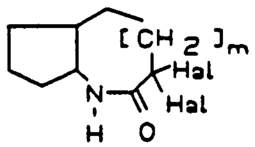

(XXII)

in der m wie oben definiert ist und Hal Halogen, vorzugsweise Chlor oder Brom, bedeutet, halogeniert und diese katalytisch zu Verbindungen der Formel XXIII

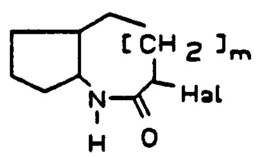

(XXIII)

in der m und Hal obige Bedeutung besitzen, reduziert.

Verbindungen der Formel XXI in der m wie oben definiert ist, lassen sich z.B. durch Alkylierung und Kondensation der Verbindungen der Formel XVIII oder den Alkalimetallsalzen der Verbindungen der Formel XXIV (wobei $R^5$ wie oben definiert ist, jedoch $R^5 \neq H$) mit
ω-Halogenbuttersäuren bzw. den
ω-Halogenbuttersäurenderivaten wie z.B.
4-Brombuttersäurebromid oder
4-Brombuttersäurealkylester oder den entsprechenden
$C_5$-Homologen in an sich bekannter Weise herstellen.

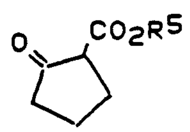

(XXIV)

Als Halogenierungsmittel für Verbindungen der Formel XXI kommen beispielsweise anorganische Säurehalogenide, wie $PCl_5$, $SO_2Cl_2$, $POCl_3$, $SOCl_2$, $PBr_3$, oder Halogen, wie Brom oder Chlor, in Betracht. Von Vorteil ist die Verwendung von $PCl_5$ oder $POCl_3$ in Kombination mit $SO_2Cl_2$ in einem organischen Lösungsmittel. Als Zwischenstufe bildet sich ein Imidhalogenid, das mit den genannten Halogenierungsmitteln und anschließende Hydrolyse unter basischen Bedingungen, vorzugsweise mit wäßrigem Alkalicarbonat weiter zu einer Verbindung der Formel XXII reagiert.

Die Verbindungen der Formel XXII werden nachfolgend in einem polaren aprotischen Lösungsmittel wie beispielsweise einem Alkohol, vorzugsweise Ethanol, oder einer Carbonsäure wie beispielsweise Essigsäure unter Zusatz eines Säureacceptors wie beispielsweise Natriumacetat oder Triethylamin zu einer Verbindung der Formel XXIII, worin Hal die oben genannte Bedeutung besitzt, katalytisch reduziert. Als Katalysator kommen beispielsweise Raney-Nickel oder Palladium bzw. Platin auf Tierkohle in Betracht.

Verbindungen der Formel XXIII können auch direkt oder im Gemisch mit Verbindungen der Formel XXII durch Halogenierung der Verbindungen der Formel XXI beim Einsatz geringerer Mengen der obengenannten Halogenierungsmittel hergestellt werden.

Verbindungen der Formel XXIII können auch erhalten werden, indem man Verbindungen der Formel XVIII in einer Kondesationsreaktion mit Dihalogensäurederivaten der Formel XXV umsetzt,

$$\mathrm{Hal\text{-}CH_2\text{-}\overset{\overset{Hal}{|}}{C}H_2\text{-}CH\text{-}R^6} \qquad \mathrm{XXV}$$

in der Hal Halogen wie z.B. Chlor oder Brom und $R^6$ eine Carbonsäurefunktion wie z.B. eine Carboxy-, Carbalkoxy-, Carbonsäurehalogenidgruppe oder Cyanogruppe bedeuten.

Die Verbindungen der Formel XXIII werden mit Nucleophilen umgesetzt, die das Halogen durch eine Gruppe substituieren, die in weiteren Reaktionschritten eine Aminogruppe freisetzen. Z.B. werden Verbindungen der Formel XXIII mit einem Azid, vorzugsweise Natriumazid in einem apolaren oder polaren Lösungsmittel wie z.B. Dimethylsulfoxid bei Temperaturen zwischen 0°C und 100°C, vorzugsweise zwischen 50° - 100°C umgesetzt. Man erhält Azide der oben genannten Formel XXVI. Die nucleophile Substitution erfolgt unter Walden-Umkehr.

Die Azide lassen sich durch katalytische Hydrierung mit Metallkatalysatoren oder durch Reaktion mit komplexen Hydriden in die Verbindungen der Formel XV überführen. Z.B. wird die Azidgruppe mit Raney-Nickel in einem Alkohol wie z.B. Ethanol bei Raumtemperatur unter Rühren zur Aminoverbindung der Formel XV reduziert.

Die als Zwischenprodukte verwendeten Verbindungen der Formeln XV, XVI, XXI, XXII und XXIII besitzen mehrere chirale Zentren. Sie können somit als Diasteromere vorliegen, die durch Kristallisation oder chromatographische Methoden getrennt werden können. Zur Verwendung können Diastereomere oder Diastereomerengemische bzw. Enantiomere oder Enantiomerengemische kommen.

Die Diastereomeren der Verbindungen der Formeln XV, XVI, XXI, XXII und XXIII sind im folgenden beschrieben, wobei m und Hal wie oben definiert sind:

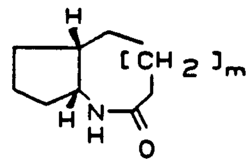

XXIa

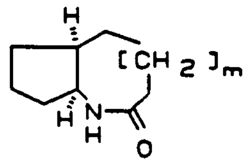

XXIb

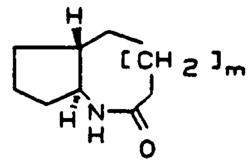

XXIc

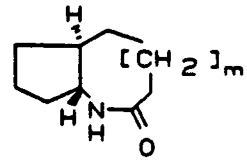

XXId

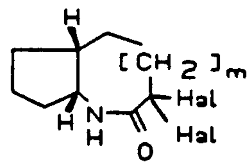

XXIIa

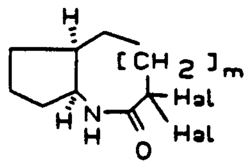

XXIIb

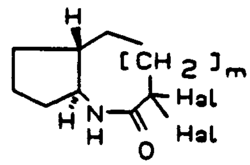

XXIIc

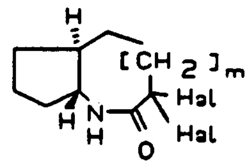

XXIId

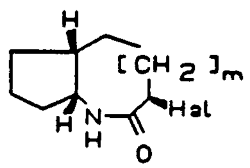

XXIIIa

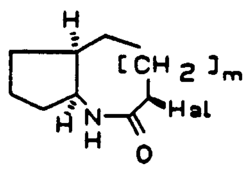

XXIIIb

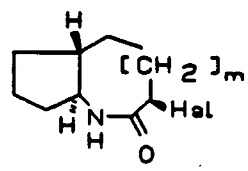

XXIIIc

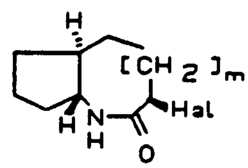

XXIIId

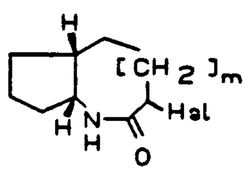

XXIII e

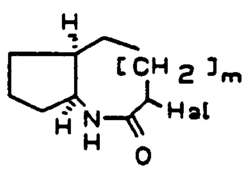

XXIIIf

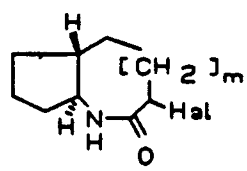

XXIIIg

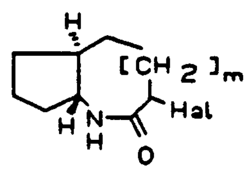

XXIIIh

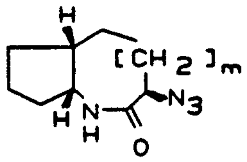
XVIa

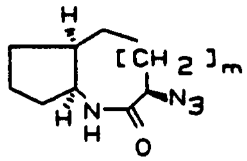
XVIb

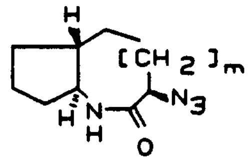
XVIc

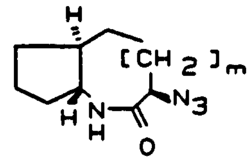
XVId

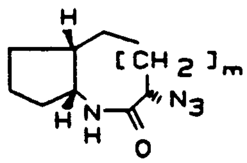
XVIe

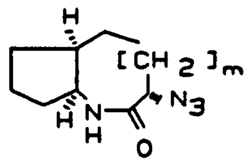
XVIf

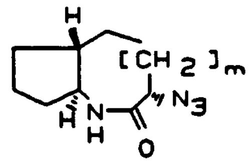
XVIg

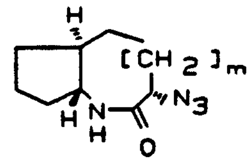
XVIh

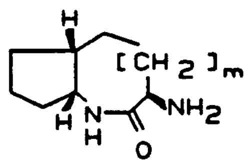
XVa

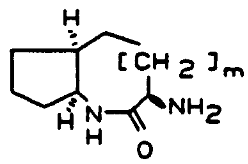
XVb

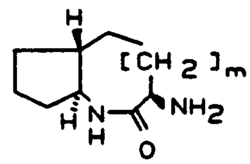
XVc

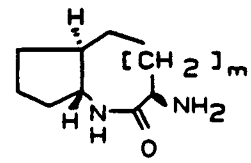
XVd

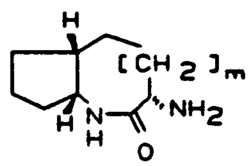
XVe

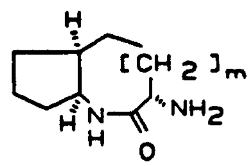
XVf

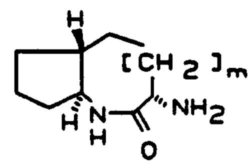
XVg

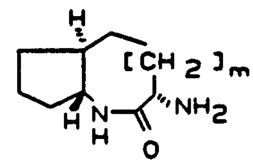
XVh

Vorteilhafterweise trennt man nach Halogenierung der Verbindungen der Formel XXI die Verbindungen der Formel XXII in die Diastereomeren XXII a + b (Bild und Spiegelbild der cis-Verbindung) und XXII c + d (Bild und Spiegelbild der trans-Verbindung). Diese Diastereomere setzt man zweckmäßigerweise getrennt in die weiteren Reaktionen ein, um sich Trennprobleme zu ersparen.

Aus den Verbindungen der Formel XXII a + b erhält man nach den oben beschriebenen Verfahrensweisen die Verbindungen der Formel XXIII a + f (Bild ung Spiegelbild der cis-exo Verbindung) und die Verbindungen der Formel XXIII b + e (Bild und Spiegelbild der cis-endo Verbindung) und aus den Verbindungen der Formel XXII c + d die Verbindungen der Formel XXIII c + h und die Verbindungen der Formel XXIII d + g. Da die Umsetzung der Verbindungen der Formel XXIII mit $NaN_3$ unter Walden-Umkehr erfolgt, erhält man aus

XXIII a + f → XVI e + b
XXIII b + e → XVI f + a
XXIII c + h → XVI g + d
XXIII d + g → XVI h + c

Die nach den obigen Verfahren hergestellten Verbindungen der Formel XV können leicht duch Alkylierung mit Verbindungen der Formel VI in Verbindungen der Formel III überführt werden (Verfahrensvariante c), S.

18)

Dabei wird zweckmäßigerweise die Umsetzung in einem aprotischen apolaren oder polaren Lösungsmittel in Gegenwart einer starken Base durchgeführt, die z.B. Natriumhydrid sein kann. Natriumhydrid deprotoniert den Lactamstickstoff und das gebildetet Salz reagiert dann besonders vorteilhaft mit Verbindungen der Formel VI, in der V = Halogen, insbesondere Brom, bedeutet, zu Verbindungen der Formel III. Die Salzbildung kann in einem Temperaturbereich zwischen -30 ° C und +80 ° C erfolgen, während die weitere Umsetzung bei 0 ° bis 150 ° C geschehen kann.

Unter diesen Bedingungen können auch leicht Verbindungen der Formel XV in Verbindingen der Formel XVII ($R^b \neq H$) überführt werden. Die Verbindungen der Formel XVII ($R^b \neq H$) können anschließend durch katalytische Hydrierung mit Metallkatalysatoren oder durch Reaktion mit komplexen Hydriden zu Verbindungen der Formel III reduziert werden.

Die Verfahrensbedingungen zur Herstellung der Verbindungen der Formel V gemäß Variante d) gleichen weitgehend den der Variante a) bei der Herstellung von Verbindungen der Formel I.

Die Erfindung betrifft weiterhin die Verwendung von Verbindungen der Formel I als Heilmittel bei der Behandlung oder Prophylaxe insbesondere von kardiovaskularen Störungen.

Die Erfindung betrifft ferner pharmazeutische Mittel, die eine Verbindung der Formel I und einen physiologisch unbedenklichen Träger enthalten sowie ein Verfahren zur Herstellung dieser Mittel, das dadurch gekennzeichnet, ist daß man eine Verbindung der Formel I zusammen mit einem physiologisch unbedenklichen Träger und gegebenenfalls weiteren Hilfsstoffen in eine geeignete Darreichungsform bringt.

Die erfindungsgemäßen Verbindungen der Formel I sind Hemmer des Angiotensin-Converting-Enzyme (ACE), das die Umwandlung von Angiotensin I in das pressorisch wirksame Angiotensin II katalysiert. Nach intraduodenaler Gabe an der narkotisierten Ratte wird eine starke Hemmwirkung auf die durch intravenöse Gabe von 310 ng Angiotensin I hervorgerufene Pressorreaktion beobachtet.

Die Verbindungen der Formel I und ihre Salze besitzen lang anhaltende intensiv blutdrucksenkende Wirkung. Sie können zur Bekämpfung des Bluthochddrucks verschiedener Genese oder Herzerkrankungen, z.B. Herzinsuffizienz eingesetzt werden. Auch ihre Kombination mit anderen blutdrucksenkenden, z.B. $Ca^{2+}$-Antagonisten, gefäßerweiternden oder diuretisch wirkenden Verbindungen ist möglich. Typische Vertreter dieser Wirkklassen sich z.B. in Erhardt-Ruschig, Arzneimittel, 2. Auflage, Weinheim 1972 beschrieben. Die Anwendung kann intravenös, subcutan oder peroral erfolgen.

Die Dosierung liegt peroraler Gabe bei 0,01 - 10 mg/kg/Tag, insbesondere bei 0,07 3,0 mg/kg/Tag. Sie kann in schweren Fällen auch erhöht werden, da toxische Effekte bisher nicht beobachete wurden. Auch eine Herabsetzung der Dosis ist möglich und vor allem dann angebracht, wenn gleichzeitig Diuretika oder Calciumantagonisten verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsform gebracht, wie Tabletten, Dragrees, Steckkapseln, wäßrige, alkoholische oder ölige Suspensionen oder wäßrige, alkoholische oder ölige Lösungen. Als inerte Träger kommen z.B. Gummi arabicum, Magnesiumcarbonat, Kaliumphosphat, Milchzucker, Glucose oder Stärke, insbesondere Maisstärke in Betracht. Dabei kann die Zubereitung sowohl als Trocken- als auch als Feuchtgranulat erfolgen. Als öligen Trägerstoffe oder Lösungsmittel kommen beispielsweise pflanzliche oder tierische Öle in Betracht, wie Sonnenblumenöl oder Lebertran.

Zur subcutanen oder intravenösen Applikation werden die aktiven Verbindungen oder deren physiologisch verträglichen Salze, gewünschtenfalls mit den dafür üblichen Substanzen wie Lösungsmittler oder weiteren Hilfsstoffen in Lösung, Suspension oder Emulsion gebracht.

Als Lösungsvermittler für die neuen aktiven Verbindungen und deren Salze kommen z.B. in Frage: Wasser, Propandiol oder Glycerin, daneben auch Zuckerlösungen wie Glucose- oder Mannitlösungen oder auch eine Mischung aus den genannten Lösungsmitteln.

Systemisch, insbesondere aber topisch angewandt, können die erfindungsgemäßen Verbindungen zur Glaukombehandlung dienen.

Die nachfolgenden Beispiele dienen der Erläutertung der Erfindung.

Beispiel I

(3-S, 5a-S,8a-S)-1-Carboxymethyl-3-(S)-[1-(S)-ethoxycarbonyl-3-phenylpropylamino]-2-oxo-perhydrocyclopent[b]azepin (cis-exo)

(1) 4-[2-Oxo-cyclopentyl]-buttersäure

28 g Natriumhydrid (55 %ig) werden in 400 ml trockenem DMF suspendiert und dazu 100 g Cyclopentanon-2-carbonsäureethylester in 400 ml trockenem DMF unter Eiskühlung zugetropft. Nach Ende der Wasserstoffentwicklung wird 4-Brombuttersäureethylester zugetropft. Nach 14 Stunden Rückfluß wird die Lösung einrotiert, der Rückstand in Wasser aufgenommen und mit Methylenchlorid extrahiert. Nach dem Trocknen wird das Methylenchlorid abgedampft und der Rückstand destilliert.

Ausbeute: 144 g 4-[1-Ethoxycarbonyl-2-oxo-cyclopentyl]-buttersäureethylester, Kp: 139-145 °C (0,09 mm Hg)

Oben erhaltene 136 g werden in 312 ml Eisessig gelöst, mit 77 ml konzentrierter wäßriger HCl versetzt und 16 Stunden refluxiert. Nach üblicher Aufarbeitung wird der Rückstand destilliert.

Ausbeute: 52 g Kp: 142 ° - 158 °C/0,008 mm Hg

(2) 4-(2-Oxo-cyclopentyl)-buttersäureethylester

17 g aus Beispiel I (1) werden in 170 ml ethanolischer HCl 14 Stunden stehen gelassen. Nach dem Aufarbeiten wird der Rückstand destilliert.

Ausbeute: 15 g Kp: 98 ° - 104 °C/0,1 mm Hg

(3) 4-(2-Hydroximino-cyclopentyl)-buttersäureethylester

13 g aus Beispiel I (2) werden zu einer 60 °C warmen Lösung von 6,8 g Hydroxylaminhydrochlorid und 6.5 g Natriumacetat in 35 ml Wasser zugetropft. Nach 80 min Rühren wird nach dem Abkühlen mit Methylenchlorid extrahiert. Der nach dem Eindampfen zurückbleibende Rückstand wird über Kieselgel mit Cyclohexan/Essigester 7:3 als Laufmittel getrennt.

Ausbeute: 12 $R_f$: 0,3 (Kieselgel; $CH_2Cl_2/CH_3OG$ 95:5)

(4) 4-(2-Amino-cyclopentyl)-buttersäureethylester

12,8 g Oxim aus Beispiel I (3) werden in 128 ml Eisessig gelöst, 1,3 g Platinoxid zugegeben und im Autoklaven 8 Stunden bei 10 Atmosphären Wasserstoffdruck hydriert. Nach dem Absaugen des Katalysators wird eingeengt im Vakuum in Methylenchlorid aufgenommen und mit 2 n wäßriger HCl extrahiert. Die HCl-Phase wird mit Natronlauge basisch gestellt und mit Methylenchlorid extrahiert.

Ausbeute: 3,4 g

(5) 4-(2-Amino-cyclopentyl)-buttersäure

3,4 g Aminester aus Beispiel I (4) werden in 20 ml 6n-wäßriger HCl gelöst und 5 Stunden refluxiert. Nach dem Abkühlen wird mit $CH_2Cl_2$ extrahiert und die HCl-Phase einrotiert. Der Rückstand wird mehrere Male mit Toluol eingedampft und mit Essigesster/Isopropanol versetzt.

Ausbeute: 2,7 g Fp: 174-177 °C (HCl-Salz)

(6) 2-Oxo-perhydrocyclopent[b]azepin

4,1 g Aminosäurehydrochlorid aus Beispiel I (5) werden in 40 ml Methylenchlorid aufgeschlämmt, dazu unter Eiskühlung 12,8 ml N-Ethylmorpholin zugegeben und dazu 12,8 ml 50 % n-Propanphosphonsäureanhydrid in Methylenchlorid bei 0 °C zugetropft. Die Lösung steht 14 Stunden bei Raumtemperatur. Es wird dann mit 200 ml $CH_2Cl_2$ verdünnt, mit Wasser, 2n HCl und gesättigter $NaHCO_3$-Lösung extrahiert. Die $CH_2Cl_2$-Phase wird getrocknet und eingedampft.

Ausbeute: 2,6 g Fp: 131-133 °C Umkristallisation aus Petrolether Fp: 133 °-135 °C

(7) 2-Oxo-3,3-dichlor-perhydrocyclopent[b]azepin

1,0 g Lactam aus Beispiel I (6) werden im 28 ml Xylol gelöst und unter $N_2$ 4,1 g $PCl_5$ zugegeben, es wird 30 Minuten auf 50 °C, dann weitere 30 Minuten auf 90 °C erhitzt. Nach dem Abkühlen wird im Vakuum eingeengt. Der Rückstand wird mit gesättigter wäßriger $Na_2CO_3$-Lösung versetzt, dann mit $H_2O$ verdünnt. Das Gemisch wird mit $CH_2Cl_2$ extrahiert, die Methylenchloridphase getrocknet und eingeengt. Der Rückstand wird mit Diisopropylether verrührt.

Ausbeute: 1,0 g Fp: 135-140 °C

Diese 1 g stellen ein Gemisch dar, das über Kieselgel mit Cyclohexan/Essigester 4:1 getrennt wird. Das zuerst eluierte Produkt ist die cis-Verbindung (5a-RS,8aRS)-2-oxo-3,3-dichlor-perhydrocyclopent[b]azepin (A),

Fp: 132 ° - 135 °C;

$^1H$-NMR ($CDCl_3$): $H_{8a}$: 4,08 ppm (ddd, J ~ 2,3 + 5,5 + 5,5 Hz).

Die danach eluierte Verbindung ist das trans-Produkt (5a-RS,8a-SR)-2-oxo-3,3-dichlor-perhydrocyclopent-[b]azepin (B), Fp: 194-196 °C;

$^1$H-NMR($CDCl_3$): $H_{8a}$: 3,6 ppm
(ddd, J ~ 7,5 + 10 + 10 Hz)
(A) und (B) stellen Razemate dar.

(8) (5a-RS,8a-RS)-2-Oxo-3-chlor-perhydrocyclopent[b]azepin (cis)

100 mg der cis-Verbindung (A) aus Beispiel I (7) werden in 10 ml Eisessig, der 55 mg wasserfreies Natriumacetat enthält, gelöst, mit 10 mg Pd/C (5 %ig) versetzt und bei Raumtemperatur unter Normaldruck hydriert bis 1 Moläquivalent Wasserstoff aufgenommen wurde. Es wurde vom Katalysator abfiltriert, die Lösung eingeengt, mit gesättigter wäßriger $NaHCO_3$-Lösung versetzt, mit $CH_2Cl_2$ extrahiert und die $CH_2Cl_2$-Phase nach dem Trocknen eingeengt. Der Rückstand besteht aus 2 diastereomeren Monochlorverbindungen, die über Kieselgel mit Cyclohexan/Essigester 1:1 getrennt wurden.

Das zuerst eluierte Produkt ist die
"cis-exo"-Verbindung (A)
(3-RS,5a-RS,8a-RS)-2-Oxo-3-chlor-perhydrocyclopent[b]-azepin
Fp: 112 ° - 113 °C
$^1$H-NMR ($CDCl_3$): $H_3$: 4,25 ppm (Multiplett)
$H_{8a}$: 4,52 ppm (Multiplett)

Die nachfolgend eluierte Verbindung ist die
"cis-endo"-Verbindung (B)
(3-SR,5a-RS,8a-RS)-2-oxo-3-chlor-perhydrocyclopent[b] azepin
Fp: 130 ° - 131 °C
$^1$H-NMR ($CDCl_3$): $H_3$: 3,92 ppm (Multiplett)
$H_{8a}$: 4,71 ppm (dd)

(9) (3-SR,5a-SR,8a-RS)-2-Oxo-3-chlor-perhydrocyclopent[b]azepin (trans)

100 mg der trans-Verbindung (B) aus Beispiel I (7) werden wie in Beispiel I (8) beschrieben, hydriert. Es entsteht eine Monochlor-Verbindung.
Fp: 180 ° - 183 °C
$^1$H-NMR($CDCl_3$): $H_3$: 3,3 ppm (Multiplett)
$H_{8a}$: 4,65 ppm (dd)

Die relative Konfiguration wurde durch $^1$H-NMR-NOE (Nuclear-Overhauser-Effekt) Messungen bestimmt.

(10) (3-SR,5a-RS,8a-RS)-2-Oxo-3-azido-perhydrocyclopent[b]azepin (cis-endo)

100 mg der "cis-exo"-Verbindung (A) aus Beispiel I (8) werden in 2 ml DMSO gelöst und 41,5 mg Natriumazid zugegeben. Es wird 6 Stunden auf 80 °C erhitzt. Nach dem Abkühlen wird mit Wasser versetzt und anschließend mit Methylenchlorid extrahiert. Nach dem Trocknen wird die $CH_2Cl_2$-Phase eingeengt. Der Rückfluß wird mit Diisopropylether verrührt.
Ausbeute: 77 mg Fp: 108-110 °C
$^1$H-NMR ($CDCl_3$): $H_3$: 3,85 ppm (Multiplett)
$H_{8a}$: 4,02 ppm (dd)

(11) (3-RS,5a-RS,8a-RS)-2-Oxo-3-azido-perhydrocyclopent[b]azepin (cis-exo)

100 mg der "cis-endo"-Verbindung (B) aus Beispiel I (8) werden wie im Beispiel I (10) beschrieben umgesetzt.
Ausbeute: 81 mg Fp: 68-69 °C
$^1$H-NMR ($CDCl_3$): $H_3$: 4,12 (Multiplett)
$H_{8a}$: 4,27 (Multiplett; nach H/D-Austausch dd)

(12) (3-RS,5a-SR,8a-RS)-2-Oxo-3-azido-perhydrocyclopent[b]azepin (trans)

1,5 g der trans-Verbindung aus Beispiel I (9) werden wie in Beispiel I (10) beschrieben umgesetzt.
Ausbeute: 1,4 g Fp: 116 ° -118 °C
$^1$H-NMR($CDCl_3$): $H_3$: 3,65 ppm (Multiplett)
$H_{8a}$: 4,38 ppm (Dublett)

(13) 3-SR,5a-RS,8a-RS)-1-Tert-butoxycarbonylmethyl-2-oxo-3-azido-perhydrocyclopent[b]azepin (cis-endo)

122 mg Natriumhydrid-Suspension (55-60 %) werden mit n-Hexan gewaschen und das Natriumhydrid in 27 ml trockenen DMF aufgeschlämmt. Bei 0 °C werden dann 900 mg der "cis-endo"-Azidverbindung aus Beispiel I (10) zugetropft und 45 min nachgerührt. Anschließend werden bei 0 °C 994 mg Bromessigsäuretert.-butylester in trockenem DMF zugetropft; man läßt auf Raumtemperatur kommen und rührt über Nacht nach. Das DMF wird abrotiert, der Rückstand in 50 ml Methylenchlorid verteilt. Die Wasserphase wird weitere Male mit Methylenchlorid extrahiert. Nach dem Trocknen werden die Methylenchloridphasen eingeengt und der Rückstand mit Petrolether verrührt.
Ausbeute: 0,65 g Fp: 102 ° - 105 °C

$^1$H-NMR ($CDCl_3$): 1,45 ppm (Singulett, 9H)
4,07 ppm (Multiplett)
4,16 ppm (Quartett)

(14) (3-RS,5a-RS,8a-RS)-1-Tert.butoxycarbonylmethyl-2-oxo-3-azido-perhydrocyclopent[b]azepin (cis-exo)

Analog Beispiel I (13) werden 900 mg der "cis-exo"-Azidverbindung aus Beispiel I (11) umgesetzt.
Fp: 75 ° - 77 °C
$^1$H-NMR ($CDCl_3$): 1,45 ppm (Singulett, 9H)
3,74 ppm (Multiplett, 1H)
3,78 ppm (Dublett, 1H)
3,95 ppm (Multiplett, 1H)
4,45 ppm (Dublett, 1H)

(15) (3-RS,5a-SR,8a-RS)-1-Tert.-butoxycarbonylmethyl-2-oxo-3-azido-perhydrocyclopent[b]azepin (trans)

Analog Beispiel I (14) werden 600 mg der trans-Azidverbindung aus Beispiel I (12) umgesetzt.
Ausbeute: 0,6 g Öl
$^1$H-NMR ($CDCl_3$): 1,45 ppm (Singulett, 9H)
3,98 ppm (Dublett, 1H)
4,08 ppm (Multiplett, 1H)
4,22 ppm (Dublett, 1H)
4,56 ppm (dd, 1H)

(16) 3-SR,5a-RS,8a-RS)-1-Tert.-butoxycarbonymethyl-2-oxo-3-amino-perhydrocyclopent[b]azepin (cis-endo)

600 mg Azidverbindung aus Beispiel I (13) werden in 10 ml Ethanol gelöst, mit 100 mg Pd/C (10 %) versetzt und bei Raumtemperatur und Normaldruck hydriert. Nach dem Absaugen des Katalysators wird eingeengt.
Ausbeute: quantitativ
$^1$H-NMR ($CDCl_3$): 3,73 ppm (dd, 1H)
4,0 ppm (Dubulett, 1H)
4,14 ppm (Quartett, 1H)
4,28 ppm (Dublett, 1H)

(17) (3-RS,5a-RS,8a-RS)-1-Tert.-butoxycarbonylmethyl-2-oxo-3-amino-perhydrocyclopent[b]azepin (cis-exo)

Analog Beispiel I (16) werden 205 mg der "cis-exo"-Azidverbindung aus Beispiel I (14) hydriert.
Ausbeute. 104 mg
$^1$H-NMR ($CDCl_3$): 3,65 ppm (Multiplett, 2H)
3,82 ppm (Dublett, 1H)
4,39 ppm (Dublett, 1H)

(18) (3-RS,5a-SR,8a-RS)-1-Tert.-butoxycarbonylmethyl-2-oxo-3-amino-perhydrocyclopent[b]azepin (trans)

Analog beispiel I (16) werden 492 mg der trans-Azidverbindung aus Beispiel I (15) hydriert.
Ausbeute: 480 mg Fp: 70 ° - 74 °C
$^1$H-NMR ($CDCl_3$): 3,85-4,03 ppm (Multiplett, 2H)
3,98 ppm (Dublett, 1H)
4,18 ppm (Dublett, 1H)

(19) (3-RS,5a-RS,8a-RS)-1-Tert.-butoxycarbonylmethyl-2-oxo-3-[1-(S)-ethoxycarbonyl-3-phenyl -propyl-amino]-perhydrocyclopent[b]azepin (cis-exo)

180 mg Amin aus Beispiel I (17) und 67 mg Triethylamin werden in 0,6 ml trockenem Methylchlorid gelöst. Dazu werden in 225 mg (D)-2-Trifluormethylsulfonyloxy-4-phenyl-buttersäureethylester in 0,3 ml trockenem Methylchlorid zugegeben und 14 Stunden bei 5°C gelassen. Danach wird mit Wasser gewaschen und die Methylenchloridphase nach dem Trocknen eingeengt.
Ausbeute: 0,33 g Diastereomerengemisch

(20) (3-S,5a-S,8a-S)-1-Tert.-butoxycarbonylmethyl-2-oxo-3-[1-(S)-ethoxycarbonyl-3-phenylpropylamino]-perhydrocyclopent[b]azepin (cis-exo)

Das Diastereomerengemisch aus Beispiel I (19) wird über Kieselgel mit Cyclohexan/Essigester 65:35 säulenchromatograhisch getrennt. Es werden 2 Diastereomere erhalten. Zuerst eluiert man das
Diastereomer I: $[\alpha]_D^{20}$ = +2,5 ° (Methanol), danach
Diastereomer II: $[\alpha]_D^{20}$ = -11,1 ° (Methanol)
Das Diastereomer I sollte die

(3-R,5a-R,8a-R)-Konfiguration und das Diastereomer II die (3-S,5a-S,8a-S)-Konfiguration besitzen.
Die Konfiguration wurde aus den biologischen Ergebnissen der Endprodukte (Verbindungen aus Beispiel I (21) bzw. Beispiel II) extrapoliert.

(21) (3S,5a-S,8a-S)-1-Carboxymethyl-2-oxo-3-[1-(S)-ethoxycarbonyl-3-phenylpropylamino]-perhydrocyclopent[b]azepin (cis-exo)

90 mg Diastereomer II aus Beispiel I (20) werden in 0,4 ml Trifluoressigsäure gelöst und 140 min bei Raumtemperatur stehen gelassen. Danach wird die Trifluoressigsäure abgedampft, der Rückstand mehrere Male mit Toluol einrotiert. Der Rückstand wird in Methanol/Wasser aufgenommen und mit Ionenaustauscher (IRA 93, Acetatform) auf pH 4,2 gebracht. Es wird vom Ionenaustauscher abgesaugt, die Lösung mit Toluol einrotiert und der Rückstand mit Essigester und Diethylether weitere Male einrotiert.

Ausbeute: 61 mg $[\alpha]_D^{20}$ = -4.0° (c = 0,5, Methanol)

Beispiel II

(3-R,5a-R,8a-R)-1-Carbonylmethyl-2-oxo-3-[1-(S)-ethoxycarbonyl-3-phenylpropylamino]-perhydrocyclopent-[b]azepin

Analog Beispiel I (21) werden 90 mg Diastereomer I aus Beispiel I (20) umgesetzt.
Ausbeute: 67 mg Fp: 95 ° - 103 °C; $[\alpha]_D^{20}$ : +1,3 ° (c = 0,6 Methanol)

Beispiel III

(3-S,5a-R,8a-R)-1-Carboxymethyl-2-oxo-3-[1-(S)-ethoxycarbonyl-3-phenylpropylamino]-perhydrocyclopent-[b]azepin (cis endo)

(1) (3-S,5a-R,8a-R)-1-Tert.-butoxycarbonylmethyl-2-oxo-3-[1-(S)-ethoxycarbonyl-3-phenylpropyl-amino]-perhydrocyclopent[b]azepin

Analog Beispiel I (19) werden 580 mg Amin aus Beispiel I (16) umgesetzt.
Ausbeute: 970 mg Diastereomerengemisch, das analog Beispiel I (20) säulenchromatographisch getrennt wird. Diastereomer I wird zuerst eluiert, dann Diastereomer II.

Diastereomer I: $[\alpha]_D^{20}$ = -5,8 ° ($CHCl_3$)
Diastereomer II: $[\alpha]_D^{20}$ = +7.0 ° ($CHCl_3$)

Diastereomer I sollte aufgrund der biologischen Daten des Endproduktes (Verbindung aus Beispiel IV) die (3-R,5a-S,8a-S)-Konfiguration besitzen und Diastereomer II aufgrund der biologischen Daten des Endproduktes (Verbindung aus Beispiel III (2)) die (3-S,5a-R,8a-R)-Konfiguration.

(2) (3-S,5a-R,8a-R)-1-Carboxymethyl-2-oxo-3-[1-(S)-ethoxycarbonyl-3-phenylpropylamino]-perhydrocyclopent[b]azepin

Analog Beispiel I (21) werden 360 mg Diasereomer II aus Beispiel III (1) umgesetzt.

Ausbeute: 251 mg $[\alpha]_D^{20}$ = +40,6 ° (Methanol)

$^1$H-NMR ($CDCl_3$): 2,7 ppm (Triplett, 2H)
3,34 ppm (Triplett, 1H)
3,72 ppm (Triplett, 1H)
4,05-4,35 ppm (Multiplett, 5H)

Beispiel IV

(3-R,5a-R,8a-S)-1-Carboxymethyl-2-oxo-3-[1-(S)-ethoxycarbonyl-3-phenylpropylamino]-perhydrocyclopent-[b]azepin

Analog Beispiel I (21) werden 370 mg Diastereomer I aus Beispiel III (1) umgesetzt.
Ausbeute: 222 mg $[\alpha]_D^{20}$ = -7,4 ° (Methanol)

$^1$H-NMR($CDCl_3$): 2,7 ppm (Multiplett, 2H)
3,21 ppm (Triplett, 1H)
3,6 ppm (Triplett, 1H)
3,95 - 4,25 ppm (Multiplett, 5H)

Beispiel V

(3-S,5aR,8a-S)-1-Carboxymethyl-2-oxo-3-[1-(S)-ethoxycarbonyl-3-phenylpropylamino]-perhydrocyclopent[b]-azepin (trans)

(1) (3-S,5a-R,8a-S)-1-Tert.-butyloxycarbonylmethyl-2-oxo-3-[1-(S)-ethoxycarbonyl-3-phenylpropylamino]-perhydrocyclopent[b]azepin (trans)

Analog Beispiel I (19) werden 451 mg Amin aus Beispiel I (18) umgesetzt.

Ausbeute: 811 mg Diastereomerengemisch, das analog Beispiel I (20) säulenchromatographisch getrennt wird. Diastereomer I wird zuerst eluiert, Diastereomer II danach.

Diastereomer I : $[\alpha]_D^{20}$ = +0.9 ° (c=0,94, Methanol)

Diastereomer II: $[\alpha]_D^{20}$ = -22,9 ° (c=0,71, Methanol)

Diastereomer I sollte aufgrund der biologischen Daten des Endproduktes (Verbindung aus Beispiel VI) die (3-R,5a-S,8a-R)-Konfiguration besitzen und Diastereomer II die (3-S,5a-R,8a-S)-Konfiguration aufgrund der biologischen Daten des Endproduktes aus Beispiel V (2).

(2) (3-S,5a-R,8a-S)-1-Carboxymethyl-2-oxo-3-[1-(S)-ethoxycarbonyl-3-phenylpropylamino]-perhydrocyclopent[b]Azepin (trans)

Analog Beispiel I (21) werden 194 mg Diastereomer II aus Beispiel V (1) umgesetzt.

Ausbeute: 85 mg $[\alpha]_D^{20}$ = -8,9 ° (c = 0,67, Methanol)

$^1$H-NMR ($CDCl_3$): 2,87 ppm (Multiplett, 2H)
3,32 ppm (Multiplett, 1H)
3,7-4,0 ppm (Multiplett, 2H)
4,05-4,3 ppm (Multiplett, incl. Quartett, 3H)
4,48 ppm (breites Dublett, 1H)

Beispiel VI

(3-R,5a-S,8a-R)-1-Carboxymethyl-2-oxo-3-[1-(S)-ethoxycarbonyl-3-phenylpropylamino]-perhydrocyclopent-[b]azepin (trans)

Analog Beispiel I (21) werden 245 mg Diastereomer I aus Beispiel V (1) umgesetzt.

Ausbeute: 114 mg $[\alpha]_D^{20}$ = + 37,6 ° (c= 0,95, Methanol)

$^1$H-NMR ($CDCl_3$): 2,7-2,95 ppm (Multiplett, 2H)
3,26 ppm (Multiplett, 1H)
3,73-3,86 ppm (Multiplett, 2H)
4,08 ppm (Multiplett, 1H)
4,22-4,4 ppm (Multiplett, 3H)

Beispiel VII

(3-S,5a-R,8a-S)-1-Carboxymethyl-2-oxo-3-[1-(S)-ethoxycarbonyl-3-cyclohexylpropylamino]-perhydrocyclopent[b]azepin (trans)

(3-S,5a-R,8a-S)-1-Tert.-butoxycarbonylmethyl-2-oxo-3-[1-(S)-ethoxycarbonyl-3-cyclohexylpropylamino]-perhydrocyclopent[b]azepin

200 mg Diastereomer II aus Beispiel V (1) werden in 20 ml Methanol gelöst, dazu 150 mg Rhodium auf Kohle (5 %) gegeben und unter 50 Atmosphären Wasserstoffdruck 20 Stunden bei 100 °C hydriert.

Ausbeute: 169 mg $[\alpha]_D^{20}$ = -28,2 ° (c=1,4, Methanol)

(2) (3-S,5a-R,8a-S)-1-Carboxymethyl-2-oxo-3-[1-(S)-ethoxycarbonyl-3-cyclohexylpropylamino]-perhydrocyclopent[b]azepin

135 mg der Verbindung aus Beispiel VII (1) werden analog Beispiel I (21) umgesetzt. Es wird mit ethanolischer HCl das Hydrochlorid gemacht.

Ausbeute: 87 mg $[\alpha]_D^{20}$ = -17,2° (c = 0,5, Methanol) Fp: 170 °C (Zers.)

Beispiel VIII

(3-S,5a-R,8a-R)-1-Carboxymethyl-2-oxo-3-[1-(S)-ethoxycarbonyl-n-butylamino]-perhydrocyclopent[b]-azepin(cis-endo)

(1) (3-S,5a-R,8a-R)-1-Tert.-butoxycarbonylmethyl-2-oxo-3-[1-(S)-ethoxycarbonyl-n-butylamino]-perhydrocyclopent[b]azepin

200 mg des Racemats aus Beispiel I (16) werden analog dem Beispiel I (19) umgesetzt mit der Ausnahme, daß anstelle von D-2-Trifluoromethylsulfonyloxy-4-phenyl buttersäureethylester D-2-Trifluoromethylsulfonyloxy-n-pentancarbonsäureethylester genommen wird.
Ausbeute: 310 mg Diastereomerengemisch, das säulenchromatographisch analog Beispiel I (20) über Kieselgel getrennt wird. Zuerst wird das Diastereomer I eluiert, dann Diastereomer II.

Diastereomer I besitzt wahrscheinlich die (3-R,5a-S,8a-S)-Konfiguration und Diastereomer II die (3-S,5a-R,8a-R)-Konfiguration in Analogie zu Beispiel V (1)
m/e = 410

(2) (3-S,5a-R,8a-R)-1-Carboxymethyl-2-oxo-3-[1-(S)-ethoxycarbonyl-n-butylamino]-perhydrocyclopent[b]-azepin

130 mg Diastereomer II aus Beispiel VIII (l) werden analog Beispiel I (21) umgesetzt.
Ausbeute: 90 mg m/e: 354

Beispiel IX

(3-R,5a-S,8a-S)-1-Carboxymethyl-2-oxo-3-[1-(S)-ethoxycarbonyl-n-butylamino]-perhydrocyclopent[b]-azepin(cis-endo)

120 mg Diastereomer I aus Beispiel VIII (1) werden analog Beispiel I (21) umgesetzt.
Ausbeute: 85 mg m/e: 354

Beispiel X

Setzt man in Beispiel VIII (1) anstelle der "cis-endo"-Verbindung aus Beispiel I (16) die "cis-exo"-Verbindung aus Beispiel I (17) oder die trans-Verbindung aus Beispiel I (18) ein und arbeitet wie in Beispiel VIII und IX angegeben, so erhält man die Verbindung (3-S,5a-S,8a-S)-1-Carboxymethyl-2-oxo-3-(1-(S)-ethoxycarbonyl-n-butylamino]-perhydrocyclopent[b]azepin (cis-exo) und das (3-R,5a-R,8a-R)-Diastereomere oder die Verbindung (3-S,5a-R,8a-S)-1-Carboxymethyl-2-oxo-3-[1-(S)-ethoxycarbonyl-n-butylamino]-perhydrocyclopent[b]azepin und das (3-R,5a-S,8a-R)-Diastereomere.

Beispiel XI

(3-S,5a-R,8a-R)-1-Carboxymethyl-2-oxo-3-[1-(S)-carboxy-3-phenylpropylamino]-perhydrocyclopent[b]-azepin(cis-endo)

277 mg der Verbindung aus Beispiel III (2) werden in 4,5 ml Wasser suspendiert. Dazu werden 0,465 ml 4 n wäßrige KOH-Lösung gegeben und 14 Stunden bei Raumtemperatur stehen gelassen. Es wird filtriert und das Filtrat mit konzentrierter wäßriger HCl auf pH = 1,5 gestellt. Der Niederschlag wird abgesaugt, mit Wasser gewaschen und über $P_2O_5$ getrocknet.

Ausbeute: 223 mg Fp: 243 ° - 246 °C $[\alpha]_D^{20}$ = +51,5 ° (c = 1, Methanol)

$^1$H-NMR(DMSO-$d_6$): 2,64 ppm (Multiplett, 2H)
3,13 ppm (Multiplett, 1H)
3,93 ppm (dd)

Beispiel XII

(3-S,5a-R,8a-S)-1-Carboxymethyl-2-oxo-3-[1-(S)-carboxy-3-phenylpropylamino]-perhydrocyclopent[b]-azepin(trans)

Analog Beispiel XI werden 200 mg der Verbindung aus Beisiel V (2) umgesetzt.

Ausbeute: 125 mg Fp: 209 ° - 211 °C, $[\alpha]_D^{20}$ : - 11 ° (c=1, Methanol)
$^1$H-NMR(DMSO-$d_6$): 2,7 ppm (Multiplett, 2H)
3,03 ppm (Multiplett, 1H)
3,48 ppm (Dublett, 1H)
3,98 ppm (dd, 2H)
4,72 ppm (Multiplett, 1H)
m/e: 533 (M + $H^+$ + 2 TMS) nach Silylierung (TMS = Trimethylsilyl)

Beispiel XIII

3-S,5a-S,8a-S)-1-Carboxymethyl-2-oxo-3-[1-(S)-carboxy-3-phenylpropylamino]-perhydrocyclopent[b]azepin (cis-exo)

Analog Beispiel XI werden 200 mg der Verbindung aus Beispiel I (21) umgesetzt.
Ausbeute: 114 mg
m/e: 533 (M + $H^+$ + 2 TMS) nach Silylierung
(TMS = Trimethylsilyl)

Beispiel XIV

(3-S,5a-R,8a-S)-1-Carboxymethyl-2-oxo-3-[1-(S)-carboxy-3-cyclohexylpropylamino]-perhydrocyclopent[b]-azepin(trans)

Analog Beispiel XI werden 78 mg der Verbindung aus Beispiel VII (2) umgesetzt.
Ausbeute: 40 mg $[\alpha]_D^{20}$ = -12,1 ° (c=0,4, Methanol)
m/e: 538 (M + $H^+$ + 2 TMS) nch Silylierung
(TMS = Trimethylsilyl)

Beispiel XV

Analog Beispiel XI werden aus den Verbindungen aus
a) Beispiel VIII (2)
b) Beispiel X (3-S-cis-exo-Diastereomer)
c) Beispiel X (3-S-trans-Diastereomer)
die Verbindungen
a) (3-S,5a-R,8a-R)-1-Carboxymethyl-2-oxo-3-[1-(S)-carboxy-n-butylamino]-perhydrocyclopent[b]azepin,
b) (3-S,5a-S,8a-S)-1-Carboxymethyl-2-oxo-3-[1-(S)-carboxy-n-butylamino]-perhydrocyclopent[b]azepin und
c) (3-S,5a-R,8a-S)-1-Carboxymethyl-2-oxo-3-[1-(S)-carboxy-n-butylamino]-perhydrocyclopent[b]azepin
erhalten.

Beispiel XVI

In analoger Weise zu den Beispielen I - XV kann man die entsprechenden Perhydrocyclopent[b]azocin-Derivate herstellen, wie z.B. (3-S,6a-R,9a-R)-1-Carboxymethyl-2-oxo-3-(1-(S)-Carboxy-3-phenylpropylamino)-perhydro-2H-cyclopent[b]-azocin entsprechend Beispiel I, Beispiel III und Beispiel XI.

**Patentansprüche**

**Patentansprüche für folgende Vertragsstaaten : AT, BE, CH, DE, FR, GB, IT, LI, LU, NL, SE**

**1.** Verbindungen der Formel I,

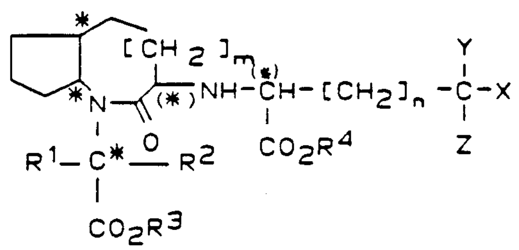

(I)

in welcher
m = 1 oder 2 und
n = 0, 1 oder 2 bedeuten,

$R^1$ und $R^2$ gleich oder verschieden sind und Wasserstoff, ($C_1$-$C_6$)-Alkyl, das gegebenenfalls monosubstituiert sein kann durch Hydroxy, Mercapto, ($C_1$-$C_2$)-Alkoxy, ($C_1$-$C_2$)-Alkylmercapto, Carboxy, ($C_1$-$C_2$)-Alkxoycarbonyl, 3-Indolyl, Imidazolyl, Carbamoyl, Amino oder Guanidino, ($C_2$-$C_6$)-Alkenyl, ($C_3$-$C_9$)-Cycloalkyl, ($C_3$-$C_9$)-Cycloalkenyl, ($C_3$-$C_7$)-Cycloalkyl-($C_1$-$C_4$)-alkyl, ($C_6$-$C_{12}$)-Aryl, teilhydriertes ($C_6$-$C_{12}$)-Aryl oder ($C_6$-$C_{12}$)-Aryl-($C_1$-$C_4$)-alkyl, das im Arylteil eine Hydroxygruppe tragen kann, bedeuten,

$R^3$ und $R^4$ gleich oder verschieden sind und Wasserstoff, ($C_1$-$C_6$)-Alkyl, ($C_2$-$C_6$)-Alkenyl oder ($C_6$-$C_{12}$)-Aryl-($C_1$-$C_4$)-Alkyl, das im Arylteil durch Methoxy oder Nitro monosubstituiert sein kann, bedeuten,

Y Wasserstoff oder Hydroxy,

Z Wasserstoff oder

Y und Z zusammen Sauerstoff bedeuten und

X für ($C_1$-$C_6$)-Alkyl, das durch Amino, Acylamino, ($C_1$-$C_4$)-Alkylamino und/oder Di-($C_1$-$C_4$)-Alkylamino substituiert sein kann, ($C_2$-$C_6$)-Alkenyl, ($C_5$-$C_9$)-Cycloalkyl,

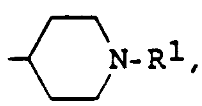

($C_6$-$C_{12}$)-Aryl, das durch ($C_1$-$C_4$)-Alkyl, ($C_1$-$C_4$)-Alkoxy, Hydroxy, Halogen, Nitro Amino, ($C_1$-$C_4$)-Alkylamino, Di-($C_1$-$C_4$)-Alkylamino und/oder Methylendioxy mono-, di- oder trisubstituiert sein kann, oder Indol-3-yl steht, wobei in den obengenannten Resten $R^1$, $R^2$ und X freies Hydroxy, Mercapto, Carboxy, Amino oder Guanidino gegebenenfalls durch in der Peptidchemie übliche Schutzgruppen geschützt sind, sowie deren physiologisch unbedenklichen Salze.

**2.** Verbindungen der Formel I gemäß Anspruch 1, dadurch gekennzeichnet, daß
m = 1 und
n = 1 ist,

$R^1$ Wasserstoff, ($C_1$-$C_3$)-Alkyl, ($C_3$-$C_6$)-Cycloalkyl ($C_3$-$C_6$)-Cycloalkyl-($C_1$-$C_2$)-alkyl oder Allyl,

$R^2$ Wasserstoff, ($C_1$-$C_3$)-Alkyl, ($C_3$-$C_6$)-Cycloalkyl, ($C_3$-$C_6$)-Cycloalkyl-($C_1$-$C_2$)-alkyl oder Allyl,

$R^3$ Wasserstoff, ($C_1$-$C_4$)-Alkyl, Benzyl oder 4-Methoxybenzyl,

$R^4$ Wasserstoff, ($C_1$-$C_4$)-Alkyl, Benzyl oder 4-Methoxybenzyl,

X Phenyl oder mit Fluor und/oder Chlor mono- oder disubstituiertes Phenyl, Methyl, Cyclohexyl oder Aminoethyl,

Y Wasserstoff oder Hydroxy und

Z Wasserstoff bedeuten, oder

Y und Z zusammen für Sauerstoff steht.

**3.** Verbindungen der Formel I gemäß einem der Ansprüche 1 und 2, dadurch gekennzeichnet, daß
m = 1,
n = 1,
$R^1$, $R^2$, und $R^3$ jeweils Wasserstoff,
$R^4$ Wasserstoff oder Ethyl,
Y und Z jeweils Wasserstoff und
X Phenyl bedeuten.

**4.** Verbindungen der Formel I gemäß Anspruch 1, dadurch gekennzeichnet, daß die mit einem einem Stern [(*)] bezeichneten chiralen Kohlenstoffatome S-Konfiguration besitzen.

**5.** 1-Carboxymethyl-3-(S)-[1-(S)-ethoxycarbonyl-3-phenyl-propylamino]-2-oxo-perhydrocyclopent[b]azepin,
1-Carboxymethyl-3-(S)-[1-(S)-carboxy-3-phenylpropylamino]-2-oxo-perhydrocyclopent[b]azepin,
1-Carboxymethyl-3-(S)-[1-(S)-ethoxycarbonyl-butylamino]-2-oxo-perhydrocyclopent[b]azepin,
1-Carboxymethyl-3-(S)-[1-(S)-carboxy-butylamino]-2-oxo-perhydrocyclopent[b]azepin,
1-Carboxymethyl-3-(S)-[1-(S)-ethoxycarbonyl-5-aminopentylamino]-2-oxo-perhydrocyclopent[b]azepin,
1-Carboxymethyl-3-(S)-[1-(S)-carboxy-5-amino-pentyl-amino]-2-oxo-perhydrocyclopent[b]azepin,
deren Stereoisomeren und deren physiologisch unbedenkliche Salze.

**6.** 1-Carboxymethyl-3-(S)-[1-(S)-ethoxycarbonyl-3-phenylpropylamino]-2-oxo-perhydro-2H-cyclopent[b]-azocin,
1-Carboxymethyl-3-(S)-[1-(S)-carboxy-3-phenylpropylamino]-2-oxo-perhydro-2H-cyclopent[b]azocin,
deren Stereoisomere und deren physiologisch unbedenkliche Salze.

**7.** Verbindung gemäß einem oder mehreren der Ansprüche 1-6 zur Anwendung als Heilmittel.

**8.** Mittel enthaltend eine Verbindung gemäß einem oder mehreren der Ansprüche 1-6 und einem physiologisch unbedenklichen Träger.

**9.** Mittel gemaß Anspruch 8, enthaltend eine Verbindung gemäß einem oder mehreren der Ansprüche 1-6 in Kombination mit einem Diuretikum.

**10.** Verbindung gemäß einem oder mehreren der Ansprüche 1-6 zur Anwendung als Heilmittel bei der Behandlung des Bluthochdrucks oder von Herzerkrankungen.

**11.** Verfahren zur Herstellung von Verbindungen der Formel I, gemäß Anspruch 1, dadurch gekennzeichnet, daß man
a) eine Verbindung der Formel II

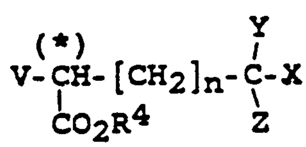

(II)

in der n, $R^4$, X, Y und Z wie in Anspruch 1 definiert sind, $R^4$ jedoch nicht Wasserstoff bedeutet und V für eine nucleophil substituierbare Abgangsgruppe steht, mit einer Verbindung der Formel III,

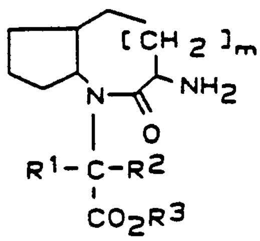

(III)

in der m, $R^1$, $R^2$, und $R^3$ wie im Anspruch 1 definiert sind, $R^3$ jedoch nicht Wasserstoff bedeutet, umsetzt,

b) eine Verbindung der vorstehend definierten Formel III mit einer Verbindung der Formel IV,

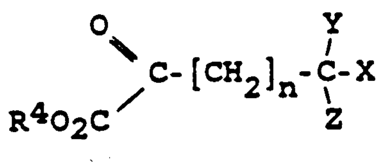

(IV)

in der n, $R^4$ und X vorstehende Bedeutungen haben und Y und Z jeweils Wasserstoff bedeuten, in Gegenwart eines Reduktionsmittels umsetzt,

c) eine Verbindung der Formel V,

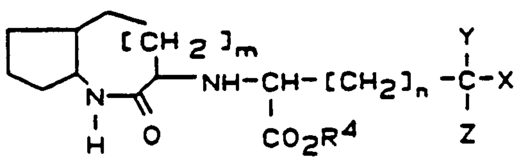

(V)

in der m, n, $R^4$, X, Y und Z wie oben definiert sind, mit einer Verbindung der Formel VI,

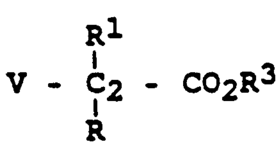

(VI)

in der $R^1$, $R^2$, $R^3$ und V wie oben unter a) definiert sind, alkyliert,

d) eine Verbindung der Formel VII,

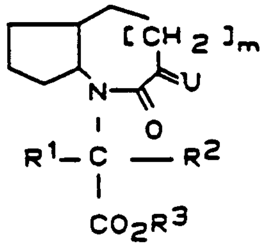

(VII)

in der m, $R^1$, $R^2$ und $R^3$ die oben unter a) definierten Bedeutungen haben und U eine Oxo-Gruppe oder zusammen Wasserstoff und eine unter a) definierte Gruppe V bedeutet, mit einem Amin der Formel VIII

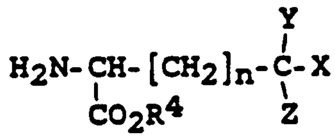

(VIII)

in der n, $R^4$ und X wie oben unter a) definiert sind und Y und Z Wasserstoff bedeuten, umsetzt, wobei im Falle U = Oxo die Umsetzung in Gegenwart eines Reduktionsmittels erfolgt,
e) eine Verbindung der Formel IX,

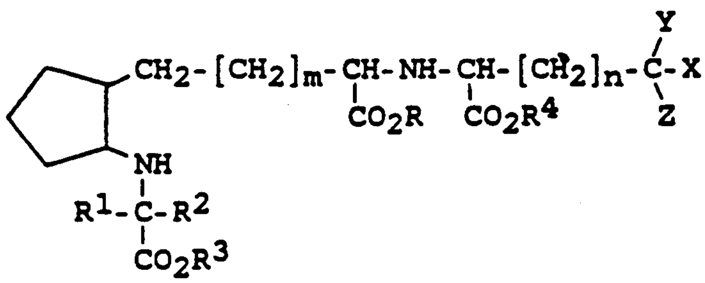

(IX)

in der m, n, $R^1$, $R^2$, $R^3$, $R^4$, X, Y und Z die oben unter a) angegebenen Bedeutungen besitzen und R Wasserstoff oder eine Estergruppe bedeutet, cyclisiert oder
f) zur Herstellung von Verbindungen der Formel I, in der Y und Z zusammen Sauerstoff bedeuten, eine Verbindung der unter a) definierten Formel III mit einer Verbindung der Formel X

$R^4O_2C\text{-}CH=CH\text{-}CO\text{-}X$ (X)

in der $R^4$ und X wie oben in Formel I definiert sind, in an sich bekannter Weise in einer Michael-Reaktion umsetzt oder die obengenannte Verbindung der Formel III in an sich bekannter Weise in einer Mannich-Reaktion umsetzt mit einer Verbindung der Formel XI,

$OHC\text{-}CO_2R^4$ (XI)

und einer Verbindung der Formel XII,

$X\text{-}CO\text{-}CH_3$ (XII)

in welchen $R^4$ und X die im Anspruch 1 in Formel I definierten Bedeutungen haben, falls Y und Z zusammen Sauerstoff bedeuten, diese Gruppe gegebenenfalls zu Y = Hydroxy oder Wasserstoff und Z = Wasserstoff reduziert,

i) gewünschtenfalls temporär zum Schutz funktioneller Gruppen eventuell eingeführte Schutzgruppen in an sich bekannter Weise abspaltet,
ii) gegebenenfalls Carboxygruppen $CO_2R^3$ und/oder $CO_2R^4$ ($R^3$, $R^4$ = H) in an sich bekannter Weise unter Bildung von Verbindungen der Formel I ($R^3$ und/oder $R^4$ ≠ H) verestert,
iii) gegebenenfalls die Reste $R^3$ und/oder $R^4$ ($R^3$, $R^4$ ≠ H) hydrolytisch oder hydrogenolytisch unter Bildung der freien Carboxygruppe(n) abspaltet,

oder die Reihenfolge dieser Schritte i-iii umkehrt,
und die auf diese Weise erhaltenen Verbindungen der Formel I gegebenenfalls in ihre physiologisch annehmbaren Salze überführt.

**12.** Verbindungen der Formel XIII,

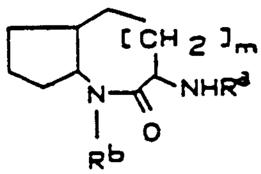

(XIII)

in der m 1 oder 2 bedeutet,

a) $R^a$ und $R^b$ jeweils Wasserstoff bedeuten,

b) $R^a$ Wasserstoff bedeutet und $R^b$ für einen Rest

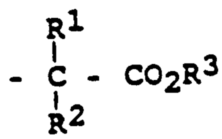

in dem $R^1$, $R^2$ und $R^3$ wie in Anspruch 1 definiert sind, steht oder

c) $R^a$ für einen Rest

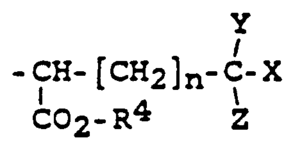

in dem n, $R^4$, X, Y und Z wie im Anspruch 1 definiert sind, steht und $R^b$ Wasserstoff bedeutet, oder

d) $R^a$ für einen Rest

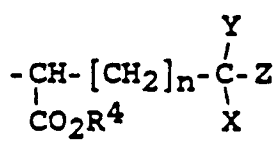

,

in dem n, $R^4$ , X, Y und Z wie oben definiert sind, und $R^b$ für einen Rest

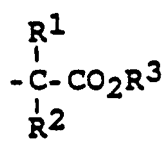

in dem $R^1$, $R^2$ und $R^3$ wie oben definiert sind, stehen.

**13.** Verfahren zur Herstellung von Verbindungen der Formel XIII gemäß Anspruch 12, dadurch gekennzeichnet, daß man

a) Verbindungen der Formel XIV,

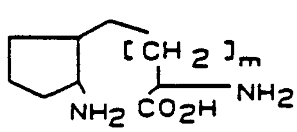

(XIV)

in welcher m 1 oder 2 bedeutet, cyclisiert zu Verbindungen der Formel XV (≅ Formel XIII mit $R^a$,$R^b$ jeweils = Wasserstoff),

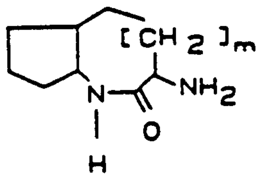

(XV)

in welcher m wie oben definiert ist,
b) Verbindungen der Formel XVI,

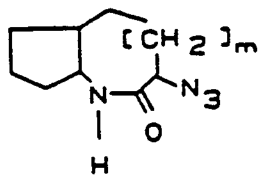

(XVI)

in welcher m wie oben definiert ist, zu Verbindungen der oben definierten Formel XV reduziert,
c) Verbindungen der obigen Formel XV gegebenenfalls mit Verbindungen der Formel VI,

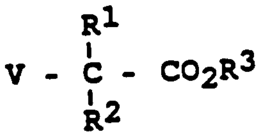

(VI)

in welcher V, $R^1$, $R^2$ und $R^3$ wie im Anspruch 12 unter c) definiert sind, zu Verbindungen der Formel III (≅ Formel XIII mit $R^a$ = H, $R^b$ ≠ H) alkyliert,
d) Verbindungen der obigen Formel XV gegebenenfalls mit Verbindungen der im Anspruch 11 unter a) definierten Formel II zu Verbindungen der Formel V (≅ Formel XV mit $R^a$ ≠ H, $R^b$ = H) umsetzt,
e) Verbindungen der obigen Formel XV mit einer Verbindung der Formel X

$R^4O_2C\text{-}CH=CH\text{-}CO\text{-}X$ (X)

in der $R^4$ und X wie oben in Formel I definiert sind, in an sich bekannter Weise in einer Michael-Reaktion umsetzt oder die obengenannte Verbindung der Formel III in an sich bekannter Weise in einer Mannich-Reaktion umsetzt mit einer Verbindung der Formel XI,

$OHC\text{-}CO_2R^4$ (XI)

und einer Verbindung der Formel XII,

$X\text{-}CO\text{-}CH_3$ (XII)

in welchen $R^4$ und X die im Anspruch 1 in Formel I definierten Bedeutungen haben, falls Y und Z zusammen Sauerstoff bedeutet, diese Gruppe gegebenenfalls zu Y = Hydroxy oder Wasserstoff und Z = Wasserstoff reduziert,
f) oder eine Verbindung der Formel XVI mit Verbindungen der Formel VI zu Verbindungen der Formel XVII,

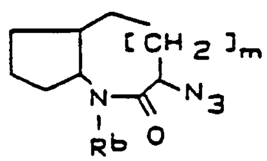

(XVII)

in der m und $R^b$ ($R^b \neq H$) wie in Anspruch 12 definiert sind, alkyliert und diese Verbindungen durch katalytische Hydrierung mit Metallkatalysatoren oder durch Reaktion mit komplexen in Hydriden in Verbindungen der Formel XIII ($R^a$ = H, $R^b \neq H$ überführt.

**14.** Verfahren zur Herstellung eines Mittels gemäß Anspruch 8, dadurch gekennzeichnet, daß man eine Verbindung der Formel I zusammen mit einem physiologisch unbedenklichen Träger und gegebenenfalls weiteren Hilfsstoffen in eine geeignete Darreichungsform bringt.

**Patentansprüche für folgenden Vertragsstaat : ES**

**1.** Verfahren zur Herstellung von Verbindungen der Formel I

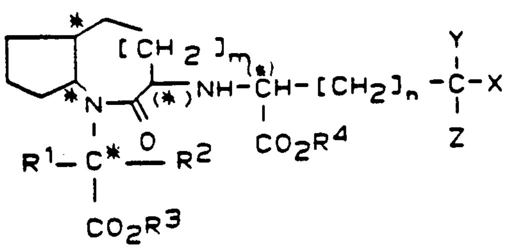

(I)

in welcher
m = 1 oder 2 und
n = 0, 1 oder 2 bedeuten,

| | |
|---|---|
| $R^1$ und $R^2$ | gleich oder verschieden sind und Wasserstoff, ($C_1$-$C_6$)-Alkyl, das gegebenenfalls monosubstituiert sein kann durch Hydroxy, Mercapto, ($C_1$-$C_2$)-Alkoxy, ($C_1$-$C_2$)-Alkylmercapto, Carboxy, ($C_1$-$C_2$)-Alkxoycarbonyl, 3-Indolyl, Imidazolyl, Carbamoyl, Amino oder Guanidino, ($C_2$-$C_6$)-Alkenyl, ($C_3$-$C_9$)-Cycloalkyl, ($C_3$-$C_9$)-Cycloalkenyl, ($C_3$-$C_7$)-Cycloalkyl-($C_1$-$C_4$)-alkyl, ($C_6$-$C_{12}$)-Aryl, teilhydriertes ($C_6$-$C_{12}$)-Aryl oder ($C_6$-$C_{12}$)-Aryl-($C_1$-$C_4$)-alkyl, das im Arylteil eine Hydroxygruppe tragen kann, bedeuten, |
| $R^3$ und $R^4$ | gleich oder verschieden sind und Wasserstoff, ($C_1$-$C_6$)-Alkyl, ($C_2$-$C_6$)-Alkenyl oder ($C_6$-$C_{12}$)-Aryl-($C_1$-$C_4$)-Alkyl, das im Arylteil durch Methoxy oder Nitro monosubstituiert sein kann, bedeuten, |
| Y | Wasserstoff oder Hydroxy, |
| Z | Wasserstoff oder |
| Y und Z | zusammen Sauerstoff bedeuten und |
| X | für ($C_1$-$C_6$)-Alkyl, das durch Amino, Acylamino, ($C_1$-$C_4$)-Alkylamino und/oder Di-($C_1$-$C_4$)-Alkylamino substituiert sein kann, ($C_2$-$C_6$)-Alkenyl, ($C_5$-$C_9$)-Cycloalkyl, |

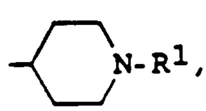

($C_6$-$C_{12}$)-Aryl, das durch ($C_1$-$C_4$)-Alkyl, ($C_1$-$C_4$)-Alkoxy, Hydroxy, Halogen, Nitro Amino, ($C_1$-$C_4$)-Alkylamino, Di-($C_1$-$C_4$)-Alkylamino und/oder Methylendioxy mono-, di- oder trisubstituiert sein kann, oder Indol-3-yl steht, wobei in den obengenannten Resten $R^1$, $R^2$ und X freies Hydroxy, Mercapto, Carboxy, Amino oder Guanidino gegebenenfalls durch in der Peptidchemie übliche Schutzguppen geschützt sind, sowie deren physiologisch unbedenklichen Salzen, dadurch gekennzeichnet, daß man

a) eine Verbindung der Formel II

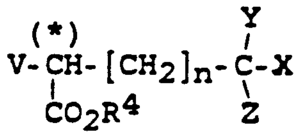

(II)

in der n, $R^4$, X, Y und Z oben definiert sind, $R^4$ jedoch nicht Wasserstoff bedeutet und V für eine nucleophil substituierbare Abgangsgruppe steht, mit einer Verbindung der Formel III,

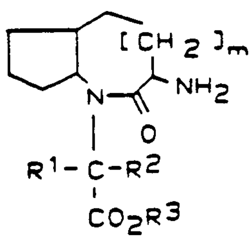

(III)

in der m, $R^1$, $R^2$ und $R^3$ und wie oben definiert sind, $R^3$ jedoch nicht Wasserstoff bedeutet, umsetzt,
b) eine Verbindung der vorstehend definierten Formel III mit einer Verbindung der Formel IV,

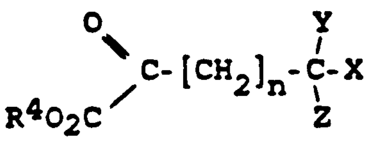

(IV)

in der n, $R^4$ und X vorstehende Bedeutungen haben und Y und Z jeweils Wasserstoff bedeuten, in Gegenwart eines Reduktionsmittels umsetzt,
c) eine Verbindung der Formel V,

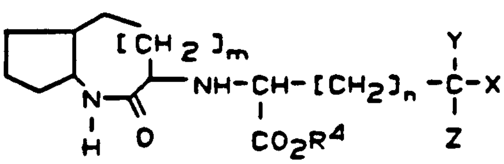

(V)

in der m, n, $R^4$, X, Y und Z wie oben definiert sind, mit einer Verbindung der Formel VI,

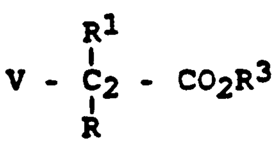

(VI)

in der $R^1$, $R^2$, $R^3$ und V wie oben unter a) definiert sind, alkyliert,

d) eine Verbindung der Formel VII,

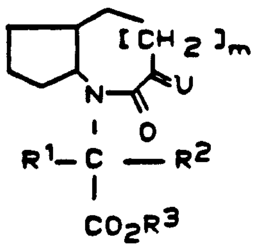

(VII)

in der m, $R^1$, $R^2$ und $R^3$ die oben unter a) definierten Bedeutungen haben und U eine Oxo-Gruppe oder zusammen Wasserstoff und eine unter a) definierte Gruppe V bedeutet, mit einem Amin der Formel VIII,

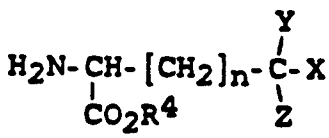

(VIII)

in der n, $R^4$ und X wie oben unter a) definiert sind und Y und Z Wasserstoff bedeuten, umgesetzt, wobei im Falle U = Oxo die Umsetzung in Gegenwart eines Reduktionsmittels erfolgt,

e) eine Verbindung der Formel IX,

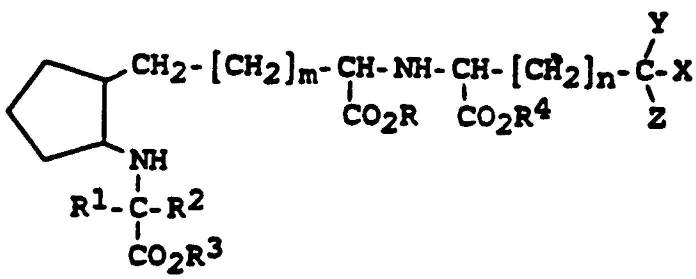

(IX)

in der m, n, $R^1$, $R^2$, $R^3$, $R^4$, X, Y und Z die oben unter a) angegebenen Bedeutungen besitzen und R Wasserstoff oder eine Estergruppe bedeutet, cyclisiert oder

f) zur Herstellung von Verbindungen der Formel I, in der Y und Z zusammen Sauerstoff bedeutet, eine Verbindung der unter a) definierten Formel III mit einer Verbindung der Formel X

$R^4O_2C\text{-}CH=CH\text{-}CO\text{-}X$ (X)

in der $R^4$ und X wie oben in Formel I definiert sind,in an sich bekannter Weise in einer Michael-Reaktion umsetzt oder die obengenannte Verbindung der Formel III in an sich bekannter Weise in einer Mannich-Reaktion umsetzt mit einer Verbindung der Formel XI,

$OHC\text{-}CO_2R^4$ (XI)

und einer Verbindung der Formel XII,

$X\text{-}CO\text{-}CH_3$ (XII)

in welchen $R^4$ und X die im Anspruch 1 in Formel I definierten Bedeutungen haben. Falls Y and Z zusammen Sauerstoff bedeuten, diese Gruppe zu gegebenenfalls Y = Hydroxy oder Wasserstoff und Z = Wasserstoff reduziert,

i) gewünschtenfalls temporär zum Schutz funktioneller Gruppen eventuell eingeführte Schutzgruppen in an sich bekannter Weise abspaltet,
ii) gegebenenfalls Carboxygruppen $CO_2R^3$ und/oder $CO_2R^4$ ($R^3$, $R^4$ = H) in an sich bekannter Weise unter Bildung von Verbindungen der Formel I ($R^3$ und/oder $R^4$ ≠ H) verestert,
iii) gegebenenfalls die Reste $R^3$ und/oder $R^4$ ($R^3$, $R^4$ ≠ H) hydrolytisch oder hydrogenolytisch unter Bildung der freien Carboxygruppe(n) abspaltet,
oder die Reihenfolge dieser Schritte i - iii umkehrt,
und die auf diese Weise erhaltenen Verbindungen der Formel I gegebenenfalls in ihre physiologisch annehmbaren Salze überführt.

**2.** Verfahren gemäß Anspruch 1, dadurch gekennzeichnet, daß man Verbindungen der Formel I herstellt, in welcher
m = 1 und
n = 1 ist,

| | |
|---|---|
| $R^1$ | Wasserstoff, $(C_1-C_3)$-Alkyl, $(C_3-C_6)$-Cycloalkyl $(C_3-C_6)$-Cycloalkyl-$(C_1-C_2)$-alkyl oder Allyl, |
| $R^2$ | Wasserstoff, $(C_1-C_3)$-Alkyl, $(C_3-C_6)$-Cycloalkyl, $(C_3-C_6)$-Cycloalkyl-$(C_1-C_2)$-alkyl oder Allyl, |
| $R^3$ | Wasserstoff, $(C_1-C_4)$-Alkyl, Benzyl oder 4-Methoxybenzyl, |
| $R^4$ | Wasserstoff, $(C_1-C_4)$-Alkyl, Benzyl oder 4-Methoxybenzyl, |
| X | Phenyl oder mit Fluor und/oder Chlor mono- oder disubstituiertes Phenyl, Methyl, Cyclohexyl oder Aminoethyl, |
| Y | Wasserstoff oder Hydroxy und |
| Z | Wasserstoff bedeuten, oder |
| Y und Z | zusammen für Sauerstoff steht. |

**3.** Verfahren gemäß einem der Ansprüche 1 und 2, dadurch gekennzeichnet, daß man Verbindungen der Formel I herstellt, in welcher
m = 1,
n = 1,

| | |
|---|---|
| $R^1$, $R^2$, und R | $^3$ jeweils Wasserstoff, |
| $R^4$ | = Wasserstoff oder Ethyl, |
| Y und Z | jeweils Wasserstoff und |
| X | Phenyl bedeuten. |

**4.** Verfahren gemäß Anspruch 1, dadurch gekennzeichnet, daß man Verbindungen der Formel I herstellt, in welcher die mit einem Stern [(*)] bezeichneten chiralen Kohlenstoffatome S-Konfiguration besitzen.

**5.** Verfahren gemäß Anspruch 1, dadurch gekennzeichnet, daß man
1-Carboxymethyl-3-(S)-[1-(S)-ethoxycarbonyl-3-phenyl-propylamino]-2-oxo-perhydrocyclopent[b]azepin,
1-Carboxymethyl-3-(S)-[1-(S)-carboxy-3-phenylpropylamino]-2-oxo-perhydrocyclopent[b]azepin,
1-Carboxymethyl-3-(S)-[1-(S)-ethoxycarbonyl-butylamino]-2-oxo-perhydrocyclopent[b]azepin,
1-Carboxymethyl-3-(S)-[1-(S)-carboxy-butylamino]-2-oxo-perhydrocyclopent[b]azepin,
1-Carboxymethyl-3-(S)-[1-(S)-ethoxycarbonyl-5-aminopentylamino]-2-oxo-perhydrocyclopent[b]azepin,
1-Carboxymethyl-3-(S)-[1-(S)-carboxy-5-amino-pentyl-amino]-2-oxo-perhydrocyclopent[b]azepin,
deren Stereoisomeren oder deren physiologisch unbedenkliche Salze herstellt.

**6.** Verfahren gemäß Anspruch 1, dadurch gekennzeichnet, daß man
1-Carboxymethyl-3-(S)-[1-(S)-ethoxycarbonyl-3-phenylpropylamino]-2-oxo-perhydro-2H-cyclopent[b]-azocin,
1-Carboxymethyl-3-(S)-[1-(S)-carboxy-3-phenylpropylamino]-2-oxo-perhydro-2H-cyclopent[b]azocin,
deren Stereoisomere oder deren physiologisch unbedenkliche Salze herstellt.

**7.** Verfahren zur Herstellung von Verbindungen der Formel XIII

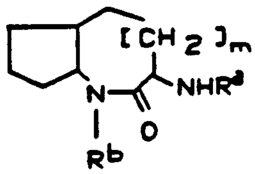 (XIII)

in der m 1 oder 2 bedeutet,
a) $R^a$ und $R^b$ jeweils Wasserstoff bedeuten,
b) $R^a$ Wasserstoff bedeutet und $R^b$ für einen Rest

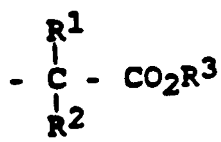

in dem $R^1$, $R^2$ und $R^3$ wie in Anspruch 1 definiert sind, steht oder
c) $R^a$ für einen Rest

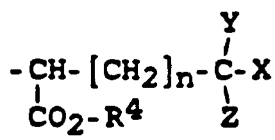

in dem n, $R^4$, X, Y und Z wie im Anspruch 1 definiert sind, steht und $R^b$ Wasserstoff bedeutet, oder
d) $R^a$ für einen Rest

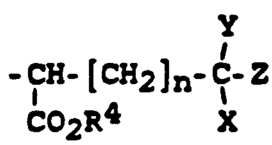 ,

in dem n, $R^4$, X, Y und Z wie oben definiert sind, und $R^b$ für einen Rest

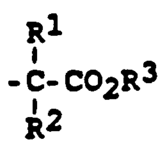

in dem $R^1$, $R^2$ und $R^3$ wie oben definiert sind, stehen, dadurch gekennzeichnet, daß man
a) Verbindungen der Formel XIV,

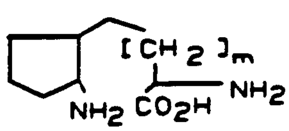 (XIV)

in welcher m 1 oder 2 bedeutet, cyclisiert zu Verbindungen der Formel XV (≅ Formel XIII mit $R^a$,$R^b$ jeweils = Wasserstoff),

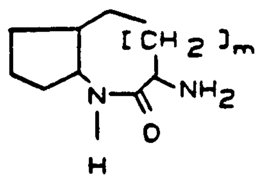

(XV)

im welcher m wie oben definiert ist,
b) Verbindungen der Formel XVI,

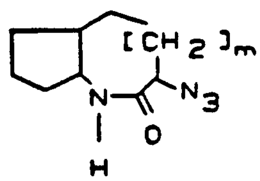

(XVI)

in welcher m wie oben definiert ist, zu Verbindungen der oben definierten Formel XV reduziert,
c) Verbindungen der obigen Formel XV gegebenenfalls mit Verbindungen der Formel VI,

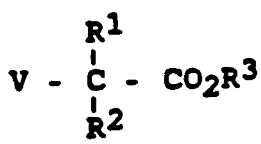

(VI)

in welcher V, $R^1$, $R^2$ und $R^3$ wie im Anspruch 1 unter c) definiert sind, zu Verbindungen der Formel III (≅ Formel XIII mit $R^a$ = H, $R^b$ ≠ H) alkyliert,
d) Verbindungen der obigen Formel XV gegebenenfalls mit Verbindungen der im Anspruch 1 ) unter a) definierten Formel II zu Verbindungen der Formel V (≅ Formel XV mit $R^a$ ≠ H, $R^b$ = H) umsetzt,
e) Verbindungen der obigen Formel XV mit einer Verbindung der Formel X

$R^4O_2C\text{-}CH=CH\text{-}CO\text{-}X$ (X)

in der $R^4$ und X wie oben in Formel I definiert sind, in an sich bekannter Weise in einer Michael-Reaktion umsetzt oder die obengenannte Verbindung der Formel III in an sich bekannter Weise in einer Mannich-Reaktion umsetzt mit einer Verbindung der Formel XI,

$OCH\text{-}CO_2R^4$ (XI)

und einer Verbindung der Formel XII,

$X\text{-}CO\text{-}CH_3$ (XII)

in welchen $R^4$ und X die im Anspruch 1 in Formel I definierten Bedeutungen haben, falls Y und Z zusammen Saurestoff bedeutet, diese Gruppe gegebenenfales zu Y = Hydroxy oder Wasserstoff und Z = Wasserstoffe reduziert,
f) oder eine Verbindung der Formel XVI mit Verbindungen der Formel VI zu Verbindungen der Formel XVII,

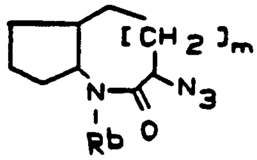

(XVII)

in der m und $R^b$ ($R^b \neq H$) wie in Anspruch 7 definiert sind, alkyliert und diese Verbindungen durch katalytische Hydrierung mit Metallkatalysatoren oder durch Reaktion mit komplexen Hydriden in Verbindungen der Formel XIII ($R^a$ = H, $R^b \neq H$) überführt.

**8.** Verfahren zur Herstellung eines pharmazeutischen Mittels enthaltend eine Verbindung hergestellt gemäß einem der Ansprüche 1 - 6, dadurch gekennzeichnet, daß man eine Verbindung der Formel I zusammen mit einem physiologisch unbedenklichen Träger und gegebenenfalls weiteren Hilfsstoffen in eine geeignete Darreichungsform bringt.

**9.** Verfahren gemäß Anspruch 8, dadurch gekennzeichnet, daß man ein Diuretikum zugibt.

**Patentansprüche für folgenden Vertragsstaat : GR**

**1.** Verfahren zur Herstellung von Verbindungen der Formel I

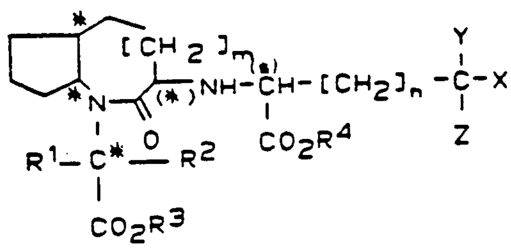

(I)

in welcher
m = 1 oder 2 und
n = 0, 1 oder 2 bedeuten,

$R^1$ und $R^2$ gleich oder verschieden sind und Wasserstoff, ($C_1$-$C_6$)-Alkyl, das gegebenenfalls monosubstituiert sein kann durch Hydroxy, Mercapto, ($C_1$-$C_2$)-Alkoxy, ($C_1$-$C_2$)-Alkylmercapto, Carboxy, ($C_1$-$C_2$)-Alkoxycarbonyl, 3-Indolyl, Imidazolyl, Carbamoyl, Amino oder Guanidino, ($C_2$-$C_6$)-Alkenyl, ($C_3$-$C_9$)-Cycloalkyl, ($C_3$-$C_9$)-Cycloalkenyl, ($C_3$-$C_7$)-Cycloalkyl-($C_1$-$C_4$)-alkyl, ($C_6$-$C_{12}$)-Aryl, teilhydriertes ($C_6$-$C_{12}$)-Aryl oder ($C_6$-$c_{12}$)-Aryl-($C_1$-$C_4$)-alkyl, das im Arylteil eine Hydroxygruppe tragen kann, bedeuten,

$R^3$ und $R^4$ gleich oder verschieden sind und Wasserstoff, ($C_1$-$C_6$)-Alkyl, ($C_2$-$C_6$)-Alkenyl oder ($C_6$-$C_{12}$)-Aryl-($C_1$-$C_4$)-alkyl, das im Arylteil durch Methoxy oder Nitro monosubstituiert sein kann, bedeuten,

Y Wasserstoff oder Hydroxy,

Z Wasserstoff oder

Y und Z zusammen Sauerstoff bedeuten und

X für $C_1$-$C_6$-Alkyl, das durch Amino, Acylamino, ($C_1$-$C_4$)-Alkylamino und/oder Di-($C_1$-$C_4$)-Alkylamino substituiert sein kann, ($C_2$-$C_6$)-Alkenyl, ($C_5$-$C_9$)-Cycloalkyl,

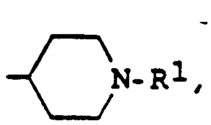

($C_6$-$C_{12}$)-Aryl, das durch ($C_1$-$C_4$)-Alkyl, ($C_1$-$C_4$)-Alkoxy, Hydroxy, Halogen, Nitro Amino, ($C_1$-$C_4$)-Alkylamino, Di-($C_1$-$C_4$)-Alkylamino und/oder Methylendioxy, mono-, di- oder trisubstituiert sein kann, oder Indol-3-yl steht, wobei in den obengenannten

Resten $R^1$, $R^2$ und X freies Hydroxy, Mercapto, Carboxy, Amino oder Guanidino gegebenenfalls durch in der Peptidchemie übliche Schutzgruppen geschützt sind, sowie deren physiologisch unbedenklichen Salzen, dadurch gekennzeichnet, daß man

a) eine Verbindung der Formel II

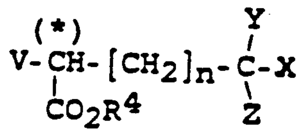

(II)

in der n, $R^4$, X, Y und Z wie oben definiert sind, $R^4$ jedoch nicht Wasserstoff bedeutet und V für eine nucleophil substituierbare Abgangsgruppe steht, mit einer Verbindung der Formel III,

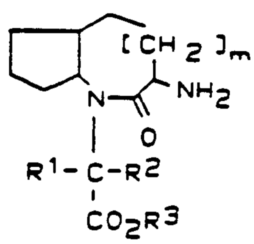

(III)

in der m, $R^1$, $R^2$, und $R^3$ wie oben definiert sind, $R^3$ jedoch nicht Wasserstoff bedeutet, umsetzt,

b) eine Verbindung der vorstehend definierten Formel III mit einer Verbindung der Formel IV,

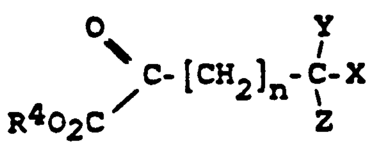

(IV)

in der n, $R^4$ und X vorstehende Bedeutungen haben und Y und Z jeweils Wasserstoff bedeuten, in Gegenwart eines Reduktionsmittels umsetzt,

c) eine Verbindung der Formel V,

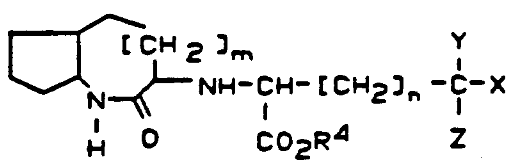

(V)

in der m, n, $R^4$, X, Y und Z wie oben definiert sind, mit einer Verbindung der Formel VI,

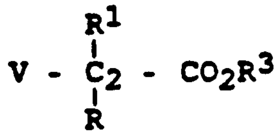

(VI)

in der $R^1$, $R^2$, $R^3$ und V wie oben unter a) definiert sind, alkyliert,

d) eine Verbindung der Formel VII,

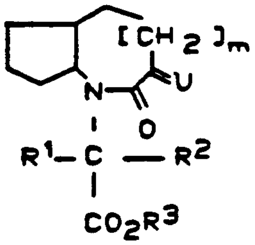

(VII)

in der m, $R^1$, $R^2$ und $R^3$ die oben unter a) definierten Bedeutungen haben und U eine Oxo-Gruppe oder zusammen Wasserstoff und eine unter a) definierte Gruppe V bedeutet, mit einem Amin der Formel VIII

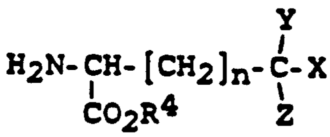

(VIII)

in der n, $R^4$ und X wie oben unter a) definiert sind und Y und Z Wasserstoff bedeuten, umsetzt, wobei im Falle U = Oxo die Umsetzung in Gegenwart eines Reduktionsmittels erfolgt,

e) eine Verbindung der Formel IX,

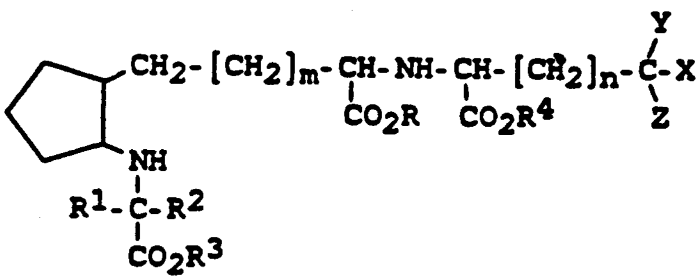

(IX)

in der m, n, $R^1$, $R^2$, $R^3$, $R^4$, X, Y und Z die oben unter a) angegebenen Bedeutungen besitzen und R Wasserstoff oder eine Estergruppe bedeutet, cyclisiert oder

f) zur Herstellung von Verbindungen der Formel I, in der Y und Z zusammen Sauerstoff bedeuten, eine Verbindung der unter a) definierten Formel III mit einer Verbindung der Formel X

$R^4O_2C\text{-}CH=CH\text{-}CO\text{-}X$ (X)

in der $R^4$ und X wie oben in Formel I definiert sind,in an sich bekannter Weise in einer Michael-Reaktion umsetzt oder die obengenannte Verbindung der Formel III in an sich bekannter Weise in einer Mannich-Reaktion umsetzt mit einer Verbindung der Formel XI,

$OHC\text{-}CO_2R^4$ (XI)

und einer Verbindung der Formel XII,

$X\text{-}CO\text{-}CH_3$ (XII)

in welchen $R^4$ und X die im Anspruch 1 in Formel I definierten Bedeutungen haben, falls Y und Z zusammen Sauerstoff bedeuten, diese Gruppe gegebenenfalls zu Y = Hydroxy oder Wasserstoff und Z = Wasserstoff reduziert,

i) gewünschtenfalls temporär zum Schutz funktioneller Gruppen eventuell eingeführte Schutzgruppen in an sich bekannter Weise abspaltet,
ii) gegebenenfalls Carboxygruppen $CO_2R^3$ und/oder $CO_2R^4$ ($R^3$, $R^4$ = H) in an sich bekannter Weise unter Bildung von Verbindungen der Formel I ($R^3$ und/oder $R^4$ ≠ H) verestert,
iii) gegebenenfalls die Reste $R^3$ und/oder $R^4$ ($R^3$, $R^4$ ≠ H) hydrolytisch oder hydrogenolytisch unter Bildung der freien Carboxygruppe(n) abspaltet,
oder die Reihenfolge dieser Schritte i-iii umkehrt,
und die auf diese Weise erhaltenen Verbindungen der Formel I gegebenenfalls in ihre physiologisch annehmbaren Salze überführt.

**2.** Verfahren gemäß Anspruch 1, dadurch gekennzeichnet, daß man Verbindungen der Formel I herstellt, in welcher
m = 1 und
n = 1 ist,
$R^1$ Wasserstoff, ($C_1$-$C_3$)-Alkyl, ($C_3$-$C_6$)-Cycloalkyl ($C_3$-$C_6$)-Cycloalkyl-($C_1$-$C_2$)-aklyl oder Allyl,
$R^2$ Wasserstoff, ($C_1$-$C_3$)-Alkyl, ($C_3$-$C_6$)-Cycloalkyl, ($C_3$-$C_6$)-Cycloalkyl-($C_1$-$C_2$)-alkyl oder Allyl,
$R^3$ Wasserstoff, ($C_1$-$C_4$)-Alkyl, Benzyl oder 4-Methoxybenzyl,
$R^4$ Wasserstoff, ($C_1$-$C_4$)-Alkyl, Benzyl oder 4-Methoxybenzyl,
X Phenyl oder mit Fluor und/oder Chlor mono- oder disubstituiertes Phenyl, Methyl, Cyclohexyl, oder Aminoethyl,
Y Wasserstoff oder Hydroxy und
Z Wasserstoff bedeuten oder
Y und Z zusammen für Sauerstoff steht.

**3.** Verfahren gemäß einem der Ansprüche 1 und 2, dadurch gekennzeichnet, daß man Verbindungen der Formel I herstellt, in welcher
m = 1,
n = 1,
$R^1$, $R^2$ und $R^3$ jeweils Wasserstoff,
$R^4$ Wasserstoff oder Ethyl,
Y und Z jeweils Wasserstoff und
X Phenyl bedeuten.

**4.** Verfahren gemäß Anspruch 1, dadurch gekennzeichnet, daß man Verbindungen der Formel I herstellt, in welcher die mit einem Stern [(*)] bezeichneten chiralen Kohlenstoffatome S-Konfiguration besitzen.

**5.** Verfahren gemäß Anspruch 1, dadurch gekennzeichnet, daß man
1-Carboxymethyl-3-(S)-[1-(S)-ethoxycarbonyl-3-phenylpropylamino]-2-oxo-perhydrocyclopent[b]-azepin,
1-Carboxymethyl-3-(S)-[1-(S)-carboxy-3-phenylpropylamino]-2-oxo-perhydrocyclopent[b]azepin,
1-Carboxymethyl-3-(S)-[1-(S)-ethoxycarbonyl-butylamino]-2-oxo-perhydrocyclopent[b]azepin,
1-Carboxymethyl-3-(S)-[1-(S)-carboxy-butylamino]-2-oxo-perhydrocyclopent[b]azepin,
1-Carboxomethyl-3-(S)-[1-(S)-ethoxycarbonyl-5-amino-pentylamino]-2-oxo-perhydrocyclopent[b]azepin,
1-Carboxymethyl-3-(S)-[1-(S)-carboxy-5-amino-pentylamino]-2-oxo-perhydrocyclopent[b]azepin,
deren Stereoisomeren oder deren physiologisch unbedenkliche Salze herstellt.

**6.** Verfahren gemäß Anspruch 1, dadurch gekennzeichnet, daß man
1-Carboxymethyl-3-(S)-[1-(S)-ethoxycarbonyl-3-phenylpropylamino]-2-oxo-perhydro-2H-cyclopent[b]-azocin,
1-Carboxymethyl-3-(S)-[1-(S)-carboxy-3-phenylpropylamino]-2-oxo-perhydro-2H-cyclopent[b]azocin,
deren Stereoisomere oder deren physiologisch unbedenkliche Salze herstellt.

**7.** Verfahren zur Herstellung von Verbindungen der Formel XIII

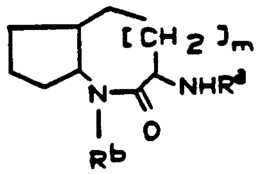

(XIII)

in der m 1 oder 2 bedeutet,

a) $R^a$ und $R^b$ jeweils Wasserstoff bedeuten,

b) $R^a$ Wasserstoff bedeutet und $R^b$ für einen Rest

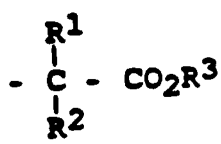

in dem $R^1$, $R^2$ und $R^3$ wie in Anspruch 1 definiert sind, steht oder

c) $R^a$ für einen Rest

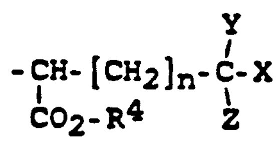

in dem n, $R^4$, X, Y und Z wie im Anspruch 1 definiert sind, steht und $R^b$ Wasserstoff bedeutet, oder

d) $R^a$ für einen Rest

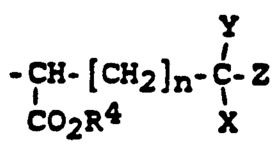

,

in dem n, $R^4$ , X, Y und Z wie oben definiert sind, und $R^b$ für einen Rest

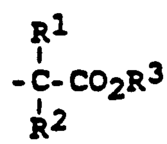

in dem $R^1$, $R^2$ und $R^3$ wie oben definiert sind, stehen, dadurch gekennzeichnet, daß man

a) Verbindungen der Formel XIV,

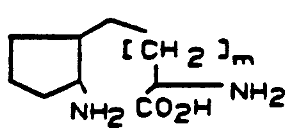

(XIV)

in welcher m 1 oder 2 bedeutet, cyclisiert zu Verbindungen der Formel XV (≅ Formel XIII mit $R^a$,$R^b$ jeweils = Wasserstoff),

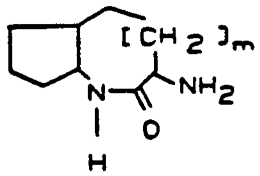

(XV)

in welcher m wie oben definiert ist,
b) Verbindungen der Formel XVI,

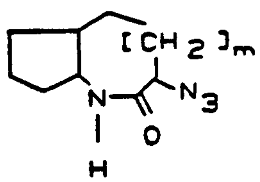

(XVI)

in welcher m wie oben definiert ist, zu Verbindungen der oben definierten Formel XV reduziert,
c) Verbindungen der obigen Formel XV gegebenenfalls mit Verbindungen der Formel VI,

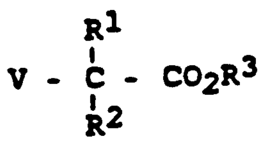

(VI)

in welcher V, $R^1$, $R^2$ und $R^3$ wie im Anspruch 1 unter c) definiert sind, zu Verbindungen der Formel III (≅ Formel XIII mit $R^a$ = H, $R^b \neq$ H) alkyliert,
d) Verbindungen der obigen Formel XV gegebenenfalls mit Verbindungen der im Anspruch 1 ) unter a) definierten Formel II zu Verbindungen der Formel V (≅ Formel XV mit $R^a \neq$ H, $R^b$ = H) umsetzt,
e) Verbindungen der obigen Formel XV mit einer Verbindung der Formel X

$R^4O_2C$-CH=CH-CO-X (X)

in der $R^4$ und X wie oben in Formel I definiert sind, in an sich bekannter Weise in einer Michael-Reaktion umsetzt oder die obengenannte Verbindung der Formel III in an sich bekannter Weise in einer Mannich-Reaktion umsetzt mit einer Verbindung der Formel XI,

OHC-$CO_2R^4$ (XI)

und einer Verbindung der Formel XII,

X-CO-$CH_3$ (XII)

in welchen $R^4$ und X die im Anspruch 1 in Formel I definierten Bedeutungen haben, falls Y und Z zusammen Sauerstoff bedeutet, diese Gruppe gegebenenfalls zu Y = Hydroxy oder Wasserstoff und Z = Wasserstoff reduziert,
f) oder eine Verbindung der Formel XVI mit Verbindungen der Formel VI zu Verbindungen der Formel XVII,

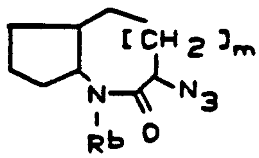

(XVII)

in der m und $R^b$ ($R^b \neq H$) wie in Anspruch 7 definiert sind, alkyliert und diese Verbindungen durch katalytische Hydrierung mit Metallkatalysatoren oder durch Reaktion mit komplexen in Hydriden in Verbindungen der Formel XIII ($R^a$ = H, $R^b \neq H$ überführt.

**8.** Verfahren zur Herstellung eines pharmazeutischen Mittels enthaltend eine Verbindung hergestellt gemäß einem der Ansprüche 1 - 6, dadurch gekennzeichnet, daß man eine Verbindung der Formel I zusammen mit einem physiologisch unbedenklichen Träger und gegebenenfalls weiteren Hilfsstoffen in eine geeignete Darreichungsform bringt.

**9.** Verfahren gemäß Anspruch 8, dadurch gekennzeichnet, daß man ein Diuretikum zugibt.

**10.** Verbindung der Formel XIII, in welcher $R^a$, $R^b$ und m wie im Anspruch 7 definiert sind.

**Claims**

**Claims for the following Contracting States : AT, BE, CH, DE, FR, GB, IT, LI, LU, NL, SE**

**1.** A compound of the formula I

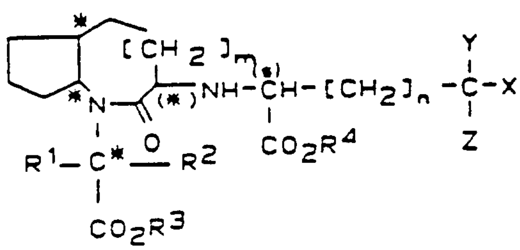

(I)

in which
m is 1 or 2 and
n is 0, 1 or 2

| | |
|---|---|
| $R^1$ and $R^2$ | are identical or different and denote hydrogen, $(C_1-C_6)$-alkyl, which can optionally be monosubstituted by hydroxyl, mercapto, $(C_1-C_2)$-alkoxy, $(C_1-C_2)$-alkylmercapto, carboxyl, $(C_1-C_2)$-alkoxycarbonyl, 3-indolyl, imidazolyl, carbamoyl, amino or guanidino, $(C_2-C_6)$-alkenyl, $(C_3-C_9)$-cycloalkyl, $(C_3-C_9)$-cycloalkenyl, $(C_3-C_7)$-cycloalkyl-$(C_1-C_4)$-alkyl, $(C_6-C_{12})$-aryl, partly hydrogenated $(C_6-C_{12})$-aryl, or $(C_6-C_{12})$-aryl-$(C_1-C_4)$-alkyl, which can carry a hydroxyl group in the aryl part, |
| $R^3$ and $R^4$ | are identical or different and denote hydrogen, $(C_1-C_6)$-alkyl, $(C_2-C_6)$-alkenyl or $(C_6-C_{12})$-aryl-$(C_1-C_4)$-alkyl, which can be monosubstituted in the aryl part by methoxy or nitro, |
| Y | denotes hydrogen or hydroxyl, |
| Z | denotes hydrogen or |
| Y and Z | together denote oxygen, and |
| X | represents $(C_1-C_6)$-alkyl, which can be substituted by amino, acylamino, $(C_1-C_4)$-alkylamino and/or di-$(C_1-C_4)$-alkylamino, $(C_2-C_6)$-alkenyl, $(C_5-C_9)$-cycloalkyl, |

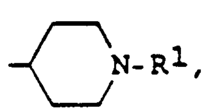

$(C_6-C_{12})$-aryl, which can be mono-, di- or trisubstituted by $(C_1-C_4)$-alkyl, $(C_1-C_4)$-alkoxy, hydroxyl, halogen, nitro, amino, $(C_1-C_4)$-alkylamino, di-$(C_1-C_4)$-alkylamino and/or methylenedioxy, or indol-3-yl, and, in the abovementioned radicals $R^1$, $R^2$ and X, free hydroxyl, mercapto, carboxyl, amino or guanidino are optionally protected by protective groups customary in peptide chemistry, or a physiologically acceptable salt thereof.

**2.** A compound of the formula I as claimed in claim 1, wherein
m is 1 and
n is 1,

| | |
|---|---|
| $R^1$ | is hydrogen, $(C_1-C_3)$-alkyl, $(C_3-C_6)$-cycloalkyl, $(C_3-C_6)$-cycloalkyl-$(C_1-C_2)$-alkyl or allyl, |
| $R^2$ | is hydrogen, $(C_1-C_3)$-alkyl, $(C_3-C_6)$-cycloalkyl, $(C_3-C_6)$-cycloalkyl-$(C_1-C_2)$-alkyl or allyl, |
| $R^3$ | is hydrogen, $(C_1-C_4)$-alkyl, benzyl or 4-methoxybenzyl, |
| $R^4$ | is hydrogen, $(C_1-C_4)$-alkyl, benzyl or 4-methoxybenzyl, |
| X | is phenyl or phenyl which is mono- or disubstituted by fluorine and/or chlorine, methyl, cyclohexyl or aminoethyl, |
| Y | denotes hydrogen or hydroxyl, and |
| Z | denotes hydrogen, or |
| Y and Z | together represent oxygen. |

**3.** A compound of the formula I as claimed in either of claims 1 and 2, wherein
m is 1,
n is 1,

| | |
|---|---|
| $R^1$, $R^2$ and $R^3$ | each denote hydrogen, |
| $R^4$ | denotes hydrogen or ethyl, |
| Y and Z | each denote hydrogen and |
| X | denotes phenyl. |

**4.** A compound of the formula I as claimed in claim 1, wherein the chiral carbon atoms labeled with an asterisk [(*)] have the S configuration.

**5.** 1-Carboxymethyl-3-(S)-[1-(S)-ethoxycarbonyl-3-phenylpropylamino]-2-oxo-perhydrocyclopent[b]azepine,
1-carboxymethyl-3-(S)-[1-(S)-carboxy-3-phenylpropylamino]-2-oxo-perhydrocyclopent[b]azepine,
1-carboxymethyl-3-(S)-[1-(S)-ethoxycarbonyl-butylamino]-2-oxo-perhydrocyclopent[b]azepine,
1-carboxymethyl-3-(S)-[1-(S)-carboxy-butylamino]-2-oxo-perhydrocyclopent[b]azepine,
1-carboxymethyl-3-(S)-[1-(S)-ethoxycarbonyl-5-aminopentylamino]-2-oxo-perhydrocyclopent[b]azepine,
1-carboxymethyl-3-(S)-[1-(S)-carboxy-5-amino-pentylamino]-2-oxo-perhydrocyclopent[b]azepine,
a stereoisomer thereof or a physiologically acceptable salt thereof.

**6.** 1-Carboxymethyl-3-(S)-[1-(S)-ethoxycarbonyl-3-phenylpropylamino]-2-oxo-perhydro-2H-cyclopent[b]-azocine,
1-carboxymethyl-3-(S)-[1-(S)-carboxy-3-phenylpropylamino]-2-oxo-perhydro-2H-cyclopent[b]azocine,
a stereoisomer thereof or a physiologically acceptable salt thereof.

**7.** A compound as claimed in one or more of claims 1-6 for use as a medicine.

**8.** An agent containing a compound as claimed in one or more of claims 1-6 and a physiologically acceptable excipient.

**9.** An agent as claimed in claim 8, containing a compound as claimed in one or more of claims 1-6 in combination with a diuretic.

**10.** A compound as claimed in one or more of claims 1-6 for use as a medicine in the treatment of hypertension or heart diseases.

**11.** A process for the preparation of a compound of the formula I as claimed in claim 1, which comprises

a) reacting a compound of the formula II

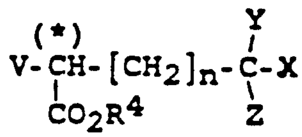

(II)

in which n, $R^4$, X, Y and Z are as defined in claim 1, but $R^4$ does not denote hydrogen, and V represents a leaving group which can be replaced nucleophilically, with a compound of the formula III

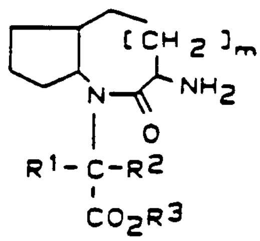

(III)

in which m, $R^1$, $R^2$ and $R^3$ are as defined in claim 1, but $R^3$ does not denote hydrogen,
b) reacting a compound of the formula III defined above with a compound of the formula IV

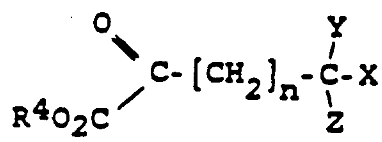

(IV)

in which n, $R^4$ and X have the above meanings and Y and Z each denote hydrogen, in the presence of a reducing agent,
c) alkylating a compound of the formula V

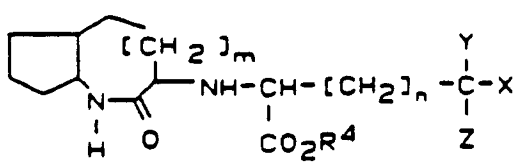

(V)

in which m, n, $R^4$, X, Y and Z are as defined above, with a compound of the formula VI

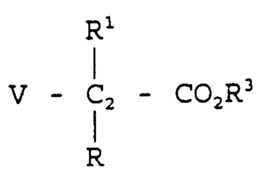

(VI)

in which $R^1$, $R^2$, $R^3$ and V are as defined above under a),

d) reacting a compound of the formula VII

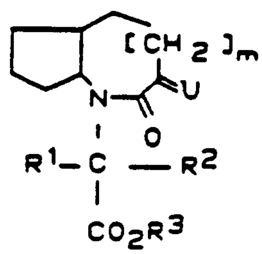

(VII)

in which m, $R^1$, $R^2$ and $R^3$ have the meanings defined above under a) and U denotes an oxo group, or together denotes hydrogen and a group V defined under a), with an amine of the formula VIII

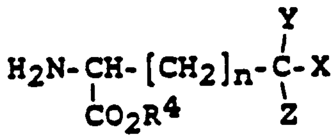

(VIII)

in which n, $R^4$ and X are as defined above under a) and Y and Z denote hydrogen, the reaction being carried out in the presence of a reducing agent in the case where U = oxo,
e) cyclizing a compound of the formula IX

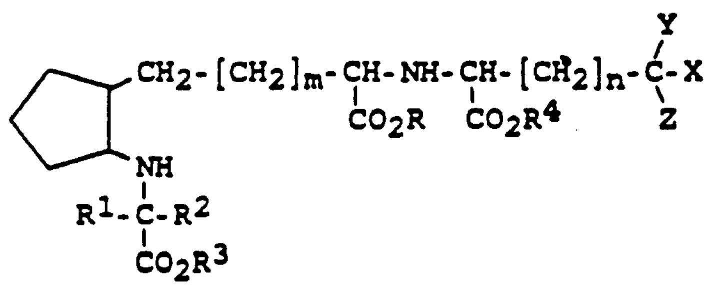

(IX)

in which m, n, $R^1$, $R^2$, $R^3$, $R^4$, X, Y and Z have the meanings given above under a) and R denotes hydrogen or an ester group, or
f) to prepare a compound of the formula I in which Y and Z together denote oxygen, reacting a compound of the formula III defined under a) with a compound of the formula X

$R^4O_2C\text{-}CH=CH\text{-}CO\text{-}X$ (X)

in which $R^4$ and X are as defined above in formula I, in a Michael reaction in a manner which is known per se or reacting the abovementioned compound of the formula III with a compound of the formula XI

$OHC\text{-}CO_2R^4$ (XI)

and a compound of the formula XII

$X\text{-}CO\text{-}CH_3$ (XII)

in which $R^4$ and X have the meaning defined in claim 1 in formula I, in a Mannich reaction in a manner which is known per se, and, if Y and Z together denote oxygen, reducing this group to, if appropriate, Y = hydroxyl or hydrogen and Z = hydrogen,

i) if desired eliminating in a manner which is known per se any protective groups introduced temporarily for protection of functional groups,

ii) if appropriate esterifying carboxyl groups $CO_2R^3$ and/or $CO_2R^4$ ($R^3$,$R^4$ = H) in a manner which is known per se to form a compound of the formula I ($R^3$ and/or $R^4$ ≠ H),

iii) if appropriate eliminating the radicals $R^3$ and/or $R^4$ ($R^3$, $R^4$ ≠ H) hydrolytically or hydrogenolytically to form the free carboxyl group(s),

or reversing the sequence of these steps i - iii,

and, if appropriate, converting the compound of the formula I obtained in this manner into a physiologically acceptable salt thereof.

**12.** A compound of the formula XIII

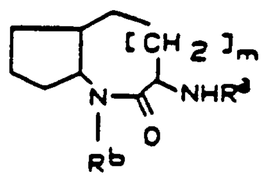

(XIII)

in which m denotes 1 or 2,

a) $R^a$ and $R^b$ each denote hydrogen,

b) $R^a$ denotes hydrogen and $R^b$ represents a radical

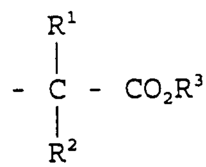

in which $R^1$, $R^2$ and $R^3$ are as defined in claim 1, or

c) $R^a$ represents a radical

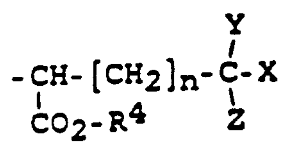

in which n, $R^4$, X, Y and Z are as defined in claim 1 and $R^b$ denotes hydrogen, or

d) $R^a$ represents a radical

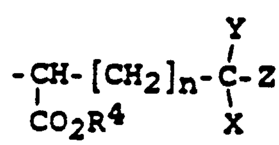

in which n, $R^4$, X, Y and Z are as defined above, and $R^b$ represents a radical

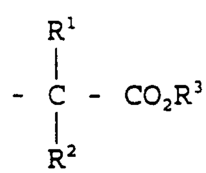

in which $R^1$, $R^2$ and $R^3$ are as defined above.

**13.** A process for the preparation of a compound of the formula XIII as claimed in claim 12, which comprises

a) cyclizing a compound of the formula XIV

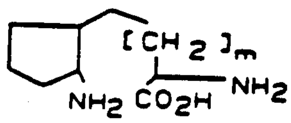

(XIV)

in which m denotes 1 or 2, to give a compound of the formula XV (≙ formula XIII where $R^a$ and $R^b$ are each hydrogen)

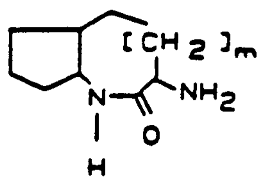

(XV)

in which m is as defined above,

b) reducing a compound of the formula XVI

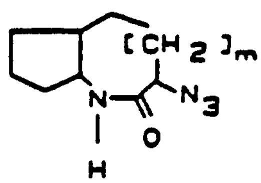

(XVI)

in which m is as defined above, to give a compound of the formula XV defined above,

c) if appropriate alkylating a compound of the above formula XV with a compound of the formula VI

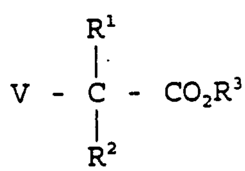

(VI)

in which V, $R^1$, $R^2$ and $R^3$ are as defined in claim 12 under c), to give a compound of the formula III (≙ formula XIII where $R^a$ = H, $R^b$ ≠ H),

d) if appropriate reacting a compound of the above formula XV with a compound of the formula II defined in claim 11) under a) to give a compound of the formula V (≙ formula XV where $R^a$ ≠ H, $R^b$ = H),

e) reacting a compound of the above formula XV with a compound of the formula X

$R^4O_2C\text{-}CH=CH\text{-}CO\text{-}X$ (X)

in which $R^4$ and X are as defined above in formula I, in a Michael reaction in a manner which is known per se, or reacting the abovementioned compound of the formula III with a compound of the formula XI

$OHC\text{-}CO_2R^4$ (XI)

and a compound of the formula XII

$X\text{-}CO\text{-}CH_3$ (XII)

in which $R^4$ and X have the meanings defined in claim 1 in formula I, in a Mannich reaction in a manner which is known per se, and, if Y and Z together denote oxygen, if appropriate reducing this group to Y = hydroxyl or hydrogen and Z = hydrogen,

f) or alkylating a compound of the formula XVI with a compound of the formula VI to give a compound of the formula XVII

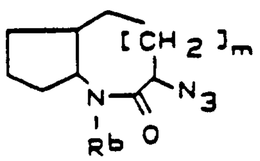

(XVII)

in which m and $R^b$ ($R^b \neq H$) are as defined in claim 12 and converting this compound into a compound of the formula XIII ($R^a$ = H, $R^b \neq H$) by catalytic hydrogenation with a metal catalyst or by reaction with a complex hydride.

**14.** A process for the preparation of an agent as claimed in claim 8, which comprises bringing a compound of the formula I into a suitable administration form together with a physiologically acceptable excipient and if appropriate other auxiliaries.

**Claims for the following Contracting State : ES**

**1.** A process for the preparation of a compound of the formula I

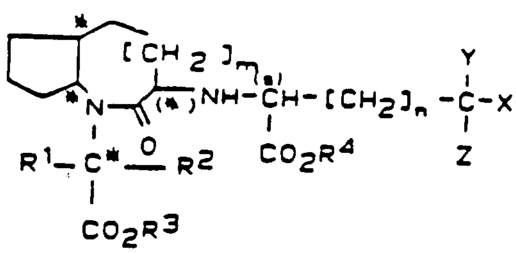

(I)

in which
m is 1 or 2 and
n is 0, 1 or 2

$R^1$ and $R^2$ are identical or different and denote hydrogen, ($C_1$-$C_6$)-alkyl, which can optionally be monosubstituted by hydroxyl, mercapto, ($C_1$-$C_2$)-alkoxy, ($C_1$-$C_2$)-alkylmercapto, carboxyl, ($C_1$-$C_2$)-alkoxycarbonyl, 3-indolyl, imidazolyl, carbamoyl, amino or guanidino, ($C_2$-$C_6$)-alkenyl, ($C_3$-$C_9$)-cycloalkyl, ($C_3$-$C_9$)-cycloalkenyl, ($C_3$-$C_7$)-cycloalkyl-($C_1$-$C_4$)-alkyl, ($C_6$-$C_{12}$)-aryl, partly hydrogenated ($C_6$-$C_{12}$)-aryl, or ($C_6$-$C_{12}$)-aryl-($C_1$-$C_4$)-alkyl, which can carry a hydroxyl group in the aryl part,

$R^3$ and $R^4$ are identical or different and denote hydrogen, ($C_1$-$C_6$)-alkyl, ($C_2$-$C_6$)-alkenyl or ($C_6$-$C_{12}$)-aryl-($C_1$-$C_4$)-alkyl, which can be monosubstituted in the aryl part by methoxy or nitro,

Y denotes hydrogen or hydroxyl,

Z denotes hydrogen or

Y and Z together denote oxygen, and

X represents ($C_1$-$C_6$)-alkyl, which can be substituted by amino, acylamino, ($C_1$-$C_4$)-alkylamino and/or di-($C_1$-$C_4$)-alkylamino, ($C_2$-$C_6$)-alkenyl, ($C_5$-$C_9$)-cycloalkyl,

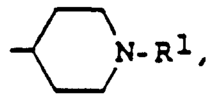

($C_6$-$C_{12}$)-aryl, which can be mono-, di- or trisubstituted by ($C_1$-$C_4$)-alkyl, ($C_1$-$C_4$)-alkoxy, hydroxyl, halogen, nitro, amino, ($C_1$-$C_4$)-alkylamino, di-($C_1$-$C_4$)-alkylamino and/or methylenedioxy, or indol-3-yl, and, in the abovementioned radicals $R^1$, $R^2$ and X, free hydroxyl, mercapto, carboxyl, amino or guanidino are optionally protected by protective groups customary in peptide chemistry,

which comprises

a) reacting a compound of the formula II

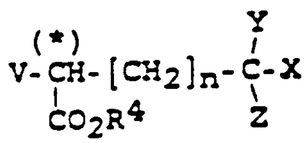

(II)

in which n, $R^4$, X, Y and Z are as defined above, but $R^4$ does not denote hydrogen, and V represents a leaving group which can be replaced nucleophilically, with a compound of the formula III

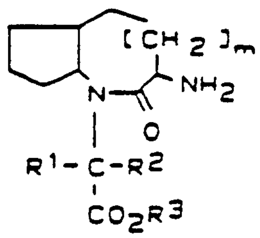

(III)

in which m, $R^1$, $R^2$ and $R^3$ are as defined above but $R^3$ does not denote hydrogen,

b) reacting a compound of the formula III defined above with a compound of the formula IV

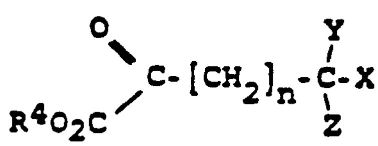

(IV)

in which n, $R^4$ and X have the above meanings and Y and Z each denote hydrogen, in the presence of a reducing agent,

c) alkylating a compound of the formula V

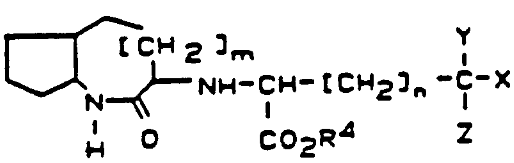

(V)

in which m, n, $R^4$, X, Y and Z are as defined above, with a compound of the formula VI

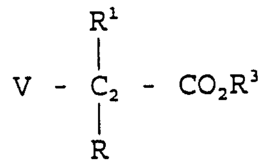

(VI)

in which $R^1$, $R^2$, $R^3$ and V are as defined above under a),

d) reacting a compound of the formula VII

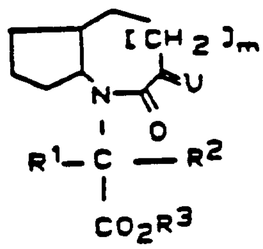

(VII)

in which m, $R^1$, $R^2$ and $R^3$ have the meanings defined above under a) and U denotes an oxo group, or together denotes hydrogen and a group V defined under a), with an amine of the formula VIII

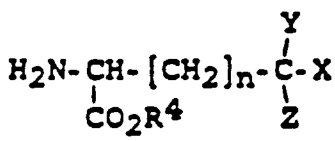

(VIII)

in which n, $R^4$ and X are as defined above under a) and Y and Z denote hydrogen, the reaction being carried out in the presence of a reducing agent in the case where U = oxo,

e) cyclizing a compound of the formula IX

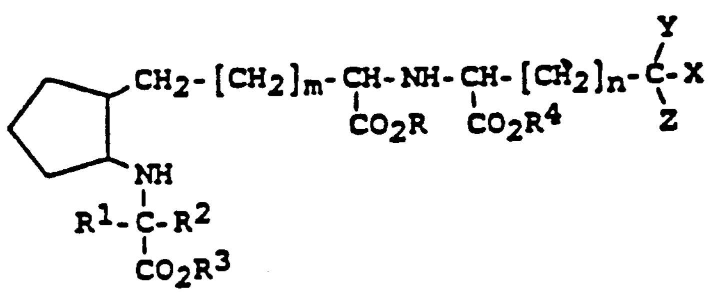

(IX)

in which m, n, $R^1$, $R^2$, $R^3$, $R^4$, X, Y and Z have the meanings given above under a) and R denotes hydrogen or an ester group, or

f) to prepare a compound of the formula I in which Y and Z together denote oxygen, reacting a compound of the formula III defined under a) with a compound of the formula X

$R^4O_2C\text{-}CH=CH\text{-}CO\text{-}X$ (X)

in which $R^4$ and X are as defined above in formula I, in a Michael reaction in a manner which is known per se or reacting the abovementioned compound of the formula III with a compound of the formula XI

$OHC\text{-}CO_2R^4$ (XI)

and a compound of the formula XII

X-CO-$CH_3$ (XII)

in which $R^4$ and X have the meaning as defined in above claim 1 in formula I, in a Mannich reaction in a manner which is known per se, and, if Y and Z together denote oxygen, reducing this group to, if appropriate, Y = hydroxyl or hydrogen and Z = hydrogen,

i) if desired eliminating in a manner which is known per se any protective groups introduced temporarily for protection of functional groups,

ii) if appropriate esterifying carboxyl groups $CO_2R^3$ and/or $CO_2R^4$ ($R^3$,$R^4$ = H) in a manner which is known per se to form a compound of the formula I ($R^3$ and/or $R^4 \neq$ H),

iii) if appropriate eliminating the radicals $R^3$ and/or $R^4$ ($R^3$, $R^4 \neq$ H) hydrolytically or hydrogenolytically to form the free carboxyl group(s),

or reversing the sequence of these steps i - iii,

and, if appropriate, converting the compound of the formula I obtained in this manner into a physiologically acceptable salt thereof.

**2.** The process as claimed in claim 1, wherein a compound of the formula I in which
m is 1 and
n is 1

| | |
|---|---|
| $R^1$ | is hydrogen, ($C_1$-$C_3$)-alkyl, ($C_3$-$C_6$)-cycloalkyl, ($C_3$-$C_6$)-cycloalkyl-($C_1$-$C_2$)-alkyl or allyl, |
| $R^2$ | is hydrogen, ($C_1$-$C_3$)-alkyl, ($C_3$-$C_6$)-cycloalkyl, ($C_3$-$C_6$)-cycloalkyl-($C_1$-$C_2$)-alkyl or allyl, |
| $R^3$ | is hydrogen, ($C_1$-$C_4$)-alkyl, benzyl or 4-methoxybenzyl, |
| $R^4$ | is hydrogen, ($C_1$-$C_4$)-alkyl, benzyl or 4-methoxybenzyl, |
| X | is phenyl or phenyl which is mono- or disubstituted by fluorine and/or chlorine, methyl, cyclohexyl or aminoethyl, |
| Y | denotes hydrogen or hydroxyl, and |
| Z | denotes hydrogen, or |
| Y and Z | together represent oxygen, is prepared. |

**3.** The process as claimed in either of claims 1 and 2, wherein a compound of the formula I in which
m is 1,
n is 1,

| | |
|---|---|
| $R^1$, $R^2$ and $R^3$ | each denote hydrogen, |
| $R^4$ | denotes hydrogen or ethyl, |
| Y and Z | each denote hydrogen and |
| X | denotes phenyl, is prepared. |

**4.** The process as claimed in claim 1, wherein a compound of the formula I in which the chiral carbon atoms labeled with an asterisk [(*)] have the S configuration is prepared.

**5.** The process as claimed in claim 1, wherein
1-carboxymethyl-3-(S)-[1-(S)-ethoxycarbonyl-3-phenylpropylamino]-2-oxo-perhydrocyclopent[b]azepine,
1-carboxymethyl-3-(S)-[1-(S)-carboxy-3-phenylpropylamino]-2-oxo-perhydrocyclopent[b]azepine,
1-carboxymethyl-3-(S)-[1-(S)-ethoxycarbonyl-butylamino]-2-oxo-perhydrocyclopent[b]azepine,
1-carboxymethyl-3-(S)-[1-(S)-carboxy-butylamino]-2-oxo-perhydrocyclopent[b]azepine,
1-carboxymethyl-3-(S)-[1-(S)-ethoxycarbonyl-5-amino-pentylamino]-2-oxo-perhydrocyclopent[b]azepine,
or 1-carboxymethyl-3-(S)-[1-(S)-carboxy-5-amino-pentylamino]-2-oxo-perhydrocyclopent[b]azepine, a stereoisomer thereof or a physiologically acceptable salt thereof is prepared.

**6.** The process as claimed in claim 1, wherein
1-carboxymethyl-3-(S)-[1-(S)-ethoxycarbonyl-3-phenylpropylamino]-2-oxo-perhydro-2H-cyclopent[b]-azocine,
1-carboxymethyl-3-(S)-[1-(S)-carboxy-3-phenylpropylamino]-2-oxo-perhydro-2H-cyclopent[b]azocine, a stereoisomer thereof or a physiologically acceptable salt thereof is prepared.

**7.** A process for the preparation of a compound of the formula XIII

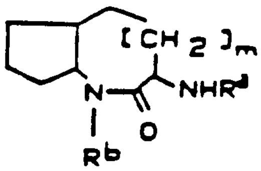

(XIII)

in which m denotes 1 or 2,

a) $R^a$ and $R^b$ each denote hydrogen,

b) $R^a$ denotes hydrogen and $R^b$ represents a radical

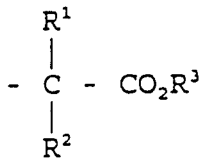

in which $R^1$, $R^2$ and $R^3$ are as defined in claim 1, or

c) $R^a$ represents a radical

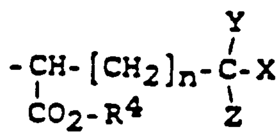

in which n, $R^4$, X, Y and Z are as defined in claim 1 and $R^b$ denotes hydrogen, or

d) $R^a$ represents a radical

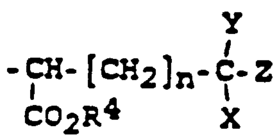

in which n, $R^4$, X, Y and Z are as defined above, and $R^b$ represents a radical

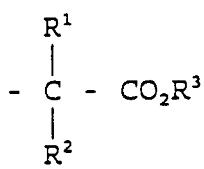

in which $R^1$, $R^2$ and $R^3$ are as defined above, which comprises

a) cyclizing a compound of the formula XIV

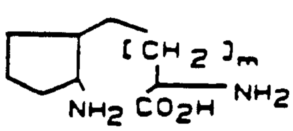

(XIV)

in which m denotes 1 or 2, to give a compound of the formula XV (≈ formula XIII where $R^a$ and $R^b$

are each hydrogen)

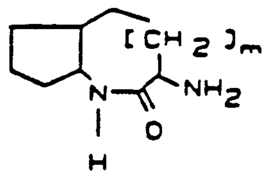

(XV)

in which m is as defined above,
b) reducing a compound of the formula XVI

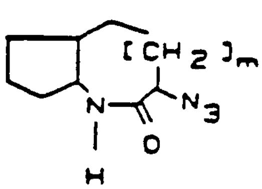

(XVI)

in which m is as defined above, to give a compound of the formula XV defined above,
c) if appropriate alkylating a compound of the above formula XV with a compound of the formula VI

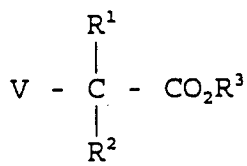

(VI)

in which V, $R^1$, $R^2$ and $R^3$ are as defined in claim 1 under c), to give a compound of the formula III (≃ formula XIII where $R^a$ = H, $R^b$ ≠ H),
d) if appropriate reacting a compound of the above formula XV with a compound of the formula II defined in claim 1) under a) to give a compound of the formula V (≃ formula XV where $R^a$ ≠ H, $R^b$ = H),
e) reacting a compound of the above formula XV with a compound of the formula X

$R^4O_2C\text{-}CH=CH\text{-}CO\text{-}X$ (X)

in which $R^4$ and X are as defined above in formula I, in a Michael reaction in a manner which is known per se, or reacting the abovementioned compound of the formula III with a compound of the formula XI

$OHC\text{-}CO_2R^4$ (XI)

and a compound of the formula XII

$X\text{-}CO\text{-}CH_3$ (XII)

in which $R^4$ and X have the meanings defined in claim 1 in formula I, in a Mannich reaction in a manner which is known per se, and, if Y and Z together denote oxygen, if appropriate reducing this group to Y = hydroxyl or hydrogen and Z = hydrogen,
f) or alkylating a compound of the formula XVI with a compound of the formula VI to give a compound of the formula XVII

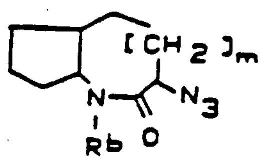 (XVII)

in which m and $R^b$ ($R^b \neq H$) are as defined above and converting this compound into a compound of the formula XIII ($R^a$ = H, $R^b \neq H$) by catalytic hydrogenation with a metal catalyst or by reaction with a complex hydride.

**8.** A process for the preparation of a pharmaceutical agent containing a compound prepared as claimed in any one of claims 1-6, which comprises bringing a compound of the formula I into a suitable administration form together with a physiologically acceptable excipient and if appropriate other auxiliaries.

**9.** The process as claimed in claim 8, wherein a diuretic as added.

**Claims for the following Contracting State : GR**

**1.** A process for the preparation of a compound of the formula I

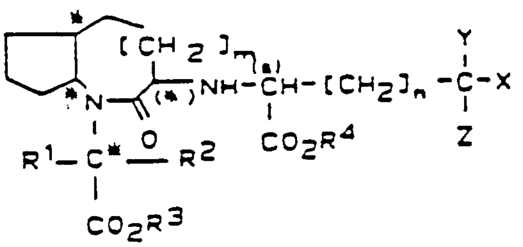 (I)

in which
m is 1 or 2 and
n is 0, 1 or 2

| | |
|---|---|
| $R^1$ and $R^2$ | are identical or different and denote hydrogen, ($C_1$-$C_6$)-alkyl, which can optionally be monosubstituted by hydroxyl, mercapto, ($C_1$-$C_2$)-alkoxy, ($C_1$-$C_2$)-alkylmercapto, carboxyl, ($C_1$-$C_2$)-alkoxycarbonyl, 3-indolyl, imidazolyl, carbamoyl, amino or guanidino, ($C_2$-$C_6$)-alkenyl, ($C_3$-$C_9$)-cycloalkyl, ($C_3$-$C_9$)-cycloalkenyl, ($C_3$-$C_7$)-cycloalkyl-($C_1$-$C_4$)-alkyl, ($C_6$-$C_{12}$)-aryl, partly hydrogenated ($C_6$-$C_{12}$)-aryl or ($C_6$-$C_{12}$)-aryl-($C_1$-$C_4$)-alkyl, which can carry a hydroxyl group in the aryl part, |
| $R^3$ and $R^4$ | are identical or different and denote hydrogen, ($C_1$-$C_6$)-alkyl, ($C_2$-$C_6$)-alkenyl or ($C_6$-$C_{12}$)-aryl-($C_1$-$C_4$)-alkyl, which can be monosubstituted in the aryl part by methoxy or nitro, |
| Y | denotes hydrogen or hydroxyl, |
| Z | denotes hydrogen or |
| Y and Z | together denote oxygen, and |
| X | represents ($C_1$-$C_6$)-alkyl, which can be substituted by amino, acylamino, ($C_1$-$C_4$)-alkylamin and/or di-($C_1$-$C_4$)-alkylamino, ($C_2$-$C_6$)-alkenyl, ($C_5$-$C_9$)-cycloalkyl, |

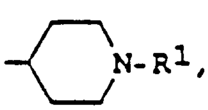

($C_6$-$C_{12}$)-aryl, which can be mono-, di- or trisubstituted by ($C_1$-$C_4$)-alkyl, ($C_1$-$C_4$)-alkoxy, hydroxyl, halogen, nitro, amino, ($C_1$-$C_4$)-alkylamino, di-($C_1$-$C_4$)-alkylamino and/or methylenedioxy, or indol-3-yl, and, in the abovementioned radicals $R^1$, $R^2$ and

X, free hydroxyl, mercapto, carboxyl, amino or guanidino are optionally protected by protective groups customary in peptide chemistry,

or a physiologically acceptable salt thereof, which comprises

a) reacting a compound of the formula II

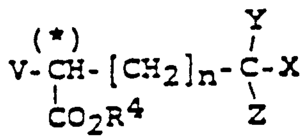

(II)

in which n, $R^4$, X, Y and Z are as defined above, but $R^4$ does not denote hydrogen, and V represents a leaving group which can be replaced nucleophilically, with a compound of the formula III

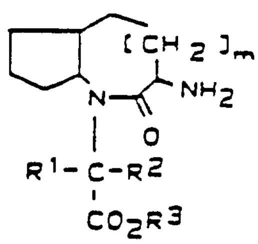

(III)

in which m, $R^1$, $R^2$ and $R^3$ are as defined above but $R^3$ does not denote hydrogen,

b) reacting a compound of the formula III defined above with a compound of the formula IV

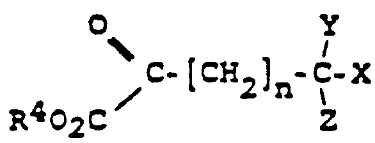

(IV)

in which n, $R^4$ and X have the above meanings and Y and Z each denote hydrogen, in the presence of a reducing agent,

c) alkylating a compound of the formula V

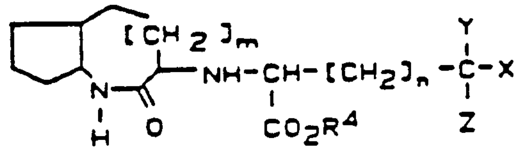

(V)

in which m, n, $R^4$, X, Y and Z are as defined above, with a compound of the formula VI

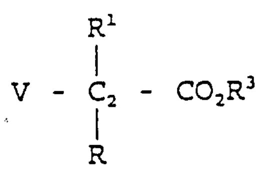

(VI)

in which $R^1$, $R^2$, $R^3$ and V are as defined above under a),

d) reacting a compound of the formula VII

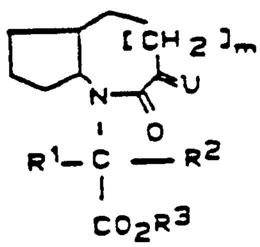

(VII)

in which m, $R^1$, $R^2$ and $R^3$ have the meanings defined above under a) and U denotes an oxo group, or together denotes hydrogen and a group V defined under a), with an amine of the formula VIII

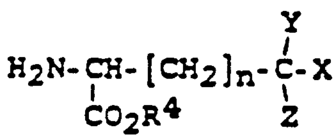

(VIII)

in which n, $R^4$ and X are as defined above under a) and Y and Z denote hydrogen, the reaction being carried out in the presence of a reducing agent in the case where U = oxo,

e) cyclizing a compound of the formula IX

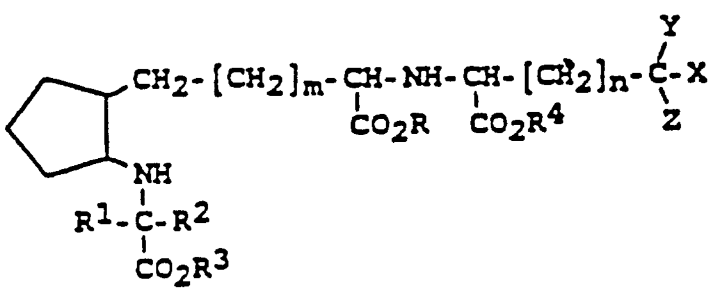

(IX)

in which m, n, $R^1$, $R^2$, $R^3$, $R^4$, X, Y and Z have the meanings given above under a) and R denotes hydrogen or an ester group, or

f) to prepare a compound of the formula I in which Y and Z together denote oxygen, reacting a compound of the formula III defined under a) with a compound of the formula X

$R^4O_2C\text{-}CH=CH\text{-}CO\text{-}X$ (X)

in which $R^4$ and X are as defined above in formula I, in a Michael reaction in a manner which is known per se or reacting the abovementioned compound of the formula III with a compound of the formula XI

$OHC\text{-}CO_2R^4$ (XI)

and a compound of the formula XII

$X\text{-}CO\text{-}CH_3$ (XII)

in which $R^4$ and X have the meaning as defined in above claim 1 in formula I, in a Mannich reaction in a manner which is known per se, and, if Y and Z together denote oxygen, reducing this group to, if appropriate, Y = hydroxyl or hydrogen and Z = hydrogen,

i) if desired eliminating in a manner which is known per se any protective groups introduced temporarily for protection of functional groups,

ii) if appropriate esterifying carboxyl groups $CO_2R^3$ and/or $CO_2R^4$ ($R^3$,$R^4$ = H) in a manner which is known per se to form a compound of the formula I ($R^3$ and/or $R^4$ ≠ H),
iii) if appropriate eliminating the radicals $R^3$ and/or $R^4$ ($R^3$, $R^4$ ≠ H) hydrolytically or hydrogenolytically to form the free carboxyl group(s),
or reversing the sequence of these steps i - iii,
and, if appropriate, converting the compound of the formula I obtained in this manner into a physiologically acceptable salt thereof.

**2.** The process as claimed in claim 1, wherein a compound of the formula I in which
m is 1 and
n is 1
$R^1$ is hydrogen, ($C_1$-$C_3$)-alkyl, ($C_3$-$C_6$)-cycloalkyl, ($C_3$-$C_6$)-cycloalkyl-($C_1$-$C_2$)-alkyl or allyl,
$R^2$ is hydrogen, ($C_1$-$C_3$)-alkyl, ($C_3$-$C_6$)-cycloalkyl, ($C_3$-$C_6$)-cycloalkyl-($C_1$-$C_2$)-alkyl or allyl,
$R^3$ is hydrogen, ($C_1$-$C_4$)-alkyl, benzyl or 4-methoxybenzyl,
$R^4$ is hydrogen, ($C_1$-$C_4$)-alkyl, benzyl or 4-methoxybenzyl,
X is phenyl or phenyl which is mono- or disubstituted by fluorine and/or chlorine, methyl, cyclohexyl or aminoethyl,
Y denotes hydrogen or hydroxyl, and
Z denotes hydrogen, or
Y and Z together represent oxygen, is prepared.

**3.** The process as claimed in either of claims 1 and 2, wherein a compound of the formula I in which
m is 1,
n is 1,
$R^1$, $R^2$ and $R^3$ each denote hydrogen,
$R^4$ denotes hydrogen or ethyl,
Y and Z each denote hydrogen and
X denotes phenyl, is prepared.

**4.** The process as claimed in claim 1, wherein a compound of the formula I in which the chiral carbon atoms labeled with an asterisk [(*)] have the S configuration is prepared.

**5.** The process as claimed in claim 1, wherein
1-carboxymethyl-3-(S)-[1-(S)-ethoxycarbonyl-3-phenylpropylamino]-2-oxo-perhydrocyclopent[b]azepine,
1-carboxymethyl-3-(S)-[1-(S)-carboxy-3-phenylpropylamino]-2-oxo-perhydrocyclopent[b]azepine,
1-carboxymethyl-3-(S)-[1-(S)-ethoxycarbonyl-butylamino]-2-oxo-perhydrocyclopent[b]azepine,
1-carboxymethyl-3-(S)-[1-(S)-carboxy-butylamino]-2-oxo-perhydrocyclopent[b]azepine,
1-carboxymethyl-3-(S)-[1-(S)-ethoxycarbonyl-5-amino-pentylamino]-2-oxo-perhydrocyclopent[b]azepine,
or
1-carboxymethyl-3-(S)-[1-(S)-carboxy-5-amino-pentylamino]-2-oxo-perhydrocyclopent[b]azepine, a stereoisomer thereof or a physiologically acceptable salt thereof is prepared.

**6.** The process as claimed in claim 1, wherein
1-carboxymethyl-3-(S)-[1-(S)-ethoxycarbonyl-3-phenylpropylamino]-2-oxo-perhydro-2H-cyclopent[b]-azocine,
1-carboxymethyl-3-(S)-[1-(S)-carboxy-3-phenylpropylamino]-2-oxo-perhydro-2H-cyclopent[b]azocine, a stereoisomer thereof or a physiologically acceptable salt thereof is prepared.

**7.** A process for the preparation of a compound of the formula XIII

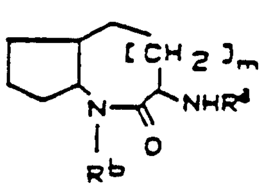

(XIII)

in which m denotes 1 or 2,

a) $R^a$ and $R^b$ each denote hydrogen,

b) $R^a$ denotes hydrogen and $R^b$ represents a radical

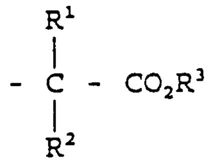

in which $R^1$, $R^2$ and $R^3$ are as defined in claim 1, or

c) $R^a$ represents a radical

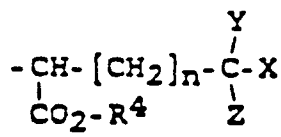

in which n, $R^4$, X, Y and Z are as defined in claim 1 and $R^b$ denotes hydrogen, or

d) $R^a$ represents a radical

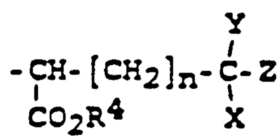 ,

in which n, $R^4$, X, Y and Z are as defined above, and $R^b$ represents a radical

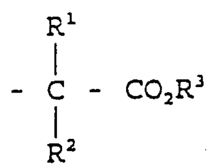

in which $R^1$, $R^2$ and $R^3$ are as defined above, which comprises

a) cyclizing a compound of the formula XIV

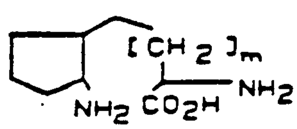 (XIV)

in which m denotes 1 or 2, to give a compound of the formula XV (≃ formula XIII where $R^a$ and $R^b$ are each hydrogen)

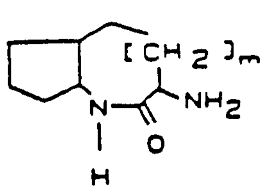 (XV)

in which m is as defined above,
b) reducing a compound of the formula XVI

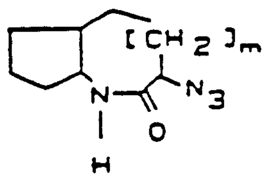

(XVI)

in which m is as defined above, to give a compound of the formula XV defined above,
c) if appropriate alkylating a compound of the above formula XV with a compound of the formula VI

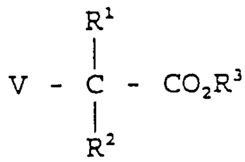

(VI)

in which V, $R^1$, $R^2$ and $R^3$ are as defined in claim 1 under c), to give a compound of the formula III (≈ formula XIII where $R^a = H$, $R^b \neq H$),
d) if appropriate reacting a compound of the above formula XV with a compound of the formula II defined in claim 1) under a) to give a compound of the formula V (≈ formula XV where $R^a \neq H$, $R^b = H$),
e) reacting a compound of the above formula XV with a compound of the formula X

$R^4O_2C\text{-}CH=CH\text{-}CO\text{-}X$ (X)

in which $R^4$ and X are as defined above in formula I, in a Michael reaction in a manner which is known per se, or reacting the abovementioned compound of the formula III with a compound of the formula XI

$OHC\text{-}CO_2R^4$ (XI)

and a compound of the formula XII

$X\text{-}CO\text{-}CH_3$ (XII)

in which $R^4$ and X have the meanings defined in claim 1 in formula I, in a Mannich reaction in a manner which is known per se, and, if Y and Z together denote oxygen, if appropriate reducing this group to Y = hydroxyl or hydrogen and Z = hydrogen,
f) or alkylating a compound of the formula XVI with a compound of the formula VI to give a compound of the formula XVII

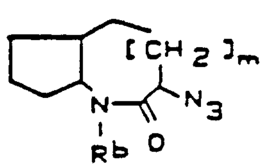

(XVII)

in which m and $R^b$ ($R^b \neq H$) are as defined above and converting this compound into a compound of the formula XIII ($R^a = H$, $R^b \neq H$) by catalytic hydrogenation with a metal catalyst or by reaction with a complex hydride.

**8.** A process for the preparation of a pharmaceutical agent containing a compound prepared as claimed in any one of claims 1-6, which comprises bringing a compound of the formula I into a suitable administration form together with a physiologically acceptable excipient and if appropriate other auxiliaries.

**9.** The process as claimed in claim 8, wherein a diuretic as added.

**10.** A compound of the formula XIII in which $R^a$, $R^b$ and m are as defined in claim 7.

**Revendications**
**Revendications pour les Etats contractants suivants : AT, BE, CH, DE, FR, GB, IT, LI, LU, NL, SE**

**1.** Composés de formule I

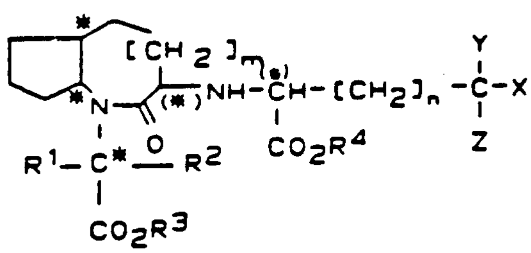

(I)

dans laquelle
m représente 1 ou 2 et
n représente 0, 1 ou 2,

$R^1$ et $R^2$ sont identiques ou différents et représentent un atome d'hydrogène ou un radical alkyle en $C_1$-$C_6$ qui peut éventuellement être monosubstitué par un groupe hydroxy, mercapto, alcoxy en $C_1$-$C_2$, alkyl($C_1$-$C_2$)-mercapto, carboxy, alcoxy($C_1$-$C_2$)-carbonyle, 3-indolyle, imidazolyle, carbamoyle, amino ou guanidino, un radical alcényle en $C_2$-$C_6$, cycloalkyle en $C_3$-$C_9$, cycloalcényle en $C_3$-$C_9$, cycloalkyl($C_3$-$C_7$)-alkyle($C_1$-$C_4$), aryle en $C_6$-$C_{12}$,un radical aryle en $C_6$-$C_{12}$ partiellement hydrogéné ou un radical aryle($C_6$-$C_{12}$)-alkyle($C_1$-$C_4$) qui peut porter un groupe hydroxy sur le fragment aryle,

$R^3$ et $R^4$ sont identiques ou différents et représentent un atome d'hydrogène ou un groupe alkyle en $C_1$-$C_6$, alcényle en $C_2$-$C_6$ ou aryl($C_6$-$C_{12}$)-alkyle($C_1$-$C_4$) qui peut être monosubstitué sur le fragment aryle par le groupe méthoxy ou nitro,

Y représente un atome d'hydrogène ou le groupe hydroxy,

Z représente un atome d'hydrogène ou

Y et Z représentent ensemble un atome d'oxygène et

X représente un radical alkyle en $C_1$-$C_6$ qui peut être substitué par un groupe amino, acylamino, alkyl($C_1$-$C_4$)-amino et/ou dialkyl($C_1$-$C_4$)-amino, un radical alcényle en $C_2$-$C_6$, cycloalkyle en $C_5$-$C_9$,

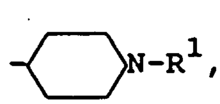

un radical aryle en $C_6$-$C_{12}$ qui peut être mono-, di- ou trisubstitué par un ou des atomes d'halogène ou groupes alkyle en $C_1$-$C_4$, alcoxy en $C_1$-$C_4$, hydroxy, nitro, amino, alkyl-($C_1$-$C_4$)-amino, dialkyl($C_1$-$C_4$)-amino et/ou méthylènedioxy, ou le radical indol-3-yle, dans les radicaux $R^1$, $R^2$ et X cités ci-dessus, les groupes hydroxy, mercapto, carboxy, amino ou guanidino libres étant éventuellement protégés par des groupes protecteurs usuels dans la chimie des peptides,

et sels physiologiquement acceptables de ceux-ci.

**2.** Composés de formule I selon la revendication 1, caractérisés en ce que
m = 1 et

n = 1,

| | |
|---|---|
| $R^1$ | représente un atome d'hydrogène ou un radical alkyle en $C_1$-$C_3$, cycloalkyle en $C_3$-$C_6$, cycloalkyl($C_3$-$C_6$)-alkyle-($C_1$-$C_2$) ou allyle, |
| $R^2$ | représente un atome d'hydrogène ou un radical alkyle en $C_1$-$C_3$, cycloalkyle en $C_3$-$C_6$, cycloalkyl($C_3$-$C_6$)-alkyle-($C_1$-$C_2$) ou allyle, |
| $R^3$ | représente un atome d'hydrogène ou un radical alkyle en $C_1$-$C_4$, benzyle ou 4-méthoxy-benzyle, |
| $R^4$ | représente un atome d'hydrogène ou un radical alkyle en $C_1$-$C_4$, benzyle ou 4-méthoxy-benzyle, |
| X | représente le radical phényle ou un radical phényle mono-ou disubstitué par un ou des atomes de fluor et/ou de chlore, le radical méthyle, cyclohexyle ou aminoéthyle, |
| Y | représente un atome d'hydrogène ou le groupe hydroxy, et |
| Z | représente un atome d'hydrogène ou |
| Y et Z | représentent ensemble un atome d'oxygène. |

**3.** Composés de formule I selon l'une des revendications 1 et 2, caractérisés en ce que
m = 1,
n = 1,

| | |
|---|---|
| $R^1$, $R^2$ et $R^3$ | représentent chacun un atome d'hydrogène, |
| $R^4$ | représente un atome d'hydrogène ou le groupe éthyle, |
| Y et Z | représentent chacun un atome d'hydrogène et |
| X | représente le groupe phényle. |

**4.** Composés de formule I selon la revendication 1, caractérisés en ce que les atomes de carbone chiraux indiqués par un astérisque [(*)] ont la configuration S.

**5.** 1-carboxyméthyl-3-(S)-[1-(S)-éthoxycarbonyl-3-phénylpropylamino]-2-oxo-perhydrocyclopent[b]azépine,
1-carboxyméthyl-3-(S)-[1-(S)-carboxy-3-phénylpropylamino]-2-oxo-perhydrocyclopent[b]azépine,
1-carboxyméthyl-3-(S)-[1-(S)-éthoxycarbonyl-butylamino]-2-oxo-perhydrocyclopent[b]azépine,
1-carboxyméthyl-3-(S)-[1-(S)-carboxy-butylamino]-2-oxo-perhydrocyclopent[b]azépine,
1-carboxyméthyl-3-(S)-[1-(S)-éthoxycarbonyl-5-aminopentylamino]-2-oxo-perhydrocyclopent[b]-azépine,
1-carboxyméthyl-3-(S)-[1-(S)-carboxy-5-aminopentylamino]-2-oxo-perhydrocyclopent[b]azépine,
leurs stéréoisomères et leurs sels physiologiquement acceptables.

**6.** 1-carboxyméthyl-3-(S)-[1-(S-)éthoxycarbonyl-3-phénylpropylamino]-2-oxo-perhydro-2H-cyclopent[b]-azocine,
1-carboxyméthyl-3-(S)-[1-(S)-carboxy-3-phénylpropylamino]-2-oxo-perhydro-2H-cyclopent[b]-azocine,
leurs stéréoisomères et leurs sels physiologiquement acceptables.

**7.** Composé selon une ou plusieurs des revendications 1 à 6, pour utilisation en tant que médicament.

**8.** Produit contenant un composé selon une ou plusieurs des revendications 1 à 6 et un véhicule physiologiquement acceptable.

**9.** Produit selon la revendication 8, contenant un composé selon une ou plusieurs des revendications 1 à 6, en association avec un diurétique.

**10.** Composé selon une ou plusieurs des revendications 1 à 6, pour utilisation en tant que médicament dans le traitement de l'hypertension artérielle ou de cardiopathies.

**11.** Procédé pour la préparation de composés de formule I selon la revendication 1, caractérisé en ce que

a) on fait réagir un composé de formule II

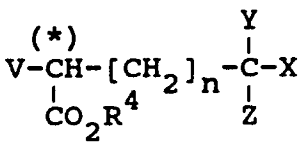

(II)

dans laquelle n, $R^4$, X, Y et Z sont tels que définis dans la revendication 1, $R^4$ ne représente toutefois pas un atome d'hydrogène et V représente un groupe séparable par substitution nucléophile, avec un composé de formule III

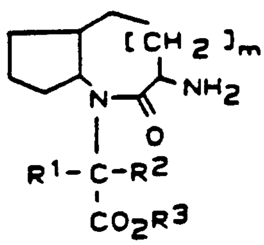

(III)

dans laquelle m, $R^1$, $R^2$ et $R^3$ sont tels que définis dans la revendication 1, mais $R^3$ ne représente pas un atome d'hydrogène,

b) on fait réagir en présence d'un réducteur un composé de formule III définie précédemment, avec un composé de formule IV

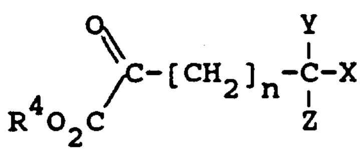

(IV)

dans laquelle n, $R^4$ et X ont les significations précédentes et Y et Z représentent chacun un atome d'hydrogène,

c) on soumet à une alkylation un composé de formule V

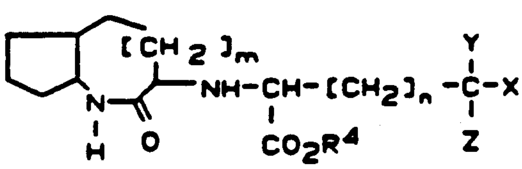

(V)

dans laquelle m, n, $R^4$, X, Y et Z sont tels que définis plus haut, avec un composé de formule VI

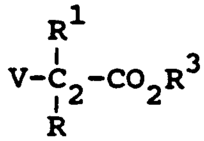

(VI)

dans laquelle $R^1$, $R^2$, $R^3$ et V sont tels que définis en a),

d) on fait réagir un composé de formule VII

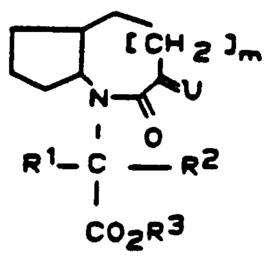

(VII)

dans laquelle m, $R^1$, $R^2$ et $R^3$ ont les significations définies en a) et U représente un groupe oxo ou conjointement un atome d'hydrogène et un groupe V défini en a), avec une amine de formule VIII

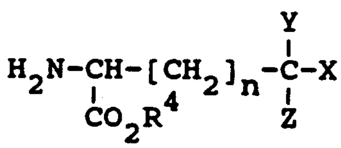

(VIII)

dans laquelle n, $R^4$ et X sont tels que définis plus haut en a) et Y et Z représentent des atomes d'hydrogène, dans le cas où U = oxo, la réaction étant effectuée en présence d'un réducteur,

e) On soumet à une cyclisation un composé de formule IX

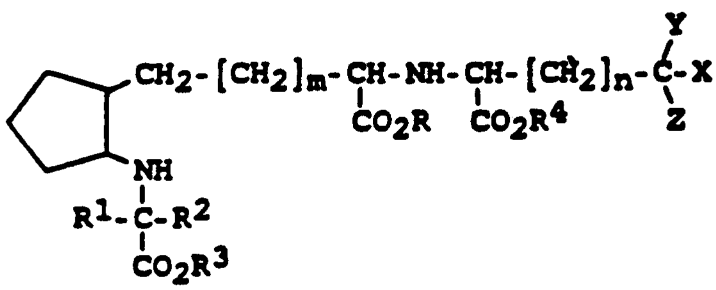

(IX)

dans laquelle m, n, $R^1$, $R^2$, $R^3$, $R^4$, X, Y et Z ont les significations données en a) et R représente un atome d'hydrogène ou un groupe ester, ou

f) pour la préparation des composés de formule I dans lesquels Y et Z représentent ensemble un atome d'oxygène, on fait réagir d'une façon connue en soi, dans une réaction de Michael, un composé de formule III, définie en a), avec un composé de formule X

$R^4O_2C\text{-}CH=CH\text{-}CO\text{-}X$ (X)

dans laquelle $R^4$ et X sont tels que définis plus haut dans la formule I, ou on fait réagir le composé précité de formule III, d'une façon connue en soi, dans une réaction de Mannich, avec un composé de formule XI

$OHC\text{-}CO_2R^4$ (XI)

et avec un composé de formule XII

$X\text{-}CO\text{-}CH_3$ (XII)

formules dans lesquelles $R^4$ et X ont les significations définies à propos de la formule I dans la revendication 1, dans le cas où Y et Z représentent ensemble un atome d'oxygène, éventuellement on réduit ce groupe en Y = OH ou H et Z = H,

I) si on le désire, on élimine d'une façon connue en soi des groupes protecteurs éventuellement introduits temporairement pour la protection de groupes fonctionnels,

II) éventuellement on estérifie des groupes carboxy $CO_2R^3$ et/ou $CO_2R^4$ ($R^3$, $R^4$ = H), d'une façon connue en soi, avec formation des composés de formule I ($R^3$ et/ou $R^4$ ≠ H),

III) éventuellement on élimine par hydrolyse ou hydrogénolyse les radicaux $R^3$ et/ou $R^4$ ($R^3$, $R^4$ ≠ H), avec formation du(des) groupe(s) carboxy libre(s),

ou on inverse l'ordre de ces étapes I-III, et on convertit éventuellement en leurs sels physiologiquement acceptables les composés de formule I obtenus de cette façon.

**12.** Composés de formule XIII

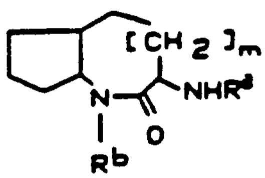

(XIII)

dans laquelle m représente 1 ou 2,

a) $R^a$ et $R^b$ représentent chacun un atome d'hydrogène,

b) $R^a$ représente un atome d'hydrogène et $R^b$ représente un radical

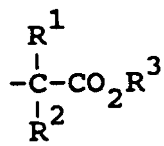

dans lequel $R^1$, $R^2$ et $R^3$ sont tels que définis dans la revendication 1, ou

c) $R^a$ représente un radical

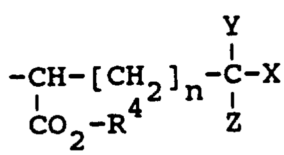

dans lequel n, $R^4$, X, Y et Z sont tels que définis dans la revendication 1, et $R^b$ représente un atome d'hydrogène, ou

d) $R^a$ représente un radical

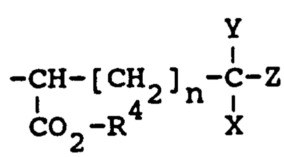

dans lequel n, $R^4$, X, Y et Z sont tels que définis plus haut, et $R^b$ représente un radical

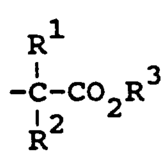

dans lequel $R^1$, $R^2$ et $R^3$ sont tels que définis plus haut.

**13.** Procédé pour la préparation de composés de formule XIII selon la revendication 12 caractérisé en ce que

a) on soumet à une cyclisation des composés de formule XIV

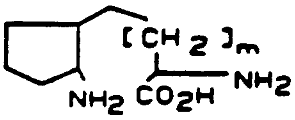

(XIV)

dans laquelle m représente 1 ou 2, pour aboutir aux composés de formule XV (≅ formule XIII dans laquelle $R^a$ et $R^b$ représentent chacun un atome d'hydrogène)

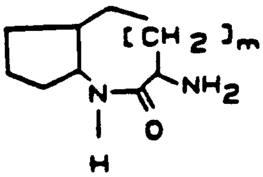

(XV)

dans laquelle m est tel que défini plus haut,

b) on réduit des composés de formule XVI

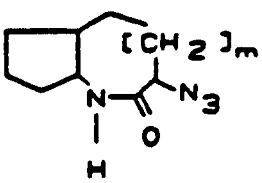

(XVI)

dans laquelle m est tel que défini plus haut, pour aboutir aux composés de formule XV définie plus haut,

c) on soumet à une alkylation des composés de formule XV ci-dessus, éventuellement avec des composés de formule VI,

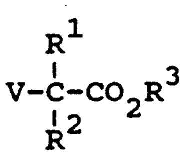

(VI)

dans laquelle V, $R^1$, $R^2$ et $R^3$ sont tels que définis en c) dans la revendication 12, pour aboutir aux composés de formule III (≅ formule XIII dans laquelle $R^a$ = H, $R^b$ ≠ H),

d) on fait réagir des composés de formule XV ci-dessus, éventuellement avec des composés de formule II définis en a) dans la revendication 11 pour aboutir aux composés de formule V (≅ formule XV dans laquelle $R^a$ ≠ H, $R^b$ = H),

e) on fait réagir d'une façon connue en soi, dans une réaction de Michael, des composés de formule XV ci-dessus avec un composé de formule X

$R^4O_2C\text{-}CH=CH\text{-}CO\text{-}X$ (X)

dans laquelle $R^4$ et x sont tels que définis plus haut dans la formule I, ou on fait réagir le composé précité de formule III, d'une façon connue en soi, dans une réaction de Mannich, avec un composé de formule XI

$OHC\text{-}CO_2R^4$ (XI)

et avec un composé de formule XII

$X\text{-}CO\text{-}CH_3$ (XII)

formules dans lesquelles $R^4$ et X ont les significations définies à propos de la formule I dans la revendication 1, dans le cas où Y et Z représentent ensemble un atome d'oxygène, éventuellement on réduit ce groupe en Y = OH ou H et Z = H,
f) ou on soumet à une alkylation un composé de formule XVI, avec des composés de formule VI, pour aboutir aux composés de formule XVII

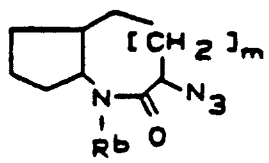

(XVII)

dans laquelle m et $R^b$ ($R^b \neq H$) sont tels que définis dans la revendication 12, et on convertit ces composés, par hydrogénation catalytique, avec des catalyseurs métalliques, ou par réaction avec des complexes dans des hydrures, en composés de formule XIII ($R^a$ = H, $R^b \neq H$).

**14.** Procédé pour la préparation d'un produit selon la revendication 8, caractérisé en ce que l'on met sous une forme d'administration appropriée un composé de formule I, conjointement avec un véhicule physiologiquement acceptable et éventuellement d'autres adjuvants.

**Revendications pour l'Etat contractant suivant : ES**

**1.** Procédé pour la préparation de composés de formule I

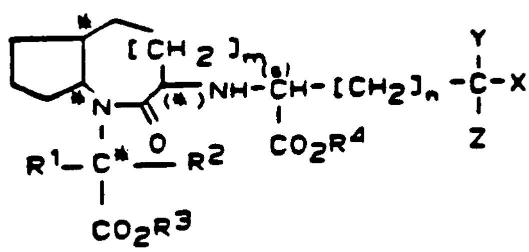

(I)

dans laquelle
m représente 1 ou 2 et
n représente 0, 1 ou 2,

$R^1$ et $R^2$ sont identiques ou différents et représentent un atome d'hydrogène ou un radical alkyle en $C_1$-$C_6$ qui peut éventuellement être monosubstitué par un groupe hydroxy, mercapto, alcoxy en $C_1$-$C_2$, alkyl($C_1$-$C_2$)-mercapto, carboxy, alcoxy($C_1$-$C_2$)-carbonyle, 3-indolyle, imidazolyle, carbamoyle, amino ou guanidino, un radical alcényle en $C_2$-$C_6$, cycloalkyle en $C_3$-$C_9$, cycloalcényle en $C_3$-$C_9$, cycloalkyl($C_3$-$C_7$)-alkyle($C_1$-$C_4$), aryle en $C_6$-$C_{12}$,un radical aryle en $C_6$-$C_{12}$ partiellement hydrogéné ou un radical aryle($C_6$-$C_{12}$)-alkyle($C_1$-$C_4$) qui peut porter un groupe hydroxy sur le fragment aryle,

$R^3$ et $R^4$ sont identiques ou différents et représentent un atome d'hydrogène ou un groupe alkyle en $C_1$-$C_6$, alcényle en $C_2$-$C_6$ ou aryl($C_6C_{12}$)-alkyle($C_1$-$C_4$) qui peut être monosubstitué sur le fragment aryle par le groupe méthoxy ou nitro,

Y représente un atome d'hydrogène ou le groupe hydroxy,

Z représente un atome d'hydrogène ou

Y et Z représentent ensemble un atome d'oxygène et

X représente un radical alkyle en $C_1$-$C_6$ qui peut être substitué par un groupe amino, acylamino, alkyl($C_1$-$C_4$)-amino et/ou dialkyl($C_1$-$C_4$)-amino, un radical alcényle en $C_2$-$C_6$,

cycloalkyle en $C_5$-$C_9$,

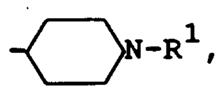

un radical aryle en $C_6$-$C_{12}$ qui peut être mono-, di- ou trisubstitué par un ou des atomes d'halogène ou groupes alkyle en $C_1$-$C_4$, alcoxy en $C_1$-$C_4$, hydroxy, nitro, amino, alkyl-($C_1$-$C_4$)-amino, dialkyl($C_1$-$C_4$)-amino et/ou méthylènedioxy, ou le radical indol-3-yle, dans les radicaux $R^1$, $R^2$ et X cités ci-dessus, les groupes hydroxy, mercapto, carboxy, amino ou guanidino libres étant éventuellement protégés par des groupes protecteurs usuels dans la chimie des peptides,

ainsi que de leurs sels physiologiquement acceptables, caractérisé en ce que

a) on fait réagir un composé de formule II

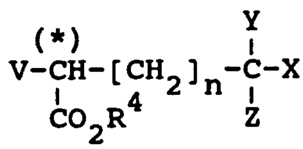

(II)

dans laquelle n, $R^4$, X, Y et Z sont tels que définis plus haut, $R^4$ ne représente toutefois pas un atome d'hydrogène et V représente un groupe séparable par substitution nucléophile, avec un composé de formule III

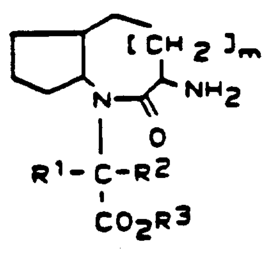

(III)

dans laquelle m, $R^1$, $R^2$ et $R^3$ sont tels que définis plus haut, mais $R^3$ ne représente pas un atome d'hydrogène,

b) on fait réagir en présence d'un réducteur un composé de formule III définie précédemment, avec un composé de formule IV

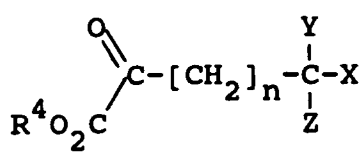

(IV)

dans laquelle n, $R^4$ et X ont les significations précédentes et Y et Z représentent chacun un atome d'hydrogène,

c) on soumet à une alkylation un composé de formule V

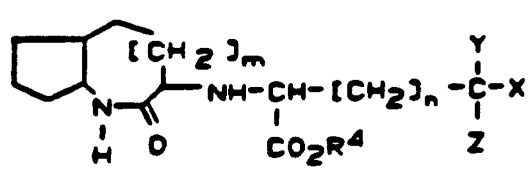

(V)

dans laquelle m, n, $R^4$, X, Y et Z sont tels que définis plus haut, avec un composé de formule VI

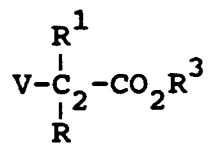

(VI)

dans laquelle $R^1$, $R^2$, $R^3$ et V sont tels que définis en a),
d) on fait réagir un composé de formule VII

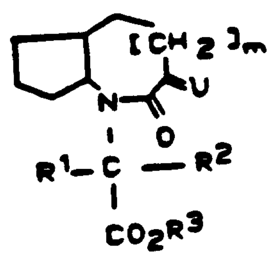

(VII)

dans laquelle m, $R^1$, $R^2$ et $R^3$ ont les significations définies en a) et U représente un groupe oxo ou conjointement un atome d'hydrogène et un groupe V défini en a), avec une amine de formule VIII

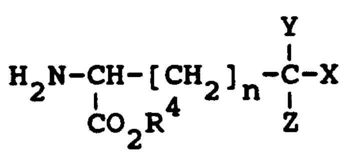

(VIII)

dans laquelle n, $R^4$ et X sont tels que définis plus haut en a) et Y et Z représentent des atomes d'hydrogène, dans le cas où U = oxo, la réaction étant effectuée en présence d'un réducteur,
e) On soumet à une cyclisation un composé de formule IX

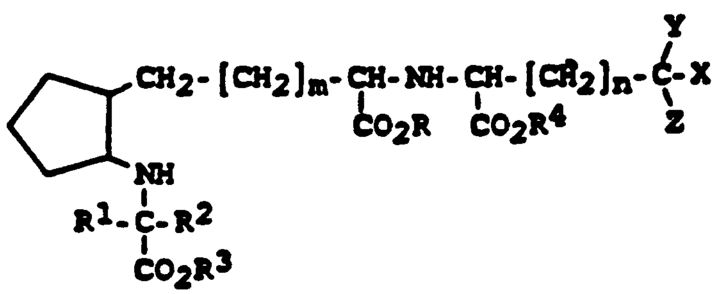

(IX)

dans laquelle m, n, $R^1$, $R^2$, $R^3$, $R^4$, X, Y et Z ont les significations données en a) et R représente un atome d'hydrogène ou un groupe ester, ou
f) pour la préparation des composés de formule I dans lesquels Y et Z représentent ensemble un atome d'oxygène, on fait réagir d'une façon connue en soi, dans une réaction de Michael, un composé de formule III, définie en a), avec un composé de formule X

$R^4O_2C\text{-}CH=CH\text{-}CO\text{-}X$ (X)

dans laquelle $R^4$ et X sont tels que définis plus haut dans la formule I, ou on fait réagir le composé précité de formule III, d'une façon connue en soi, dans une réaction de Mannich, avec un composé de formule XI

$OHC\text{-}CO_2R^4$ (XI)

et avec un composé de formule XII

$X\text{-}CO\text{-}CH_3$ (XII)

formules dans lesquelles $R^4$ et X ont les significations définies à propos de la formule I, dans le cas où Y et Z représentent ensemble un atome d'oxygène, éventuellement on réduit ce groupe en Y = OH ou H et Z = H,

I) si on le désire, on élimine d'une façon connue en soi des groupes protecteurs éventuellement introduits temporairement pour la protection de groupes fonctionnels,

II) éventuellement on estérifie des groupes carboxy $CO_2R^3$ et/ou $CO_2R^4$ ($R^3$, $R^4$ = H), d'une façon connue en soi, avec formation des composés de formule I ($R^3$ et/ou $R^4 \neq H$),

III) éventuellement on élimine par hydrolyse ou hydrogénolyse les radicaux $R^3$ et/ou $R^4$ ($R^3$, $R^4 \neq$ H), avec formation du (des) groupe(s) carboxy libre(s),

ou on inverse l'ordre de ces étapes I-III, et on convertit éventuellement en leurs sels physiologiquement acceptables les composés de formule I obtenus de cette façon.

**2.** Procédé selon la revendication 1, caractérisé en ce que l'on prépare des composés de formule I dans lesquels
m = 1 et
n = 1,

| | |
|---|---|
| $R^1$ | représente un atome d'hydrogène ou un radical alkyle en $C_1$-$C_3$, cycloalkyle en $C_3$-$C_6$, cycloalkyl($C_3$-$C_6$)-alkyle-($C_1$-$C_2$) ou allyle, |
| $R^2$ | représente un atome d'hydrogène ou un radical alkyle en $C_1$-$C_3$, cycloalkyle en $C_3$-$C_6$, cycloalkyl($C_3$-$C_6$)-alkyle-($C_1$-$C_2$) ou allyle, |
| $R^3$ | représente un atome d'hydrogène ou un radical alkyle en |
| $R^4$ | représente un atome d'hydrogène ou un radical alkyle en $C_1$-$C_4$, benzyle ou 4-méthoxy-benzyle, |
| X | représente le radical phényle ou un radical phényle mono-ou disubstitué par un ou des atomes de fluor et/ou de chlore, le radical méthyle, cyclohexyle ou aminoéthyle, |
| Y | représente un atome d'hydrogène ou le groupe hydroxy, et |
| Z | représente un atome d'hydrogène ou |
| Y et Z | représentent ensemble un atome d'oxygène. |

**3.** Procédé selon la revendication 1 ou 2, caractérisé en ce que l'on prépare des composés de formule I dans lesquels
m = 1,
n = 1,

| | |
|---|---|
| $R^1$, $R^2$ et $R^3$ | représentent chacun un atome d'hydrogène, |
| $R^4$ | représente un atome d'hydrogène ou le groupe éthyle, |
| Y et Z | représentent chacun un atome d'hydrogène et |
| X | représente le groupe phényle. |

**4.** Procédé selon la revendication 1, caractérisé en ce que l'on prépare des composés de formule I dans lesquels les atomes de carbone chiraux indiqués par un astérisque [(*)] ont la configuration S.

**5.** Procédé selon la revendication 1, caractérisé en ce que l'on prépare

la 1-carboxyméthyl-3-(S)-[1-(S)-éthoxy-carbonyl-3-phénylpropylamino]-2-oxo-perhydrocyclopent[b]-azépine,

la 1-carboxyméthyl-3-(S)-[1-(S)-carboxy-3-phénylpropylamino]-2-oxo-perhydrocyclopent[b]azépine,

la 1-carboxyméthyl-3-(S)-[1-(S)-éthoxycarbonyl-butylamino]-2-oxo-perhydrocyclopent[b]azépine,

la 1-carboxyméthyl-3-(S)-[1-(S)-carboxy-butylamino]-2-oxo-perhydrocyclopent[b]azépine,

la 1-carboxyméthyl-3-(S)-[1-(S)-éthoxycarbonyl-5-aminopentylamino]-2-oxo-perhydrocyclopent[b]-azépine,

la 1-carboxyméthyl-3-(S)-[1-(S)-carboxy-5-aminopentylamino]-2-oxo-perhydrocyclopent[b]azépine,

leurs stéréoisomères ou leurs sels physiologiquement acceptables.

**6.** Procédé selon la revendication 1, caractérisé en ce que l'on prépare
la 1-carboxyméthyl-3-(S)-[1-(S)-éthoxycarbonyl-3-phénylpropylamino]-2-oxo-perhydro-2H-cyclopent-[b]azocine,
la 1-carboxyméthyl-3-(S)-[1-(S)-carboxy-3-phénylpropylamino]-2- oxo-perhydro-2H-cyclopent[b]-azocine,
leurs stéréoisomères et leurs sels physiologiquement acceptables.

**7.** Procédé pour la préparation de composés de formule XIII

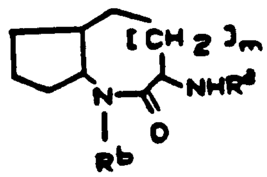

(XIII)

dans laquelle m représente 1 ou 2,
a) $R^a$ et $R^b$ représentent chacun un atome d'hydrogène,
b) $R^a$ représente un atome d'hydrogène et $R^b$ représente un radical

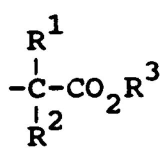

dans lequel $R^1$, $R^2$ et $R^3$ sont tels que définis dans la revendication 1, ou
c) $R^a$ représente un radical

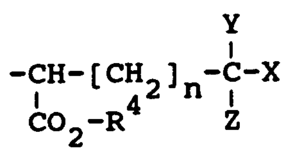

dans lequel n, $R^4$, X, Y et Z sont tels que définis dans la revendication 1, et $R^b$ représente un atome d'hydrogène, ou
d) $R^a$ représente un radical

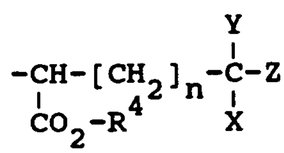

dans lequel n, $R^4$, X, Y et Z sont tels que définis plus haut, et $R^b$ représente un radical

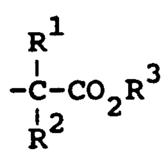

dans lequel $R^1$, $R^2$ et $R^3$ sont tels que définis plus haut,
caractérisé en ce que

a) on soumet à une cyclisation des composés de formule XIV

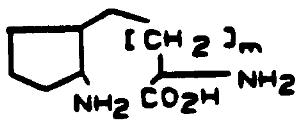

(XIV)

dans laquelle m représente 1 ou 2, pour aboutir aux composés de formule XV (≅ formule XIII dans laquelle $R^a$ et $R^b$ représentent chacun un atome d'hydrogène)

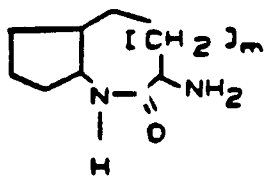

(XV)

dans laquelle m est tel que défini plus haut,
b) on réduit des composés de formule XVI

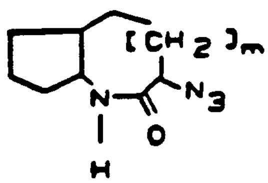

(XVI)

dans laquelle m est tel que défini plus haut, pour aboutir aux composés de formule XV définie plus haut,
c) on soumet à une alkylation des composés de formule XV ci-dessus, éventuellement avec des composés de formule VI,

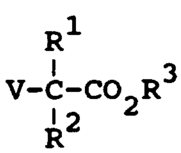

(VI)

dans laquelle V, $R^1$, $R^2$ et $R^3$ sont tels que définis en c) dans la revendication 1, pour aboutir aux composés de formule III (≅ formule XIII dans laquelle $R^a$ = H, $R^b$ ≠ H),
d) on fait réagir des composés de formule XV ci-dessus, éventuellement avec des composés de formule II définis en a) dans la revendication 1, pour aboutir aux composés de formule V (≅ formule XV dans laquelle $R^a$ ≠ H, $R^b$ = H),
e) on fait réagir d'une façon connue en soi, dans une réaction de Michael, des composés de formule XV ci-dessus avec un composé de formule X

$R^4O_2C\text{-}CH=CH\text{-}CO\text{-}X$ (X)

dans laquelle $R^4$ et x sont tels que définis plus haut dans la formule I, ou on fait réagir le composé précité de formule III, d'une façon connue en soi, dans une réaction de Mannich, avec un composé de formule XI

$OHC\text{-}CO_2R^4$ (XI)

et avec un composé de formule XII

X-CO-$CH_3$ (XII)

formules dans lesquelles $R^4$ et X ont les significations définies à propos de la formule I dans la revendication 1, dans le cas où Y et Z représentent ensemble un atome d'oxygène, éventuellement on réduit ce groupe en Y = OH ou H et Z = H,

f) ou on soumet à une alkylation un composé de formule XVI, avec des composés de formule VI, pour aboutir aux composés de formule XVII

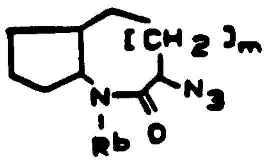

(XVII)

dans laquelle m et $R^b$ ($R^b \neq H$) sont tels que définis dans la revendication 7, et on convertit ces composés, par hydrogénation catalytique, avec des catalyseurs métalliques, ou par réaction avec des complexes dans des hydrures, en composés de formule XIII ($R^a$ = H, $R^b \neq H$).

**8.** Procédé pour la fabrication d'un produit pharmaceutique contenant un composé préparé selon l'une des revendications 1 à 6, caractérisé en ce que l'on met sous une forme d'administration appropriée un composé de formule I, conjointement avec un véhicule physiologiquement acceptable et éventuellement d'autres adjuvants.

**9.** Procédé selon la revendication 8, caractérisé en ce que l'on ajoute un diurétique.

**Revendications pour l'Etat contractant suivant : GR**

**1.** Procédé pour la préparation de composés de formule I

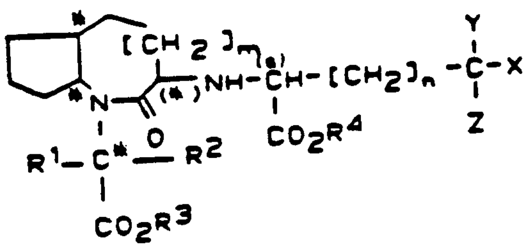

(I)

dans laquelle
m représente 1 ou 2 et
n représente 0, 1 ou 2,

$R^1$ et $R^2$ sont identiques ou différents et représentent un atome d'hydrogène ou un radical alkyle en $C_1$-$C_6$ qui peut éventuellement être monosubstitué par un groupe hydroxy, mercapto, alcoxy en $C_1$-$C_2$, alkyl($C_1$-$C_2$)-mercapto, carboxy, alcoxy($C_1$-$C_2$)-carbonyle, 3-indolyle, imidazolyle, carbamoyle, amino ou guanidino, un radical alcényle en $C_2$-$C_6$, cycloalkyle en $C_3$-$C_9$, cycloalcényle en $C_3$-$C_9$, cycloalkyl($C_3$-$C_7$)-alkyle($C_1$-$C_4$), aryle en $C_6$-$C_{12}$,un radical aryle en $C_6$-$C_{12}$ partiellement hydrogéné ou un radical aryle($C_6$-$C_{12}$)-alkyle($C_1$-$C_4$) qui peut porter un groupe hydroxy sur le fragment aryle,

$R^3$ et $R^4$ sont identiques ou différents et représentent un atome d'hydrogène ou un groupe alkyle en $C_1$-$C_6$, alcényle en $C_2$-$C_6$ ou aryl($C_6$-$C_{12}$)-alkyle($C_1$-$C_4$) qui peut être monosubstitué sur le fragment aryle par le groupe méthoxy ou nitro,

Y représente un atome d'hydrogène ou le groupe hydroxy,

Z représente un atome d'hydrogène ou

Y et Z représentent ensemble un atome d'oxygène et

X représente un radical alkyle en $C_1$-$C_6$ qui peut être substitué par un groupe amino,

acylamino, alkyl($C_1$-$C_4$)-amino et/ou dialkyl($C_1$-$C_4$)-amino, un radical alcényle en $C_2$-$C_6$, cycloalkyle en $C_5$-$C_9$,

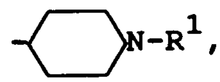

un radical aryle en $C_6$-$C_{12}$ qui peut être mono-, di- ou trisubstitué par un ou des atomes d'halogène ou groupes alkyle en $C_1$-$C_4$, alcoxy en $C_1$-$C_4$, hydroxy, nitro, amino, alkyl-($C_1$-$C_4$)-amino, dialkyl($C_1$-$C_4$)-amino et/ou méthylènedioxy, ou le radical indol-3-yle, dans les radicaux $R^1$, $R^2$ et X cités ci-dessus, les groupes hydroxy, mercapto, carboxy, amino ou guanidino libres étant éventuellement protégés par des groupes protecteurs usuels dans la chimie des peptides,

ainsi que de leurs sels physiologiquement acceptables, caractérisé en ce que

a) on fait réagir un composé de formule II

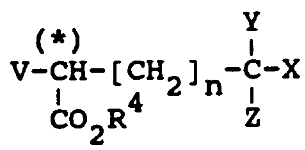

(II)

dans laquelle n, $R^4$, X, Y et Z sont tels que définis plus haut, $R^4$ ne représente toutefois pas un atome d'hydrogène et V représente un groupe séparable par substitution nucléophile, avec un composé de formule III

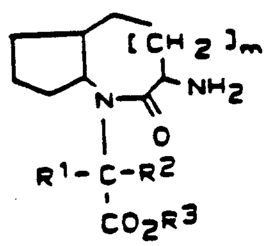

(III)

dans laquelle m, $R^1$, $R^2$ et $R^3$ sont tels que définis plus haut, mais $R^3$ ne représente pas un atome d'hydrogène,

b) on fait réagir en présence d'un réducteur un composé de formule III définie précédemment, avec un composé de formule IV

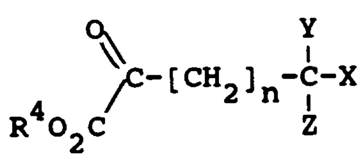

(IV)

dans laquelle n, $R^4$ et X ont les significations précédentes et Y et Z représentent chacun un atome d'hydrogène,

c) on soumet à une alkylation un composé de formule V

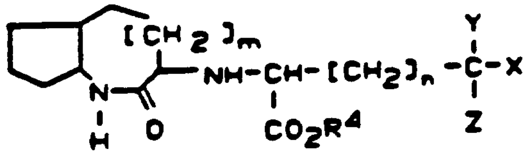

(V)

dans laquelle m, n, $R^4$, X, Y et Z sont tels que définis plus haut, avec un composé de formule VI

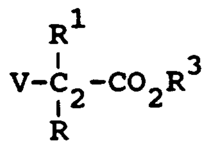

(VI)

dans laquelle $R^1$, $R^2$, $R^3$ et V sont tels que définis en a),
d) on fait réagir un composé de formule VII

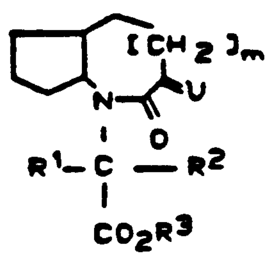

(VII)

dans laquelle m, $R^1$, $R^2$ et $R^3$ ont les significations définies en a) et U représente un groupe oxo ou conjointement un atome d'hydrogène et un groupe V défini en a), avec une amine de formule VIII

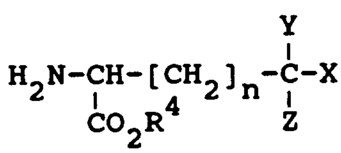

(VIII)

dans laquelle n, $R^4$ et X sont tels que définis plus haut en a) et Y et Z représentent des atomes d'hydrogène, dans le cas où U = oxo, la réaction étant effectuée en présence d'un réducteur,
e) On soumet à une cyclisation un composé de formule IX

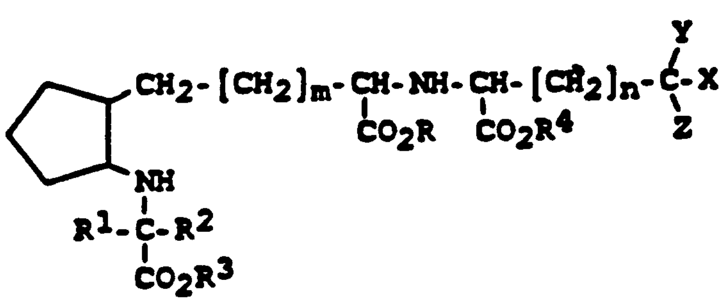

(IX)

dans laquelle m, n, $R^1$, $R^2$, $R^3$, $R^4$, X, Y et Z ont les significations données en a) et R représente un atome d'hydrogène ou un groupe ester, ou
f) pour la préparation des composés de formule I dans lesquels Y et Z représentent ensemble un atome d'oxygène, on fait réagir d'une façon connue en soi, dans une réaction de Michael, un

composé de formule III, définie en a), avec un composé de formule X

$R^4O_2C\text{-}CH=CH\text{-}CO\text{-}X$ (X)

dans laquelle $R^4$ et X sont tels que définis plus haut dans la formule I, ou on fait réagir le composé précité de formule III, d'une façon connue en soi, dans une réaction de Mannich, avec un composé de formule XI

$OHC\text{-}CO_2R^4$ (XI)

et avec un composé de formule XII

$X\text{-}CO\text{-}CH_3$ (XII)

formules dans lesquelles $R^4$ et X ont les significations définies à propos de la formule I, dans le cas où Y et Z représentent ensemble un atome d'oxygène, éventuellement on réduit ce groupe en Y = OH ou H et Z = H,

I) si on le désire, on élimine d'une façon connue en soi des groupes protecteurs éventuellement introduits temporairement pour la protection de groupes fonctionnels,

II) éventuellement on estérifie des groupes carboxy $CO_2R^3$ et/ou $CO_2R$ ($R^3$, $R^4$ = H), d'une façon connue en soi, avec formation des composés de formule I ($R^3$ et/ou $R^4 \neq$ H),

III) éventuellement on élimine par hydrolyse ou hydrogénolyse les radicaux $R^3$ et/ou $R^4$ ($R^3$, $R^4 \neq$ H), avec formation du(des) groupe(s) carboxy libre(s),

ou on inverse l'ordre de ces étapes I-III, et on convertit éventuellement en leurs sels physiologiquement acceptables les composés de formule I obtenus de cette façon.

**2.** Procédé selon la revendication 1, caractérisé en ce que l'on prépare des composés de formule I dans lesquels
m = 1 et
n = 1,

| | |
|---|---|
| $R^1$ | représente un atome d'hydrogène ou un radical alkyle en $C_1$-$C_3$, cycloalkyle en $C_3$-$C_6$, cycloalkyl($C_3$-$C_6$)-alkyle- |
| $R^2$ | représente un atome d'hydrogène ou un radical alkyle en $C_1$-$C_3$, cycloalkyle en $C_3$-$C_6$, cycloalkyl($C_3$-$C_6$)-alkyle- |
| $R^3$ | représente un atome d'hydrogène ou un radical alkyle en $C_1$-$C_4$, benzyle ou 4-méthoxy-benzyle, |
| $R^4$ | représente un atome d'hydrogène ou un radical alkyle en $C_1$-$C_4$, benzyle ou 4-méthoxy-benzyle, |
| X | représente le radical phényle ou un radical phényle mono- ou disubstitué par un ou des atomes de fluor et/ou de chlore, le radical méthyle, cyclohexyle ou aminoéthyle, |
| Y | représente un atome d'hydrogène ou le groupe hydroxy, et |
| Z | représente un atome d'hydrogène ou |
| Y et Z | représentent ensemble un atome d'oxygène. |

**3.** Procédé selon la revendication 1 ou 2, caractérisé en ce que l'on prépare des composés de formule I dans lesquels
m = 1,
n = 1,

| | |
|---|---|
| $R^1$, $R^2$ et $R^3$ | représentent chacun un atome d'hydrogène, |
| $R^4$ | représente un atome d'hydrogène ou le groupe éthyle, |
| Y et Z | représentent chacun un atome d'hydrogène et |
| X | représente le groupe phényle. |

**4.** Procédé selon la revendication 1, caractérisé en ce que l'on prépare des composés de formule I dans lesquels les atomes de carbone chiraux indiqués par un astérisque [(*)] ont la configuration S.

**5.** Procédé selon la revendication 1, caractérisé en ce que l'on prépare
la 1-carboxyméthyl-3-(S)-[1-(S)-éthoxy-carbonyl-3-phénylpropylamino]-2-oxo-perhydrocyclopent[b]-

azépine,

la 1-carboxyméthyl-3-(S)-[1-(S)-carboxy-3-phénylpropylamino]-2-oxo-perhydrocyclopent[b]azépine,

la 1-carboxyméthyl-3-(S)-[1-(S)-éthoxycarbonyl-butylamino]-2-oxo-perhydrocyclopent[b]azépine,

la 1-carboxyméthyl-3-(S)-[1-(S)-carboxy-butylamino]-2-oxo-perhydrocyclopent[b]azépine,

la 1-carboxyméthyl-3-(S)-[1-(S)-éthoxycarbonyl-5-aminopentylamino]-2-oxo-perhydrocyclopent[b]-azépine,

la 1-carboxyméthyl-3-(S)-[1-(S)-carboxy-5-aminopentylamino]-2-oxo-perhydrocyclopent[b]azépine,

leurs stéréoisomères ou leurs sels physiologiquement acceptables.

**6.** Procédé selon la revendication 1, caractérisé en ce que l'on prépare

la 1-carboxyméthyl-3-(S)-[1-(S)-éthoxycarbonyl-3-phénylpropylamino]-2-oxo-perhydro-2H-cyclopent-[b]azocine,

la 1-carboxyméthyl-3-(S)-[1-(S)-carboxy-3-phénylpropylamino]-2- oxo-perhydro-2H-cyclopent[b]-azocine,

leurs stéréoisomères et leurs sels physiologiquement acceptables.

**7.** Procédé pour la préparation de composés de formule XIII

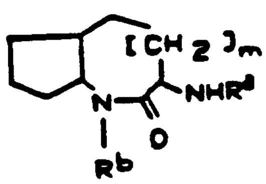

(XIII)

dans laquelle m représente 1 ou 2,

a) $R^a$ et $R^b$ représentent chacun un atome d'hydrogène,

b) $R^a$ représente un atome d'hydrogène et $R^b$ représente un radical

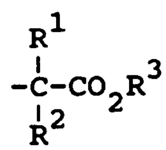

dans lequel $R^1$, $R^2$ et $R^3$ sont tels que définis dans la revendication 1, ou

c) $R^a$ représente un radical

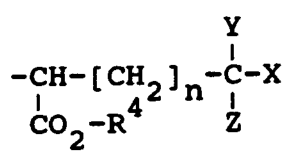

dans lequel n, $R^4$, X, Y et Z sont tels que définis dans la revendication 1, et $R^b$ représente un atome d'hydrogène, ou

d) $R^a$ représente un radical

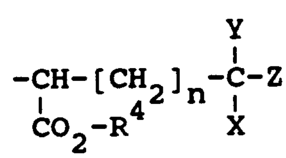

dans lequel n, $R^4$, X, Y et Z sont tels que définis plus haut, et $R^b$ représente un radical

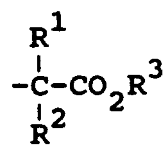

dans lequel $R^1$, $R^2$ et $R^3$ sont tels que définis plus haut,
caractérisé en ce que

a) on soumet à une cyclisation des composés de formule XIV

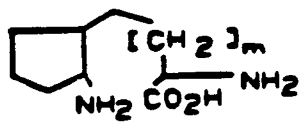

(XIV)

dans laquelle m représente 1 ou 2, pour aboutir aux composés de formule XV (≅ formule XIII dans laquelle $R^a$ et $R^b$ représentent chacun un atome d'hydrogène)

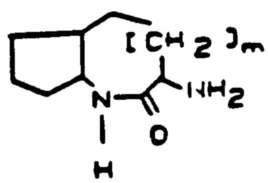

(XV)

dans laquelle m est tel que défini plus haut,

b) on réduit des composés de formule XVI

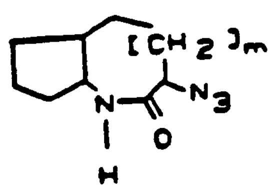

(XVI)

dans laquelle m est tel que défini plus haut, pour aboutir aux composés de formule XV définie plus haut,

c) on soumet à une alkylation des composés de formule XV ci-dessus, éventuellement avec des composés de formule VI,

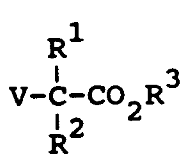

(VI)

dans laquelle V, $R^1$, $R^2$ et $R^3$ sont tels que définis en c) dans la revendication 1, pour aboutir aux composés de formule III (≅ formule XIII dans laquelle $R^a = H$, $R^b \neq H$),

d) on fait réagir des composés de formule XV ci-dessus, éventuellement avec des composés de formule II définis en a) dans la revendication 1, pour aboutir aux composés de formule V (≅ formule XV dans laquelle $R^a \neq H$, $R^b = H$),

e) on fait réagir d'une façon connue en soi, dans une réaction de Michael, des composés de formule XV ci-dessus avec un composé de formule X

$R^4O_2C\text{-}CH=CH\text{-}CO\text{-}X$ (X)

dans laquelle $R^4$ et x sont tels que définis plus haut dans la formule I, ou on fait réagir le composé précité de formule III, d'une façon connue en soi, dans une réaction de Mannich, avec un composé de formule XI

$OHC\text{-}CO_2R^4$ (XI)

et avec un composé de formule XII

$X\text{-}CO\text{-}CH_3$ (XII)

formules dans lesquelles $R^4$ et X ont les significations définies à propos de la formule I dans la revendication 1, dans le cas où Y et Z représentent ensemble un atome d'oxygène, éventuellement on réduit ce groupe en Y = OH ou H et Z = H,

f) ou on soumet à une alkylation un composé de formule XVI, avec des composés de formule VI, pour aboutir aux composés de formule XVII

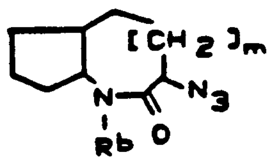

(XVII)

dans laquelle m et $R^b$ ($R^b \neq H$) sont tels que définis dans la revendication 7, et on convertit ces composés, par hydrogénation catalytique, avec des catalyseurs métalliques, ou par réaction avec des complexes dans des hydrures, en composés de formule XIII ($R^a$ = H, $R^b \neq H$).

**8.** Procédé pour la fabrication d'un produit pharmaceutique contenant un composé préparé selon l'une des revendications 1 à 6, caractérisé en ce que l'on met sous une forme d'administration appropriée un composé de formule I, conjointement avec un véhicule physiologiquement acceptable et éventuellement d'autres adjuvants.

**9.** Procédé selon la revendication 8, caractérisé en ce que l'on ajoute un diurétique.

**10.** Composé de formule XIII, dans laquelle $R^a$, $R^b$ et m sont tels que définis dans la revendication 7.